(19) Europäisches Patentamt
European Patent Office
Office européen des brevets

(11) Numéro de publication: **0 304 377 B1**

(12) # FASCICULE DE BREVET EUROPEEN

(45) Date de publication de fascicule du brevet: **18.01.95**

(51) Int. Cl.[6]: **C08F 8/40**, C08F 8/30, C08F 8/34, B01J 39/20

(21) Numéro de dépôt: **88402127.0**

(22) Date de dépôt: **18.08.88**

(54) **Polymères dérivés de polystyrènes et dextranes réticulés, leurs procédés de préparation et leurs applications pour l'analyse et la purification de molécules d'origine biologique.**

(30) Priorité: **21.08.87 FR 8711813**

(43) Date de publication de la demande:
**22.02.89 Bulletin 89/08**

(45) Mention de la délivrance du brevet:
**18.01.95 Bulletin 95/03**

(84) Etats contractants désignés:
**AT BE CH DE ES FR GB GR IT LI LU NL SE**

(56) Documents cités:
**AU-A- 533 614**
**US-A- 3 838 043**
**US-A- 3 951 744**

(73) Titulaire: **THERAPEUTIOUES SUBSTITUTIVES**
**Groupement d'Intérêt Public**
**Université Paris-Nord**
**(Laboratoire de Recherches sur les Macromolécules)**
**Avenue Jean-Baptiste Clément**
**F-93430 Villetaneuse (FR)**

(72) Inventeur: **Letourneur, Didier**
**32 Allée de la Clairière**
**F-93600 Aulnay (FR)**
Inventeur: **Douzon, Colette née Agnellet**
**6 Rue Taclet**
**F-75020 Paris (FR)**
Inventeur: **Migonney, Véronique née Touze**
**19 Rue des Callais**
**F-95600 Eaubonne (FR)**
Inventeur: **Muller, Daniel André**
**3 Impasse Guynemer**
**F-95230 Soisy-sous-Montmorency (FR)**
Inventeur: **Jozefowicz**
**65, 2éme Avenue**
**F-60260 Lamorlaye (FR)**

(74) Mandataire: **Le Brusque, Maurice et al**
**Cabinet Harlé et Phélip**
**21, rue de la Rochefoucauld**
**F-75009 Paris (FR)**

EP 0 304 377 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouvelles résines qui sont constituées par des polymères résultant de la modification des polymères aptes à servir de supports en chromatographie, que sont le polystyrènes et dextranes réticulés ; la présente invention concerne également la préparation de ces résines, ainsi que leurs applications pour l'analyse et la purification de molécules d'origine biologique, notamment comme phase stationnaire en chromatographie d' échange d' ions et d' affinité, en particulier pour réaliser le fractionnement de mélanges de protéines. Egalement, leur interaction avec des protéines, notamment les anticorps anti-ADN et anti-phospholipides présents dans le sérum des patients atteints de LED, les rend applicables à des opérations d'épuration sélective des différents types d'anticorps développés par les malades lupiques.

Ces nouvelles résines selon l' invention ont, comme originalité, de comporter des substituants copiant les sites chimiques de l'ADN et de certains phospholipides, et elles possèdent une capacité d'échange d'ions beaucoup plus importante que les supports traditionnels de chromatographie. Elles se présentent, de façon classique, sous la forme de particules dans le cas des polystyrènes réticulés ou de gels, dans le cas des dextranes réticulés, ayant à leur surface, dans une répartition statistique, les différents éléments suivants : bases puriques et pyrimidiques, pentoses, phosphates et constituants de phospholipides, fixés indépendamment, ou associés, sur les unités de polystyrène ou de dextrane avec des bras espaceurs de longueur ou de nature chimique variables ; des taux de substitution élevés peuvent être obtenus. La présence possible des groupements phosphorylés rend ces résines particulièrement intéressantes en chromatographie d'échange d'ions. Ainsi les résines selon la présente invention, porteuses de groupements phosphate, lesquels jusqu'alors n'avaient pu être greffés de façon importante sur les phases stationnaires classiques, ont des propriétés acido-basiques, et donc, d'échange d'ions, assez nettement différentes des supports habituels.

Par ailleurs, les résines à base de polystyrènes réticulés présentent l'avantage complémentaire de présenter l'excellente tenue mécanique du polymère de base, les rendant utilisables en chromatographie en phase liquide haute performance (H. P. L. C. ).

Les supports, qui ont été développés jusqu'ici en chromatographie d' échange d'ions, sont le plus souvent à base de polymères naturels, modifiés afin de porter des groupements permettant l'échange de protéines cationiques (groupements carboxyméthyle, sulfopropyle) ou anioniques (groupements aminoéthyle, diéthylaminoéthyle). Toutefois, ces supports sont des gels qui ne permettent qu'une utilisation en basse pression, ce qui limite considérablement leur emploi à l'échelle industrielle. En effet, leur faible résistance mécanique limite, voire interdit, leur utilisation en H. P. L. C. et, en conséquence, leur intérêt industriel.

Pour pallier ces inconvénients, on a déjà recherché, pour ces applications, des supports qui, de par leur réticulation, possèdent de bonnes propriétés mécaniques, c'est-à-dire une bonne rigidité. Parmi les polymères qui ont ainsi été proposés, on peut citer le polyacrylamide, le polymère trisacrylique, le poly-(méthacrylate d'hydroxyméthyle), les polymères vinyliques. Cependant, les modifications chimiques, qui ont été effectuées dans le but de conférer aux polymères de base un caractère de support d' échange d' ions, restent souvent difficiles, et les taux de substitution obtenus sont généralement faibles.

Ainsi, les supports à base de polystyrène réticulé avec un taux de l'ordre de 2% de divinylbenzène, possèdent une excellente tenue mécanique, mais leur forte hydrophobicité a jusqu'à maintenant fortement limité leur usage en chromatographie des protéines. Pour qu'un tel support soit utilisable en chromatographie d'échange d'ions pour des produits d'origine biologique, il est nécessaire qu'il soit fortement substitué par des motifs à la fois hydrophiles et ionisables.

Les polystyrènes (et copolymères du styrène) hydrophiles fonctionnels de l'invention répondent aux problèmes posés. Il est apparu en effet que de telles résines sont toujours utilisables dans des conditions d'élution en chromatographie liquide haute performance, car elles résistent sans modification à des pressions voisines de 200 bars.

La présente invention a d' abord pour objet un polymère dérivé d'un polymère ou copolymère réticulé du styrène ou d'un dextrane réticulé, caractérisé par le fait que la chaîne du polymère ou copolymère de base est substituée par un ou plusieurs groupes, identiques ou différents, appartenant aux catégories suivantes :

$-Z-A_1$ ;
$-Z-A_2$ ;
$-Z-A_1-Z'-A_2$ ;
$-Z-A_1-A_3-A_2$ ;
$-Z-A_1-A_4$

où :

- Z représente une chaîne d'espacement ;
- Z' représente une chaîne de liaison ;
- $A_1$ représente un reste phosphate ;
- $A_2$ représente le reste d' une base purique ou d'une base pyrimidique ;
- $A_3$ représente un reste de sucre ; et
- $A_4$ représente un reste d'une molécule intervenant dans la structure polaire des divers phospholipides, à l'exclusion des polymères uniquement substitués par des groupes $-Z-A_1-A_3-A_2$.

La chaîne d'espacement Z est notamment choisie parmi les restes :

- $-(CH_2)_n-$ , n valant de 1 à 6, éventuellement rendu hydrophile par remplacement d'au moins un H par un OH ; ou
- $-SO_2-NH-(CH_2)_m-$ , m valant de 1 à 6, le reste $-(CH_2)_{\overline{m}}$ étant éventuellement rendu hydrophile par remplacement d'au moins un H par un OH ; et
- dans le cas de la modification d'un dextrane réticulé, également

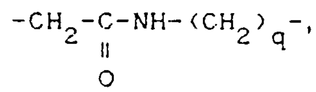

q valant de 1 à 6, le reste $-(CH_2)_q-$ étant éventuellement rendu hydrophile par remplacement d'au moins un H par un OH.

En particulier, on peut mentionner, comme chaînes d'espacement Z, les restes $-CH_2-$ ; $-(CH_2)_2-$ ; $-(CH_2)_3-$ ; $-SO_2-NH-(CH_2)_{2ou3}-$ ; $-SO_2-NH-CHOH-$ ; et $-SO_2-NH-CH_2-CHOH-CHOH-$ ; et, dans le cas de la modification d'un dextrane réticulé, également,

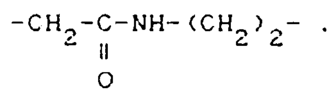

Quant à la chaîne de liaison Z', elle est notamment constituée par la chaîne $-(CH_2)_p-$ , p valant de 1 à 6, le reste $-(CH_2)_p-$ étant éventuellement rendu hydrophile par remplacement d'au moins un H par un OH.

En ce qui concerne le reste $A_2$, on peut mentionner, à titre d'exemples, les restes des bases puriques majeures que sont l'adénine et la guanine, ainsi que les restes des bases pyrimidiques majeures que sont la cytosine, la thymine et l'uracile.

Le reste $A_3$ représente notamment un reste de sucre, relié par son groupement $-CH_2-$ au reste phosphate ; à titre d'exemple, on peut citer un reste de pentose, en particulier, le reste du D-ribose (ou du 2-désoxy-D-ribose) de formule :

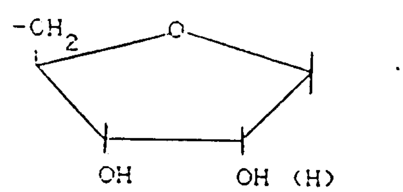

Quant au reste $A_4$, il représente notamment une molécule estérifiée de choline, d'éthanolamine, de sérine, de glycérol ou d'inositol.

L'un des deux polymères de base, qui est modifié selon la présente invention, est un homo- ou copolymère du styrène que l'on peut définir comme étant un polymère à partir d'un mélange de monomères de base comprenant, pour 100 parties en poids, 50 - 100 parties en poids de styrène éventuellement substitue comme l'α-méthylstyrène, et 0 - 50 parties en poids d'au moins un monomère copolymérisable à insaturation éthylénique choisi notamment parmi les monomères acryliques comme les acrylates et méthacrylates alkyliques inférieurs, les acrylates d'alcoxy inférieurs, l'acrylonitrile, l' acrylamide, les acrylates d'hydroxyalkyle inférieurs, les méthacrylates d'hydroxyalkyle inférieurs, l'acide acrylique et

l'acide méthacrylique. De plus, ces polymères du styrène sont réticulés, c'est-à-dire qu'un monomère de réticulation classique choisi notamment parmi les polyvinylbenzènes, comme le divinylbenzène, a été ajouté aux monomères de départ, en une quantité pouvant aller jusqu'à 5 parties en poids pour 100 parties en poids desdits monomères.

Selon l'invention, on utilisera, de préférence, l'homopolymère du styrène, lequel est un support classique ; celui que l'on a utilisé ici était un polystyrène réticulé avec au plus 5% en poids, en particulier avec 2% en poids, d'au moins un monomère de réticulation, comme le divinylbenzène, et se présentant sous la forme de billes d'une dimension voisine de 50 µm.

Pour simplification, on désignera, dans la suite du présente mémoire, les homo- et copolymères du styrène par le terme ⟨⟨polystyrène⟩⟩.

La substitution de la chaîne du polystyrène selon l' invention s'effectue principalement en position para du noyau phényle.

Par ailleurs, selon la présente invention, chaque groupe phényle non impliqué dans la réticulation peut comporter un substituant tel que défini ci-dessus.

Le polymère réticulé, qui est modifié selon l'invention, peut également être un dextrane réticulé, tel qu'un dextrane substitué en parties par des groupes carboxyméthyle. Les dextranes utilisés peuvent être ceux réticulés par l'épichlorhydrine.

La présente invention a encore pour objet un procédé de préparation du polymère dérivé du polystyrène ou dextrane réticulé, tel que défini ci-dessus, caractérisé par le fait qu'il comporte la fixation sur le polymère ou copolymère réticulé de base (polystyrène ou dextrane), en une ou plusieurs étapes, d' un reste -Z-OH, ou, dans le cas du polymère ou copolymère du styrène, d' un reste -Z-X (où X représente un halogène et Z est tel que défini ci-dessus), puis

(I) dans le cas où l'on a fixé un reste -Z-X, la réaction du polystyrène ainsi modifié avec une base purique ou pyrimidique, afin de former le substituant $-Z-A_2$;

(II) dans le cas où l'on a fixé un reste -Z-OH,

(IIa) la phosphorylation du polymère ou copolymère ainsi modifié, afin de former le substituant $-Z-A_1$; puis

- lorsque l'on souhaite former le substituant $-Z-A_1-Z'-A_2$, la réaction du reste $-Z-A_1$ avec une base purique modifiée schématisée par la formule :

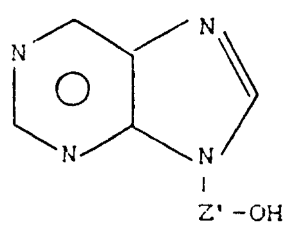

(I)

ou avec une base pyrimidique modifiée schématisée par la formule :

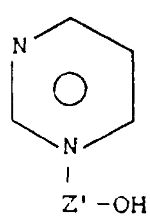

(II)

- lorque l'on souhaite former le substituant $-Z-A_1-A_3-A_2$, la réaction du reste $-Z-A_1$ avec un nucléoside de formule $-A_3-A_2$, résultant de la liaison de l' azote 9 d'une base purique ou de l'azote 1 d'une base pyrimidique avec le carbone 1' d'un sucre ;
- lorsque l'on souhaite former le substituant $-Z-A_1-A_4$, la réaction du groupement $-Z-A_1$ avec une molécule intervenant dans la structure polaire des divers phospholipides ; ou bien

(IIb) lorsque l'on souhaite former directement le substituant $-Z-A_1-A_3-A_2$, la réaction du polymère ou copolymère ainsi modifié avec un nucléoside monophosphate de formule $A_1-A_3-A_2$ ;

(IIc) lorsque l'on souhaite former le substituant $-Z-A_1-Z'-A_2$, la réaction du polymère ou copolymère ainsi modifié avec une base purique transformée schématisée par la formule :

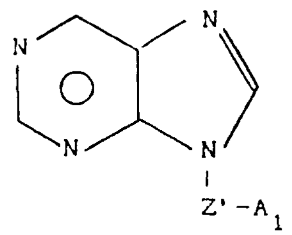

(Ia)

et qui est obtenue par phosphorylation de la base purique modifiée de formule (I), ou avec une base pyrimidique transformée schématisée par la formule :

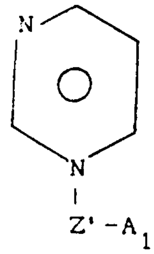

(IIa)

et qui est obtenue par la phosphorylation de la base pyrimidique modifiée de formule (II) ; ou bien

(IId) lorsqu'on souhaite former le substituant $-Z-A_1-A_4$,la réaction du polymère ou copolymère ainsi modifié avec un reste $A_4$ phosphorylé.

Dans le cas où l'on utilise, comme polymère de départ, un polystyrène réticulé :

1) On peut commencer par fixer, sur le polystyrène de départ, le radical $-CH_2-X$, en faisant réagir ledit polystyrène avec un halométhyl méthyl éther en présence de chlorure stannique.

2) On peut également commencer par fixer, sur le polystyrène de départ, le radical $-CH_2OH$, en faisant réagir, en présence d'un catalyseur transfert de phase, la résine obtenue comme indiqué ci-dessus, c'est-à-dire modifiée par des radicaux $-CH_2-X$, avec l'acétate de potassium, puis en hydrolysant l'acétate obtenu par la potasse concentrée.

3) On peut également commencer par fixer, sur le polystyrène de départ, le radical $-CH_2-CH_2-OH$,

- en faisant réagir la résine modifiée de façon à porter des radicaux $-CH_2-X$ avec le cyanure de sodium, en présence d'un catalyseur transfert de phase, pour obtenir le substituant $-CH_2-C\equiv N$ ;
- puis en faisant réagir le polystyrène ainsi modifié avec l'acide chlorhydrique en milieu alcoolique, afin d'obtenir le substituant $-CH_2-COOH$ ;
- puis en réduisant par le diborane, afin d'obtenir le susbtituant $-CH_2-CH_2-OH$.

4) On peut également commencer par fixer, sur le polystyrène de départ, le radical $-CH_2-CH_2-CH_2-OH$,

- en condensant un malonate d'alkyle sur la résine comportant des groupes $-CH_2-X$, afin d'obtenir le substituant:

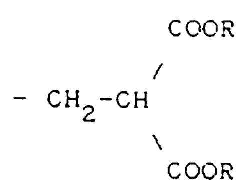

(R étant un reste alkyle) ;

- puis, en hydrolysant la fonction diester ainsi obtenue pour donner une fonction diacide à laquelle on fait subir une décarboxylation en milieu acide, afin d'obtenir le substituant $-CH_2-CH_2-COOH$ ;
- puis en réduisant par le diborane afin d'obtenir le substituant $-CH_2-CH_2-CH_2-OH$.

5) On peut également commencer par fixer sur le polystyrène de départ, le radical $-SO_2-NH-(CH_2)_{2\ ou\ 3}-OH$,

- en faisant réagir l'acide chlorosulfonique sur le polystyrène de départ, de façon à obtenir le substituant $-SO_2Cl$,
- puis en faisant agir l'éthanolamine ou la propanolamine sur le substituant précité.

6) On peut également commencer par fixer sur le polystyrène de départ, le radical $-SO_2-NH-CH_2-CHOH-CH_2OH$,

- en faisant réagir l'acide chlorosulfonique sur le polystyrène de départ, de façon à obtenir le substituant $-SO_2Cl$ ;
- puis en faisant agir l'amino-3 propanediol-1,2 sur le substituant précité.

Dans le cas où l'on utilise, comme polymère de départ, un dextrane réticulé, on peut commencer par y fixer le radical $-NH-(CH_2)_q-OH$, q allant de 1 à 6, par réaction avec un halogénure de thionyle, puis par réaction avec une amine de formule $OH-(CH_2)_q-NH_2$ ou bien, à l'aide d'un agent de couplage, par condensation avec ladite amine hydroxylée.

On réalise avantageusement la phosphorylation à l'aide du dichlorophosphate de méthyle, de l'acide phosphoreux ou de l'oxychlorure de phosphore, et notamment, à l'aide de l'oxychlorure de phosphore.

Pour préparer les bases modifiées de formules (I) ou (II) dans lesquelles $Z' = -CH_2-CH_2-$, on fait réagir la base avec le carbonate d'éthylène en présence d'un catalyseur transfert de phase.

On réalise la réaction du reste $-Z-A_1$ sur les bases modifiées des formules (I) ou (II) à l'aide d'un agent de couplage, tel que la N,N'-dicyclohexylcarbodiimide (DCCI).

Enfin, on prépare une base modifiée de formule (Ia) ou (IIa) en phosphorylant avec $POCl_3$ la base correspondante de formule respectivement (I) ou (II), puis on condense ladite base de formule (Ia) ou (IIa) sur le substituant -Z-OH à l'aide d'un agent de couplage, tel que la DCCI.

La présente invention a également pour objet l'utilisation du polymère dérivé d'un polystyrène ou dextrane réticulé, tel que défini ci-dessus, comme phase stationnaire en chromatographie d'échanges d'ions, et, particulièrement pour le fractionnement de mélanges protéiques, ainsi que comme phase stationnaire en chromatographie d'affinité, par exemple pour réaliser des épurations sélectives des différents types d'anticorps développés par les malades lupiques (anticorps anti-ADN, anti-coagulants circulants et anti-phospholipides). On peut également mentionner la purification, par chromatographie d'affinité ou d'échange d'ions, des enzymes, co-enzymes, ou complexes enzymatiques admettant comme substrats, l'ADN, l'ARN et les nucléotides ; ainsi que la purification, à partir de divers extraits plasmatiques, des facteurs de la coagulation sanguine, notamment les facteurs II, VII, IX et X.

La présente invention a également pour objet l'utilisation du polymère dérivé d'un polystyrène ou dextrane réticulé, tel que défini ci-dessus, pour la purification des molécules d'origine biologique, telles que les protéines intervenant au cours du processus de la coagulation sanguine, et dans des systèmes d'analyse biologique, tels que RIA, ELISA, électrophorèse, immunoélectrophorèse mono- et bi-dimensionnelle, électrofocalisation, etc. A titre d'exemple, on peut citer les dosages des différents types d'anticorps lupiques, et, notamment, le dosage des anticoagulants circulants et/ou des anti-phospholipides.

Pour mieux illustrer la présente invention, on en décrira ci-après plusieurs modes de mise en oeuvre Les Exemples A à H concernent la préparation de différentes résines de polystyrène modifiées intermédiaires dans la préparation des résines selon la présente invention. Les Exemples 1 à 7 concernent la préparation de différentes résines selon la présente invention. Les résultats de microanalyse pour chacune des résines mises en jeu sont rapportés dans le tableau III qui fait suite à l'Exemple 5. Les Exemples 8 à 12 illustrent les différentes utilisations des résines selon l'invention.

**Exemple A**

**Préparation du poly(parachlorométhylstyrène)**

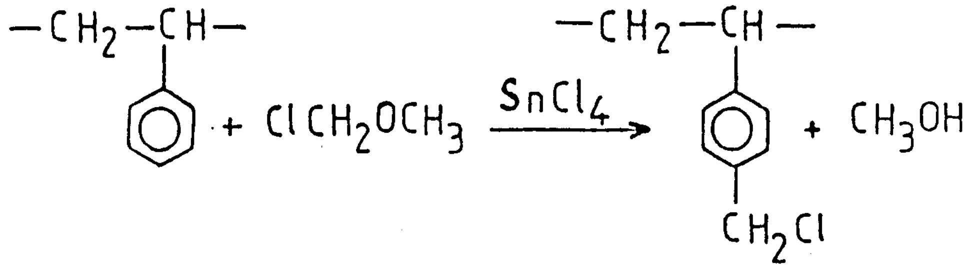


On place 24,5 g (235 méq) de polystyrène (FLUKA) dans 250 ml (4000 méq) de $CH_2Cl_2$ sous agitation, pendant 4 heures. On ajoute, à 0 ° C, 45 ml (600 méq) de $ClCH_2OCH_3$ et 10 ml (85 méq) de $SnCl_4$, puis on maintient l'agitation, à la température ambiante, pendant 2 heures et demie. Ensuite, on filtre et on laisse en suspension la résine dans $CH_2Cl_2$,puis dans un mélange HCl 3N (1/4)/dioxanne (3/4), des mélanges dioxanne/$H_2O$ en quantités croissantes de dioxanne, du méthanol et du $CH_2Cl_2$.

Le rendement est supérieur à 80%.

Cette résine PS-$CH_2Cl$ servira de base pour de nombreuses réactions.

TABLEAU I

| DOSAGE ARGENTIMETRIQUE * PS-$CH_2$ Cl (microanalyse) | | | |
|---|---|---|---|
| Méq/g de - $CH_2Cl$ | 5,1 (5,3) | 5,3 (5,15) | 5,8 (5,7) |
| % Cl en g/100 g | 18,1 (18,8) | 18,8 (18,3) | 20,6 (20,1) |
| % de Substitution | 77,8 (81,1) | 81 (78,5) | 88 (86,5) |

* Le taux de chlore de la résine préparée est déterminé par dosage des ions chlorure libérés par hydrolyse par la butylamine. Après avoir acidifié la suspension, le dosage est effectué à l'aide d'une électrode indicatrice d'argent par une solution de nitrate d'argent.

**Exemple B**

**Préparation du poly(parachlorosulfonylstyrène)**

**1ère étape :**

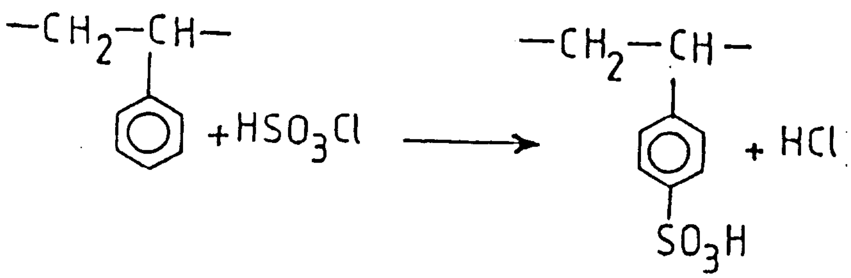

**2ème étape :**

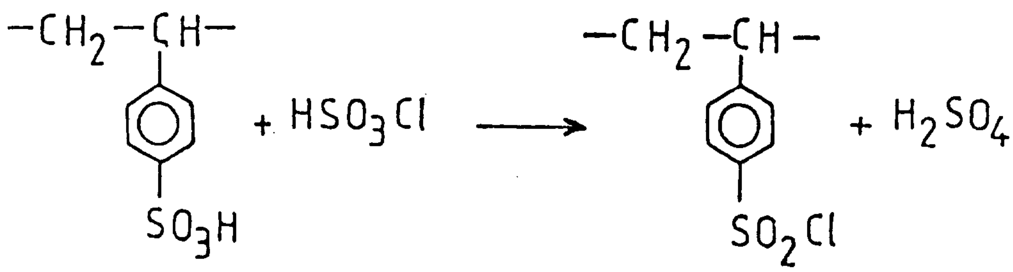

Le symbole PS remplacera, dans la suite du présent mémoire, le polystyrène substitué en position para.

On place 2,6 g (25 méq) de polystyrène (FLUKA) dans 125 ml (2000 méq) de $CH_2Cl_2$, sous agitation, pendant 4 heures, à la température ambiante. On ajoute ensuite 15 ml (225 méq) de $HSO_3Cl$ et on laisse 1 heure sous agitation. On filtre avec $CH_2Cl_2$, puis de l'acétone et du $CH_2Cl_2$, et on utilise immédiatement le produit obtenu pour réagir avec l'éthanolamine, la propanolamine ou l'amino-3 propanediol-1,2.

Le rendement total est voisin de 80%.

TABLEAU II

| DOSAGE ARGENTIMETRIQUE * DE PS-$SO_2$ Cl | | |
|---|---|---|
| Méq/g de -$SO_2$Cl | % Cl en g/100 g | % de substitution |
| 3,86 | 13,7 | 78,2 |
| 3,88 | 13,8 | 78,5 |

* Le taux de chlore de la résine préparée est déterminé par dosage des ions chlorure libérés par hydrolyse par la soude. Après avoir acidifié la suspension, le dosage est effectué à l'aide d'une électrode indicatrice d'argent par une solution de nitrate d'argent.

**Exemple C**

**Préparation du poly(parahydroxyméthylstyrène)**

**1ère étape :**

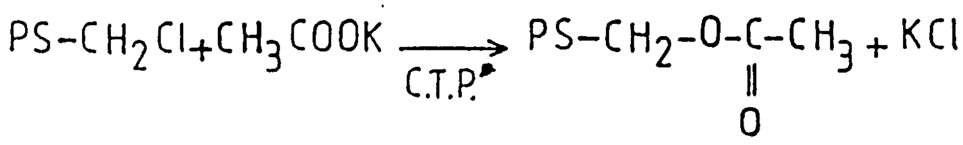


* C. T. P. = catalyseur de transfert de phase constitué par du chlorure de tricaprylylméthylammonium

**2ème étape :**

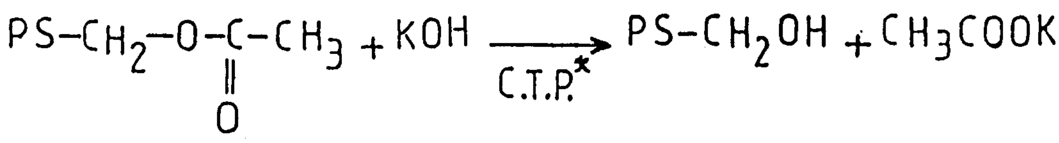


* C. T. P. = catalyseur de transfert de phase constitué par du chlorure de tricaprylylméthylammonium

On place 10 g (55 méq) de PS-$CH_2Cl$ dans 70 ml (620 méq) de $C_6H_4Cl_2$ sous agitation, pendant 1 heure. On ajoute 15 g de $CH_3COOK$ dissous dans 30 ml d'eau, 3 ml de catalyseur, et on place le ballon dans un bain d'huile à 85-90 °C, pendant environ 30 heures ; puis, sans séparer, on ajoute, dans le ballon, 15 g de KOH dissous dans 15 ml d'eau et 1 ml de catalyseur qu'on laisse toujours à la même température pendant 45 heures. Ensuite, on filtre avec de l'eau, puis des mélanges $H_2O$/tétrahydrofuranne de plus en plus concentrés en tétrahydrofuranne, du méthanol et du $CH_2Cl_2$.

Malgré la réaction en phase hétérogène, la réaction s'effectue de façon quasi totale.

**Exemple D**

**Préparation du poly(paracarboxyméthylstyrène)**

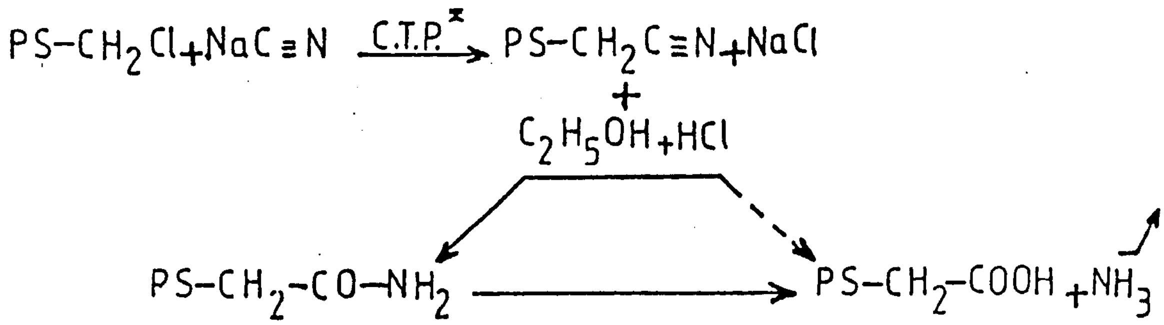


* C. T. P. = catalyseur de transfert de phase constitué par $[(C_4H_9)_4N^+, {}^-SO_4H]$

Dans un premier stade, on dissout, à chaud, 3,5 g de NaC≡N dans 50 ml de N,N-diméthylformamide, on ajoute 5 g (26 méq) de PS-$CH_2$Cl, 50 ml de N,N-diméthylformamide et 1,2 g (3,5 méq) de $[(C_4H_9)_4N^+, {}^-SO_4H]$, que l'on porte à 80 °C pendant 24 heures, on filtre ensuite en effectuant les lavages classiques avec $H_2O$, le dioxanne, le méthanol, $CH_2Cl_2$, et on sèche la résine formée qui est la paracyanométhylstyrène PS-$CH_2$-C≡N.

Dans un second stade, on place 2 g (11 méq) de PS-$CH_2$-C≡N et un mélange HCl concentré (37,5 ml)-/éthanol (12,5 ml) dans une ampoule de verre que l'on scelle. On porte l'ampoule à 110 °C, pendant 30 heures, sous agitation, puis, toujours sous agitation, pendant 4 heures à la température ambiante ; on ouvre l'ampoule (placée dans un bain de glace), et, de nouveau, on agite à la température ambiante, pendant 4 heures. On plonge le produit obtenu dans un mélange d'eau et de méthanol ; on filtre ensuite avec de plus en plus de méthanol ; ensuite, on place le produit pendant une dizaine d'heures en suspension dans de la soude 5N ; on filtre à l'eau, puis dans de la soude $10^{-2}$N ; on filtre à l'eau (on obtient PS-$CH_2$-COONa). On laisse alors en suspension dans un mélange HCl 3N(50) / dioxanne (50) pendant une dizaine d'heures, et on effectue les filtrations avec $H_2O$, le dioxanne, le méthanol et $CH_2Cl_2$

**Exemple E**

**Préparation du poly(paracarboxyéthylstyrène)**

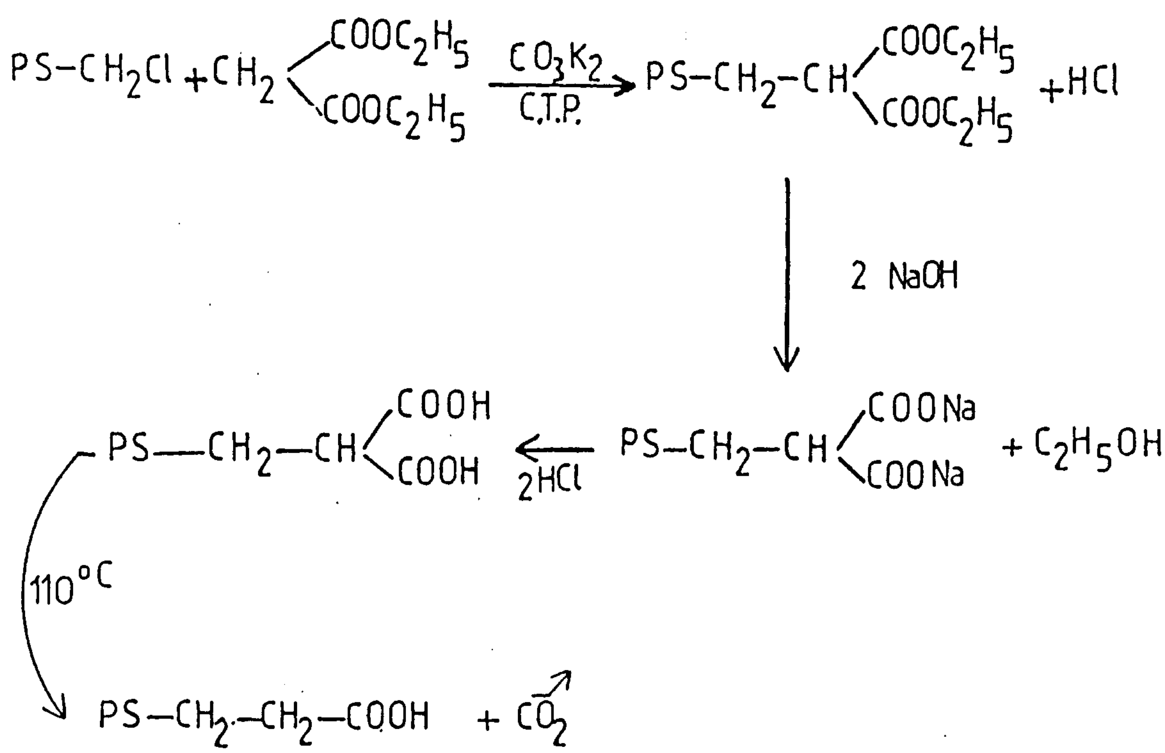


On dissout, à chaud, 5 g de $CO_3K_2$ dans 50 ml de N,N-diméthylformamide, puis on ajoute 1 g de sulfate de tétrabutylammonium, 2 g (11 méq) de PS-$CH_2$Cl et 15 ml (100 méq) de $(COOC_2H_5)_2CH_2$, à 85 °C, pendant 24 heures. On filtre ensuite avec de l'eau, du tétrahydrofuranne, du méthanol et $CH_2Cl_2$.

Ensuite, 1 g du diester obtenu est mis sous agitation dans 15 ml d'éthanol pendant 2 heures. On rajoute 50 ml de NaOH 5N, et l'on agite pendant 15 heures. Après filtration à l'eau, on met en suspension la résine dans 100 ml d'HCl 5N, à 110 °C, pendant 24 heures : puis, on filtre avec $H_2O$, du dioxanne, du méthanol et $CH_2Cl_2$.

**Exemple F1**

Préparation des composés PS-$(CH_2)_{n=1,2}$-$CH_2OH$

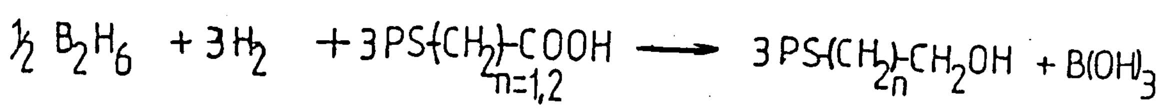

Ce premier procédé utilise une solution commerciale molaire de $B_2H_6$ dans le tétrahydrofuranne et réagit à 0 °C suivant les deux réactions ci-dessus à 95%.

Ayant eu bien soin de tout sécher avant, on place 0,8 g (3,5 méq) de PS-$(CH_2)_{1,2}$-COOH dans 35 ml de tétrahydrofuranne anhydre pendant 30 minutes ; puis on met le ballon dans un bain à -5 °C, -10 °C, pendant 1 heure. A l'aide d'une seringue, on injecte ensuite lentement 18 ml d'un mélange $B_2H_6$/tétrahydrofuranne 1 M, toujours dans le bain à la même température. On laisse agiter pendant 1 heure ; on porte le

ballon à 35 °C pendant 6 heures, puis à la température ambiante pendant une trentaine d'heures. On additionne enfin un mélange $H_2O$ (50)/tétrahydrofuranne (50), on filtre avec de l'eau, on laisse ensuite agiter dans une solution (HCl 1N, dioxane) pendant 15 heures ; on filtre et on met en suspension dans NaOH 1N, puis plusieurs fois dans NaOH $10^{-2}$ N pendant 40 heures environ et on filtre avec de l'eau, du méthanol et $CH_2Cl_2$.

**Exemple F2**

**Préparation des composés PS-$(CH_2)_{n=1,2}$-$CH_2OH$**

I $3NaBH_4 + 4BF_3 \rightarrow 2B_2H_6 + 3NaBF_4$

$$\text{II} \quad \tfrac{1}{2}\,B_2H_6 + 3H_2 + 3PS\text{-}(CH_2)_{n=1,2}\text{-}COOH \longrightarrow 3PS\text{-}(CH_2)_n\text{-}CH_2OH + B(OH)_3$$

Ce second procédé nécessite la préparation du diborane ⟨⟨in situ⟩⟩ par action du borohydrure de sodium ($NaBH_4$) sur un mélange de trifluorure de bore et de méthoxy-2 éthyl éther (⟨⟨diglyme⟩⟩), en présence de la résine PS-$(CH_2)_{n=1,2}$-COOH, suivant les réactions I, puis II et III

1 g (4 méq) de résine bien sèche est mis en suspension dans 20 ml de diglyme pendant 1 heure. On introduit, à l'aide d'une fiole, 4,5 ml d'une solution préparée par dissolution de 1 g de $NaBH_4$ dans 25 ml de diglyme (4,7 méq). On injecte ensuite lentement une solution de [0,75 ml ($BF_3$, $(C_2H_5)$-O), 1,25 ml diglyme]. Puis on additionne, lentement, à l'aide d'une ampoule, un mélange $H_2O$ (50)/diglyme (50). On verse le tout dans 200 ml d'eau. On filtre avec de l'eau, du dioxanne, du méthanol et $CH_2Cl_2$.

**Exemple G**

**Préparation du poly(para N-hydroxyéthylsulfamidestyrène) et du poly(para N-hydroxypropylsulfamide styrène)**

$$PS\text{-}SO_2Cl + H_2N\text{-}(CH_2)_{n=2,3}\text{-}OH \longrightarrow PS\text{-}SO_2\text{-}NH\text{-}(CH_2)_{n=2,3}\text{-}OH + HCl$$

On place 5,1 g (20 méq) de PS-$SO_2Cl$ dans 50 ml de $CH_2Cl_2$, sous agitation, pendant 1 heure. On ajoute 28 ml (450 méq) (d'éthanolamine ou 35 ml de propanolamine (450 méq) portés à 50 °C, pendant 24 heures. On filtre ensuite avec $CH_2Cl_2$ et avec de l'eau. On agite pendant 2 heures le produit obtenu dans un mélange HCl 2N (1/4)/$H_2O$ (3/4) ; on filtre et on agite ensuite dans l'eau, le tétrahydrofuranne et $CH_2Cl_2$.

Les rendements sont supérieurs à 90%.

**Exemple H**

**Préparation du poly(para N-dihydroxy-1,2 propylsulfamidestyrène)**

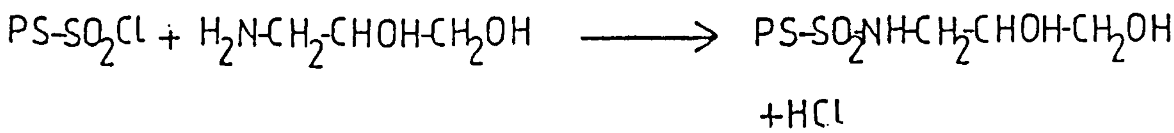

On place 4 g (16 méq) de PS-$SO_2$Cl + 10 ml de $CH_2Cl_2$ + 8 ml (130 méq) d'amino-3 propanediol-1,2 à température ambiante, pendant 1 heure, puis à 50 °C, sous agitation pendant 5 heures ; et, enfin, à la température ambiante, pendant heures ; puis, on filtre avec $CH_2Cl_2$ et de l'eau : on agite pendant 2 heures dans HCl (0,3 N), on filtre avec de l'eau et $CH_2Cl_2$.

**Exemple 1**

**Phosphorylation des résines hydroxylées**

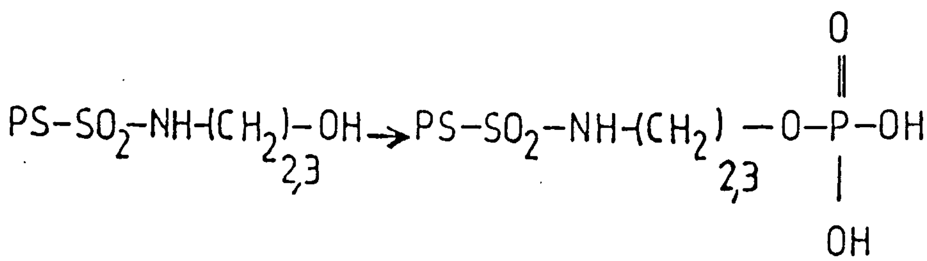

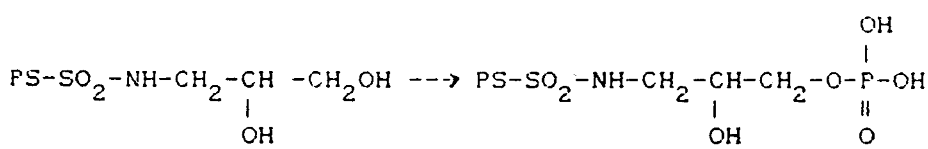

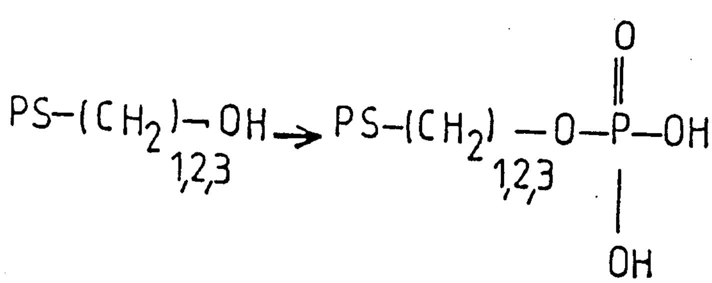

**A - Phosphorylation à l'aide du dichlorophosphate de méthyle**

L'action du dichlorophosphate de méthyle sur la pyridine, puis de leur action sur un alcool a été décrite par J. SMURT et J. CATLIN dans "Tetrahedron Letters", n° 58, pages 5081-82 (1970), puis par M. RUBINSTEIN et A. PATCHORNIK, dans "Tetrahedron Letters", vol. 31, pages 2107-2110 (1975). Dans une première étape, il se forme le sel de N-méthylpyridinium dichlorophosphate qui réagit ensuite sur un alcool.

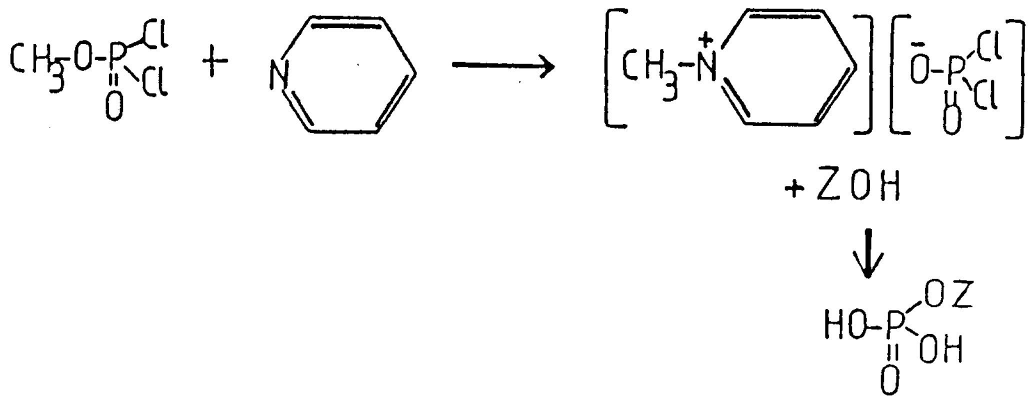


Cette méthode a été effectuée sur les deux familles de résines indiquées en tête de cet exemple :

On place, à 0 °C, 50 ml de pyridine anhydre et 5 ml (50 méq) de dichlorophosphate de méthyle, pendant 1/2 heure. On verse 2 g (environ 10 méq) de la résine hydroxylée choisie, sous agitation, à la température ambiante, pendant 24 heures. On verse ensuite le tout dans 400 ml de $CO_3HNa$ à 10% sous agitation, pendant 4 heures ; on filtre ; on met en suspension le produit obtenu dans HCl (1N) puis on filtre et on agite dans l'eau, le dioxanne et $CH_2Cl_2$.

**B - Phosphorylation à l'aide de l'acide phosphoreux.**

Le principe de cette méthode s'appuie sur l'article de H. TAKAKU ; Y. SHIMADA ; H. OKA, dans "Chem. Pharm. Bull", vol. 21, n° 8, pages 1844-45 (1973)", suivant lequel on utilise l'acide phosphoreux comme agent de phosphorylation. La réaction peut être décomposée en deux stades : tout d'abord l'acide phosphoreux $H_3PO_3$ sur le N-méthylimidazole en présence de chlorure mercurique $HgCl_2$, forme le sel de N-phosphoryl-N'-méthylimidazole, qui agit ensuite sur un alcool pour former un phosphate substitué, c'est-à-dire :

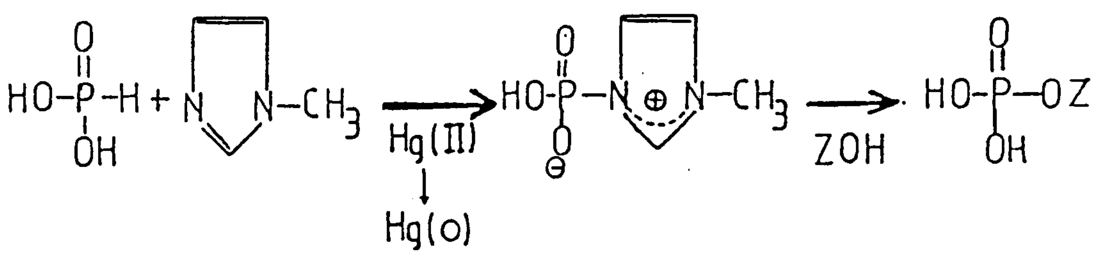


Cette méthode a été effectuée sur les deux familles de résines indiquées en tête de cet exemple.

On place 15 ml de N-méthylimidazole (180 méq), 4,1 g (15 méq) d'$HgCl_2$ et 1,5 g (15 méq) d'$H_3PO_3$ dans un ballon à 80 °C, pendant 2 heures. On ajoute 2 g (environ 10 méq) de résine hydroxylée, toujours à 80 °C, pendant 24 heures. On filtre à l'eau et on verse le tout dans 400 ml de bicarbonate de soude saturé ; on filtre avec de l'eau, du dioxanne, du méthanol et $CH_2Cl_2$.

**C - Phosphorylation à l'aide de l'oxychlorure de phosphore.**

La réaction peut être représentée ainsi :

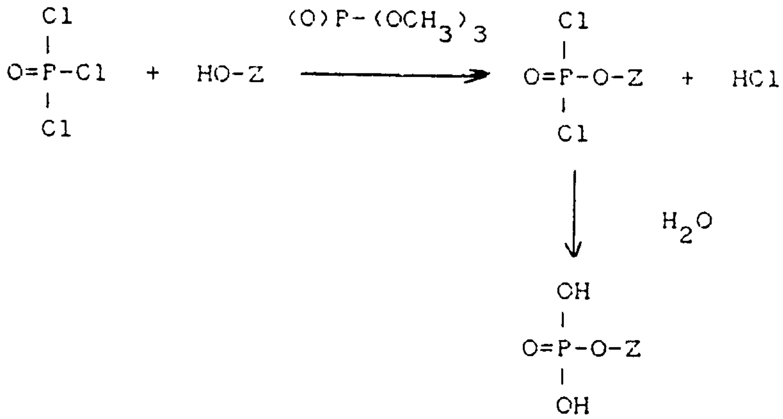


Cette méthode a été effectuée sur les trois familles de résines indiquées en tête de cet exemple.

1 g (5 méq) de résine hydroxylée est mélangé à 10 ml de PO-$(OCH_3)_3$ et à 1,5 g (10 méq) de $POCl_3$ ; le tout est porte à 60 °C, pendant 24 heures, puis versé dans l'eau et on lave avec le tétrahydrofuranne, le méthanol et $CH_2Cl_2$.

On a obtenu, en particulier, la transformation du poly(hydroxyéthyl styrène) en son composé phosphorylé dans lequel les groupes désirés

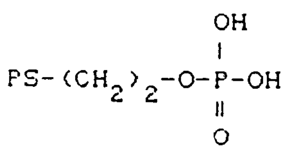

représentent 78% de la masse de la résine.

**Exemple 2**

**A - Fixation de l'uracile sur le polystyrène chlorométhylé**

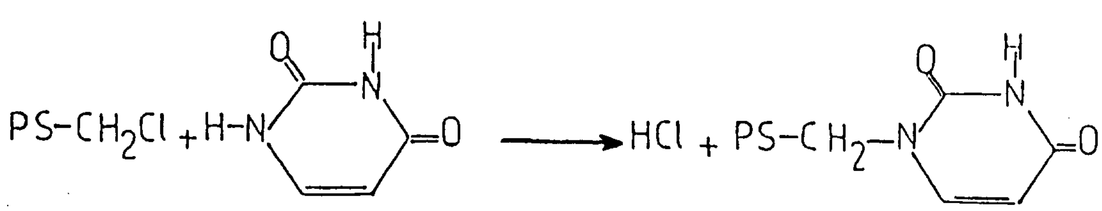

On place 2 g (10,6 méq) de PS-$CH_2Cl$ dans 20 ml de diméthylsulfoxyde anhydre dans un ballon bien sec avec une garde en $CaCl_2$ ; on ajoute 2,1 g (19 méq) d'uracile dissous à chaud dans 30 ml de diméthylsulfoxyde, et 4,5 g de $CO_3K_2$ et 80 ml de diméthylsulfoxyde. On porte le ballon à 40 °C sous argon pendant 10 heures. On verse la solution dans 600 ml d'eau glacée que l'on agite pendant 2 heures. On filtre avec de l'eau, des mélanges $H_2O$/tétrahydrofuranne de plus en plus concentrés en tétrahydrofuranne, du méthanol et $CH_2Cl_2$.

Le taux de substitution du polystyrène est de 59%.

**B - Fixation d'autres bases sur le polystyrène chlorométhylé.**

- De la même façon que précédemment, on a fixé, sur la résine PS-$CH_2Cl$, dans le diméthylsulfoxyde et en présence de carbonate de potassium, la cytosine, la thymine, l'adénine et la guanine, pour obtenir les résines modifiées respectives suivantes, les taux de substitution du polystyrène étant respectivement 53, 39, 74 et 89% :

cytosine 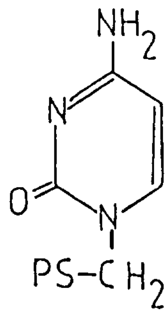

thymine 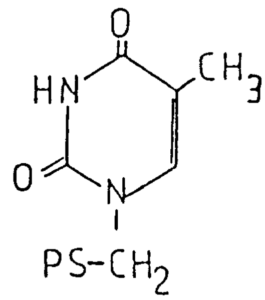

Adéninc 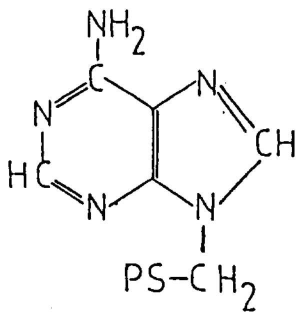

Guanine 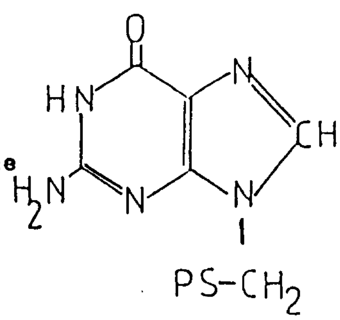

- Egalement, on a fixé l'adénine sur la résine PS-$CH_2Cl$ en conduisant la réaction dans l'orthodichlorobenzène, en présence de potasse.

**Exemple 3**

Fixation de bases sur la résine 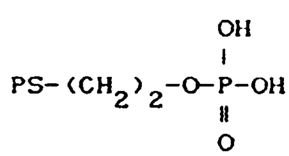

On prépare des bases N-hydroxyéthylées en faisant réagir la base choisie avec le carbonate d'éthylène en présence du catalyseur transfert de phase $[(C_4H_9)_4N^{\oplus}, {}^{\ominus}Br]$ dans le N,N-diméthylformamide anhydre. Il est facile d'obtenir les produits purs par cristallisation.

Il est possible de condenser ces bases N-hydroxyéthylées avec la résine

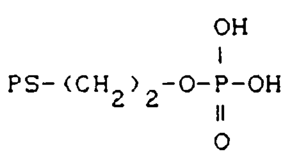

à l'aide de la N,N'-dicyclohexylcarbodiimide (D.C.C.I) dans le N,N-diméthylformamide. Le mécanisme d'action de la D.C.C.I. est le suivant :

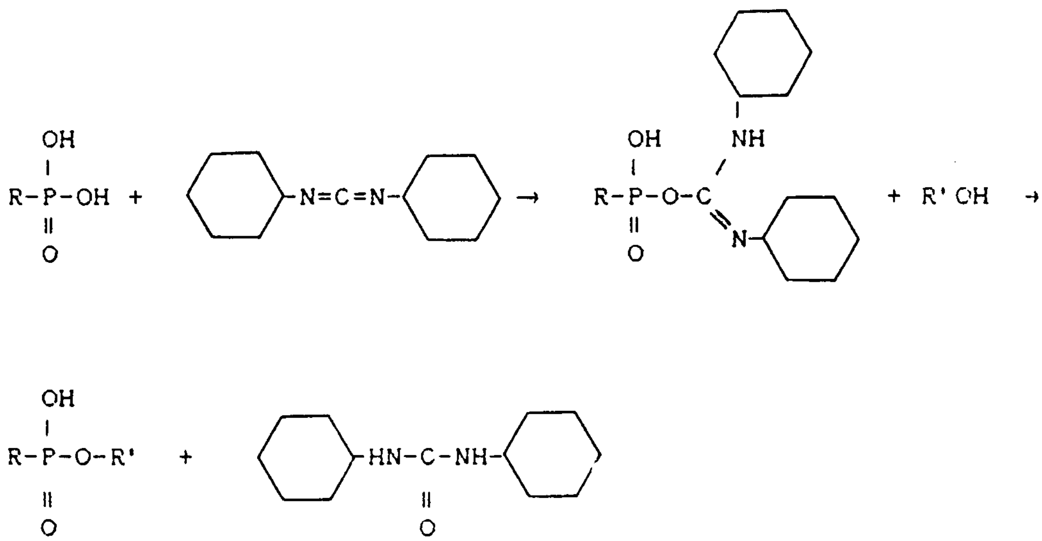

La N,N'-dicyclohexylurée est infiniment soluble dans le N,N-diméthylformamide et l'éthanol à chaud.

- Lorqu'on utilise l'adénine comme base, le schéma réactionnel est le suivant :

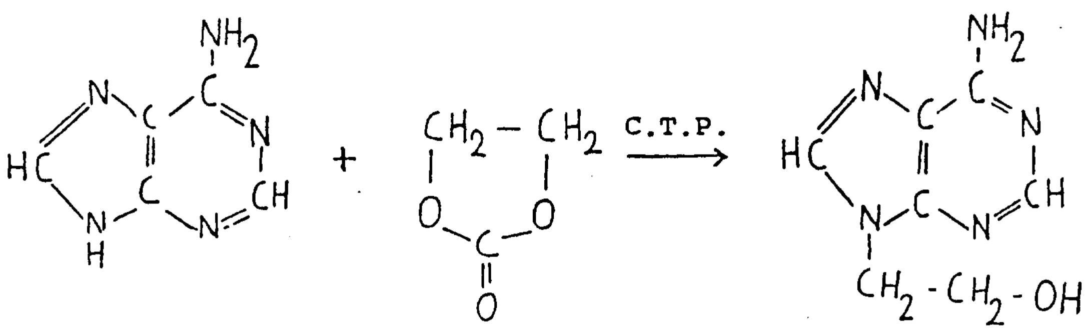


(N-9, hydroxyéthyladénine)

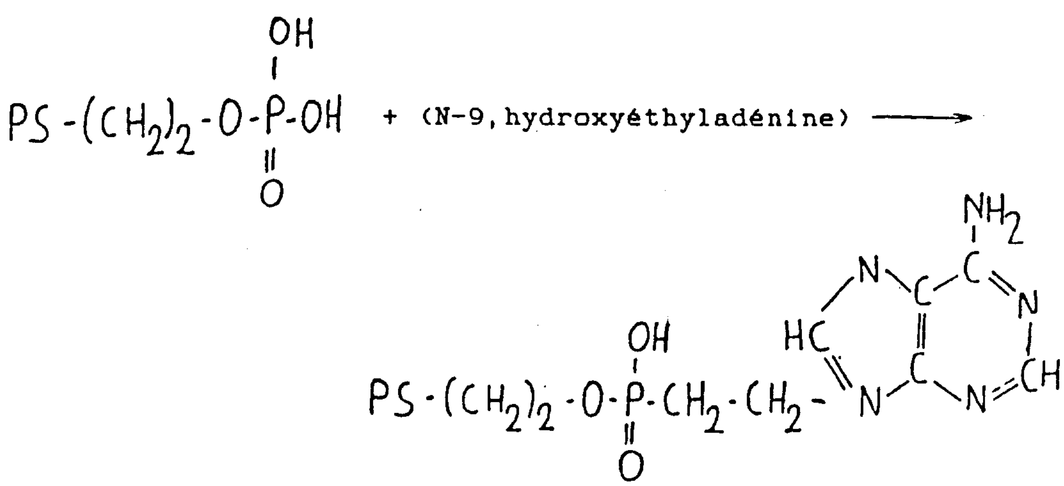


On réalise la condensation avec 1 méq/g de substitution en ((hydroxyéthylphosphate hydroxyéthyladénine⟩⟩ (soit 39% en masse) sur le polystyrène, tout en laissant 0,9 méq/g de substitution en ⟨⟨hydroxyéthylphosphate⟩⟩ (soit 22% en masse).

- Lorsque l'on utilise la cytosine comme base, on commence par préparer la N,1-hydroxyethylcytosine de formule :

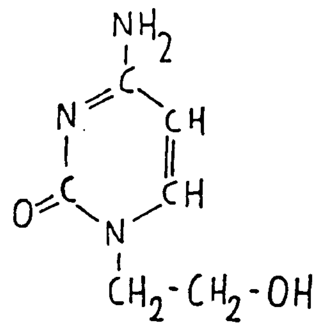

On réalise la condensation de la N,1-hydroxyéthylcytosine sur

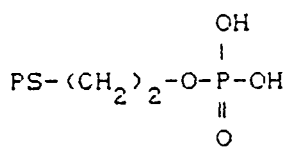

avec 0,95 méq/g de substitution en ⟨⟨hydroxyéthylphosphate hydroxyéthylcytosine⟩⟩ (soit 34% en masse) sur le polystyrène, tout en laissant 1,3 méq/g de substitution en ⟨⟨hydroxyéthylphosphate⟩⟩ - (soit 30% en masse)

- Lorsqu'on utilise la thymine comme base, on commence par préparer la N,1-hydroxyéthylthymine de formule :

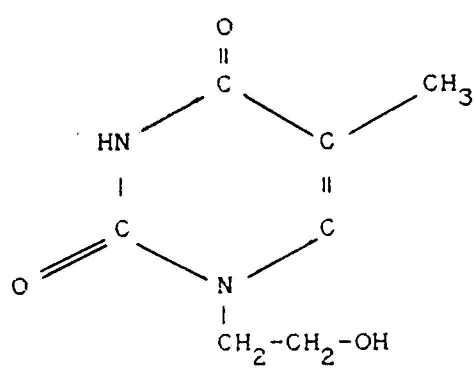

On réalise la condensation de la N,1-hydroxyéthylthymine sur

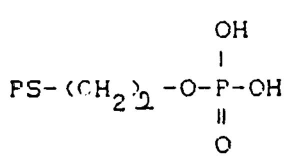

avec 0,95 méq/g de substitution en ⟨⟨hydroxyéthylphosphate hydroxyéthylthymine⟩⟩ (soit 12% en masse) sur le polystyrène, tout en laissant 1,8 méq/g de substitution en ⟨⟨hydroxyéthylphosphate⟩⟩ - (soit 42% en masse).

### Exemple 4

### Fixation de bases hydroxyéthylphosphate sur le poly(parahydroxyéthylstyrène)

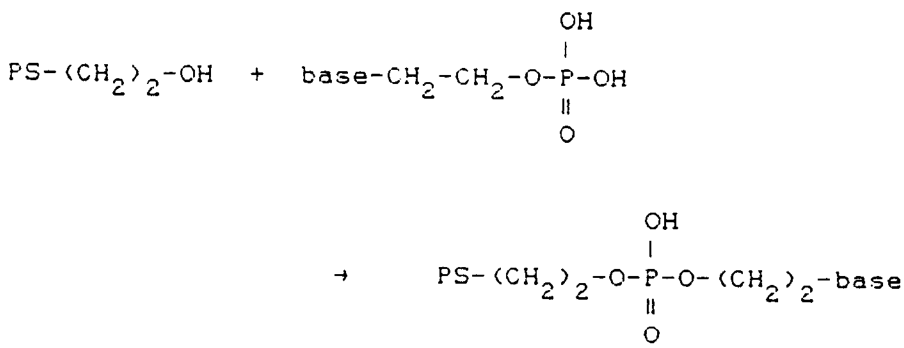

Dans le cas où la base est l'adénine, on commence par préparer l'adénine N-9,hydroxyéthyl phosphate de formule :

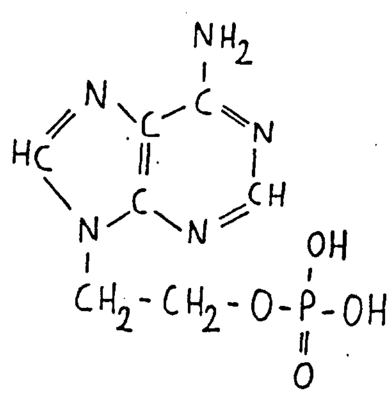

A cet effet, on réalise la phosphorylation, avec $POCl_3$, de la N,9-hydroxyéthyladénine, puis on condense le produit obtenu avec le poly(hydroxyéthylstyrène) à l'aide de la N,N'-dicyclohexylcarbodiimide, comme décrit à l'Exemple 3.

On a ainsi obtenu 1,03 méq/g de substitution (analyse identique avec P ou N), soit 40% en masse de la molécule contenant le phosphodiester et l'adénine, le reste de la résine étant constitué par du polystyrène substitué par des groupes hydroxyéthyle et/ou hydroxyméthyle, et par du polystyrène non substitué.

**Exemple 5**

**Préparation de la résine :**

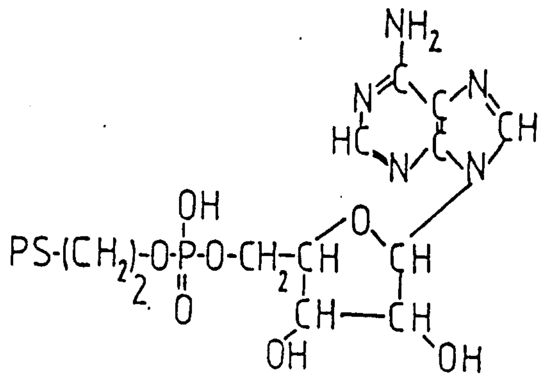

**A : Fixation d'adénosine sur la résine** $PS-(CH_2)_2-O-\overset{\overset{O}{\parallel}}{\underset{\underset{OH}{|}}{P}}-OH$

La résine (2,5 méq) est mise à gonfler dans 18 ml de D. M. F. On ajoute alors 1,3 g d'adénosine et 10 ml d'eau. Le ballon est porté a 105 ° C pendant 2 heures et demi. Après 2 évaporations sous vide et remise en suspension dans du D.M.F. anhydre, on ajoute 7 g de DCCI avec 50 ml de D.M.F. anhydre et on laisse l'ensemble 18 heures à 110 ° C. La résine est successivement lavée avec du D.M.F. et de l'éthanol chaud.

La condensation de l'adénosine donne 0,34 méq/g de substitution (soit 16,3%) et 2,54 méq/g de poly-(hydroxy éthyl phosphate)styrène (58%).

**B : Fixation d'adénosine monophosphate sur $PS-(CH_2)_2-OH$**

On procède de la même façon qu'à l'Exemple 5 A, en mettant en contact 0,4 g de résine (2,4 méq) avec 0,9 g d'adénosine monophosphate (2,6 méq).

On obtient ainsi 8% de substitution avec ainsi la même proportion de groupements phosphate, de sucre et de base. Le reste de la résine est constitué de motifs hydroxyle.

TABLEAU III - RESULTATS DE MICROANALYSE

| Exemples | Résines | % C | % H | % O | % N | % Cl | % Na | % S | % P | méq/g | % de substitution |
|---|---|---|---|---|---|---|---|---|---|---|---|
| A | $PS-CH_2Cl$ | 72 | 6,1 | | | 20,8 | | | | 5,9 | 90 |
| C | $PS-CH_2OH$ | 79,4 | 7 | 10 | | 0,5 | | | | 6,3 | 84 |
| D (1ère étape) | $PS-CH_2-C{\equiv}N$ | 82,2 | 6,5 | 2 | 8,1 | <0,1 | | | | 5,8 | 83 |
| D (2ème étape) | $PS-CH_2-COOH$ | 75,8 | 6,5 | 17,6 | ⩽0,1 | <0,2 | | | | 5,5 | 89 |
| E (1ère étape) | 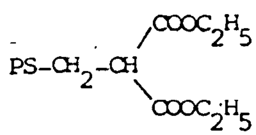 | 74 | 7,4 | 17,9 | | 0,2 | | | | 2,8 | 77 |
| E (2ème étape) | $PS-(CH_2)_2-COOH$ | 74,1 | 6,9 | 16,4 | | | | | | 5,1 | 89 |
| G | $PS-SO_2-NH-(CH_2)_2-OH$ | 50 | 5,8 | 25 | 5,21 | 0,5 | | 13,1 | | 3,7 | 84 |
| G | $PS-SO_2-NH-(CH_2)_3-OH$ | 53 | 6,3 | 24 | 4,9 | <0,2 | | 12,8 | | 3,5 | 84 |
| H | $PS-SO_2-NH-CH_2-CHOH-CH_2OH$ | 46,7 | 6 | 30 | 3,6 | <0,2 | | 12,6 | | 2,6 | 67 |
| F1 | $PS-(CH_2)_2-OH$ | 80,6 | 7,9 | 9,6 | | 0,2 | | | | 6,0 | 88 |
| F2 | $PS-(CH_2)_3-OH$ | 80,2 | 7,7 | 8,3 | | 0,3 | | | | 5,2 | 84 |
| 1 | 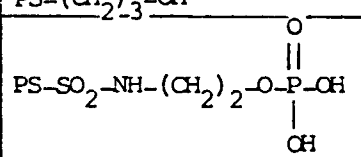 | 39,5 | 5 | 27,8 | 4,7 | 0,6 | | 10,1 | 7,8 | 2,5 | 77 |

TABLEAU III (suite) - RESULTATS DE MICROANALYSE

| Exemples | Résines | % C | % H | % O | % N | % Cl | % P | méq/g | % de substitution* |
|---|---|---|---|---|---|---|---|---|---|
| 1 | 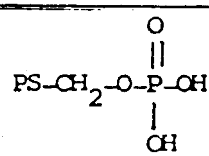 | 66,3 | 6,9 | 16 | 2,4 | 0,7 | 4,3 | 1,4 | 30 |
| 1 | 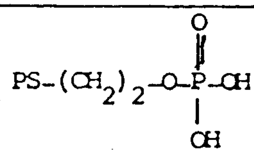 | 53,8 | 6,5 | 24 | | 2,6 | 10,5 | 3,4 | 78 |
| 1 | 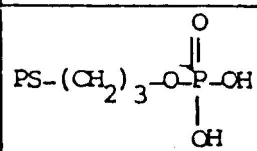 | 61,9 | 6,6 | 19,2 | | | 7,9 | 2,55 | 62 |
| 2 | PS-$CH_2$-guanine | 58,3 | 4,8 | 8,2 | 23,2 | 5,9 | | 3,3 | 89 |
| 2 | PS-$CH_2$-uracile | 71,8 | 5,7 | 13,1 | 7,2 | 1,9 | | 2,6 | 59 |
| 2 | PS-$CH_2$-thymine | 74,4 | 6,3 | 7 | 4,3 | | | 1,6 | 39 |
| 2 | PS-$CH_2$-cytosine | 70,4 | 6,4 | 8,8 | 9,8 | 3,4 | | 2,3 | 53 |
| 2 | PS-$CH_2$-adénine | 63,7 | 5,2 | | 20,7 | 5,2 | | 3 | 74 |

TABLEAU III (suite) - RESULTATS DE MICROANALYSE

| Exemples | Résines 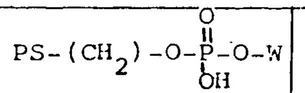 avec W = | | %C | %H | %O | %N | %Cl | %P | méq/g | % de substitution* |
|---|---|---|---|---|---|---|---|---|---|---|
| 3 | $-(CH_2)$-adénine | | 58,2 | 7,2 | 18,3 | 7 | 0,3 | 6 | 1,0 | 39 (et 20 en $-PO_4H_2$) |
| 3 | $-(CH_2)$-cytosine | | 59,3 | 6,9 | 19,6 | 3,9 | 0,4 | 6,8 | 0,93 | 34 (et 29 en $-PO_4H_2$) |
| 3 | $-(CH_2)$-thymine | | 63 | 7 | 16,6 | 1,9 | | 7,05 | 0,68 | 19 (et 36 en $-PO_4H_2$) |
| 4 | $-(CH_2)$-adénine | | 63,4 | 6,4 | 14,9 | 7,5 | 0,1 | 3,2 | 1,03 | 40 |
| 5 | 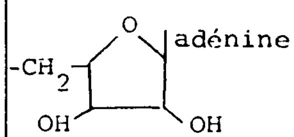 | A | 56,7 | 7 | 20,6 | 2,5 | 1,2 | 7,9 | 0,34 | 16 (et 58 en $-PO_4H_2$) |
| | | B | 76,1 | 7,5 | 12 | 11,9 | 0,48 | 0,49 | 0,17 | 8 |
| 6A | 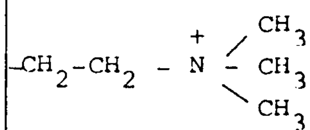 | a | 58,6 | 7,1 | 17,8 | 1,4 | 1,8 | 7,4 | 1,01 | 32 |
| | | b | 56,6 | 7,8 | 20,1 | 2,5 | 1,9 | 5,45 | 1,8 | 56 |
| 6B | 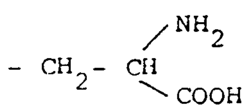 | | 67,7 | 7 | 15,2 | 2,34 | 0,2 | 5,0 | 1,6 | 50 |

* On a reporté ici les % en masse de sites substitués par la fonction désirée.

**Exemple 6 : Fixation de composants des phospholipides**

**A - Fixation de la phosphocholine sur le poly(para hydroxyéthyl styrène)**

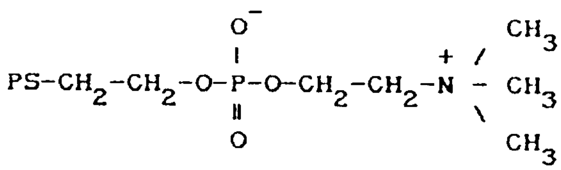

a) Première méthode

Comme il est décrit par H. J. LUCUS et al, ⟨⟨J. Am. Chem. Soc. 72, p 5491 (1950)⟩⟩, la réaction de $PCl_3$ avec l'éthylène glycol forme le 2-chloro-1,3-2-dioxaphospholane ; après oxydation dans le benzène anhydre, on obtient le 2-chloro-2-oxo-1,3-2-dioxaphospholane :

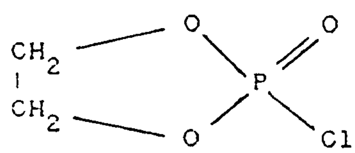

Ce composé est ensuite condensé à -10 ° C dans le dichlorométhane avec un polystyrène hydroxyéthyl. On obtient ainsi le motif :

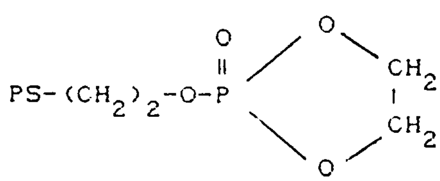

Le cycle est alors ouvert avec une solution à 20% de triméthylamine dans le T.H.F., afin d'obtenir 1 méq/g de substitution (soit 32%).

b) Deuxième méthode

On peut réaliser le même polymère en condensant sur le poly(hydroxyéthyl styrène), le bromoéthyl dichlorophosphate qui est obtenu par phosphorylation du bromoéthanol par l'oxychlorure de phosphore. Le polymère phosphorylé résultant est ensuite substitué par une solution de triméthylamine ; on obtient ainsi 1,8 méq/g de substitution (soit 56% de la masse de résine).

**B - Fixation de la phosphosérine sur le poly(para hydroxyéthyl styrène)**

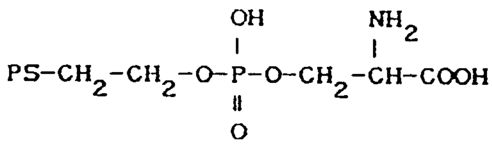

La résine (5,5 méq) est mise à gonfler dans 30 ml de N,N'-diméthylformamide (D.M.F.) pendant une heure. 1 gramme de phosphosérine dans 5 ml d'eau est alors ajouté. On porte la suspension à 100 °C pendant 2 heures. Après deux évaporations sous vide et remise en suspension dans le D.M.F. anhydre, 10 g de D.C.C.I. dans 20 ml de D.M.F. anhydre sont additionnés. Après 18 heures à 110 °C, la résine est lavée avec de la D.M.F. et de l'éthanol chaud.

La condensation de la phosphosérine donne 1,6 méq/g de substitution (analyse identique pour le phosphore et l'azote), soit 50% de la masse de la résine.

**Exemple 7 : Préparation d'une résine carboxyméthyl Sephadex® phosphorylé**

La résine Sephadex® de base présente la structure chimique suivante :

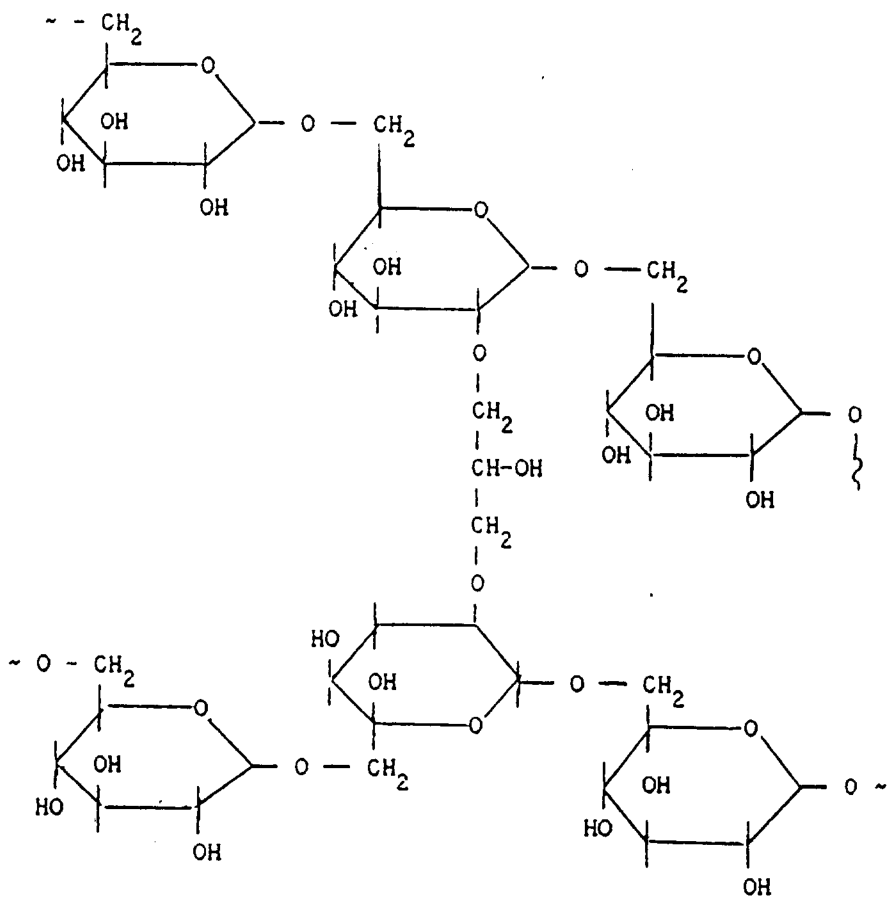

Elle résulte de la réticulation, par l'épichlorhydrine, d'un dextrane de faible polydispersité. Elle est utilisée ici à l'état en partie substitué par des groupes carboxyméthyle.

**Première étape : Préparation d'une résine carboxyméthyl Sephadex® aminohydroxylée.**

Mode opératoire A : Formation du chlorure d'acide de la résine de départ, puis condensation d'une amine hydroxylée.

10 g de carboxyméthyl Sephadex® réticulé (Pharmacia) - ci-après désigné comme CM Sephadex®, à 4 méq de COOH/g, sont mis à gonfler dans 150 ml de benzène (solution 1). 4,5 ml de chlorure de thionyle sont mis en solution dans 50 ml de benzène (solution 2). La solution 2 est ajoutée lentement à la solution 1. La température du milieu réactionnel est portée progressivement à une valeur de 50 à 70 ° C. La réaction dure 24 heures. Le produit est filtré et rincé rapidement avec du benzène sec, puis essoré.

On effectue alors une condensation d'éthanolamine, selon le schéma réactionnel suivant :

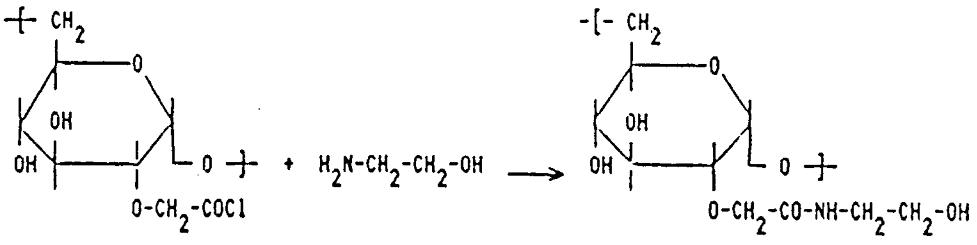

10 g du chlorure d'acide du CM Sephadex®, correspondant à environ 40 méq de chlore, sont placés en suspension dans 150 ml de pyridine. De l'éthanolamine, en excès molaire de 3 par rapport aux fonctions chlorure d'acide (soit 7 ml), est alors ajoutée au milieu réactionnel (résine + pyridine), et la température est portée progressivement à 70 ° C : à 35-40 ° C pendant une heure, en faisant suivre par un chauffage à 72 ° C pendant 24 heures. Le produit est filtré, rincé à l'eau, lavé à l'eau pour hydrolyser les chlorures restants, lavé en solutions $H_2O$/éthanol de concentration croissant es éthanol, puis il est essoré et séché sous vide, à 50 ° C, jusqu'à poids constant.

Mode opératoire B : Couplage à la N-éthoxycarbonyl-2-éthoxy-1,2-dihydroquinoléine (EEDQ) puis fixation d'une amine hydroxylée.

*Etape 1* : Couplage à l'EEDQ

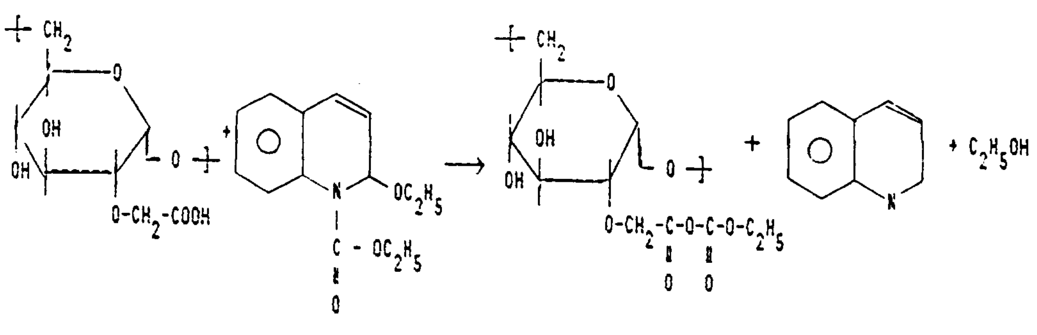

10 g de CM Sephadex® réticulé sont placés en suspension dans 70 ml d'eau. Le pH est ajusté à 3 par addition d'HCl 1 N. On ajoute alors lentement une solution de 20 g d'EEDQ (ce qui correspond à environ 80 méq) dans 160 ml d'éthanol. Le milieu réactionnel doit rester sous agitation, à température ambiante pendant 30 minutes.

*Etape 2* : Fixation de l'amine

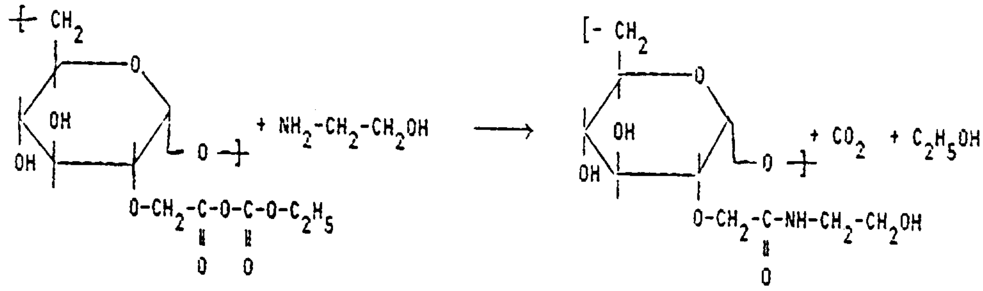


80 méq d'éthanolamine sont alors additionnés au milieu réactionnel précédent (résine-$H_2O$-EtOH-EEDQ), très lentement, avec contrôle de la stabilité du pH entre chaque ajout. Le pH est contrôlé à une valeur d'approximativement 9,3 pendant toute la durée de la réaction, c'est-à-dire pendant 20 heures, à température ambiante.

Le produit est filtré, puis lavé en mélanges $H_2O$/EtOH de concentrations croissantes en éthanol (jusqu'à 100%), puis au méthanol avant d'être essoré puis séché sous vide a 50 ° C jusqu'à poids constant.

Dans ces deux modes opératoires, on peut conduire la réaction avec d'autres amines hydroxylées, telles que propanolamine et hexanolamine, auquel cas le bras espaceur sera différent.

**Deuxième étape : Préparation du composé de l'intitulé (phosphorylation analogue à l'Exemple 1C)**

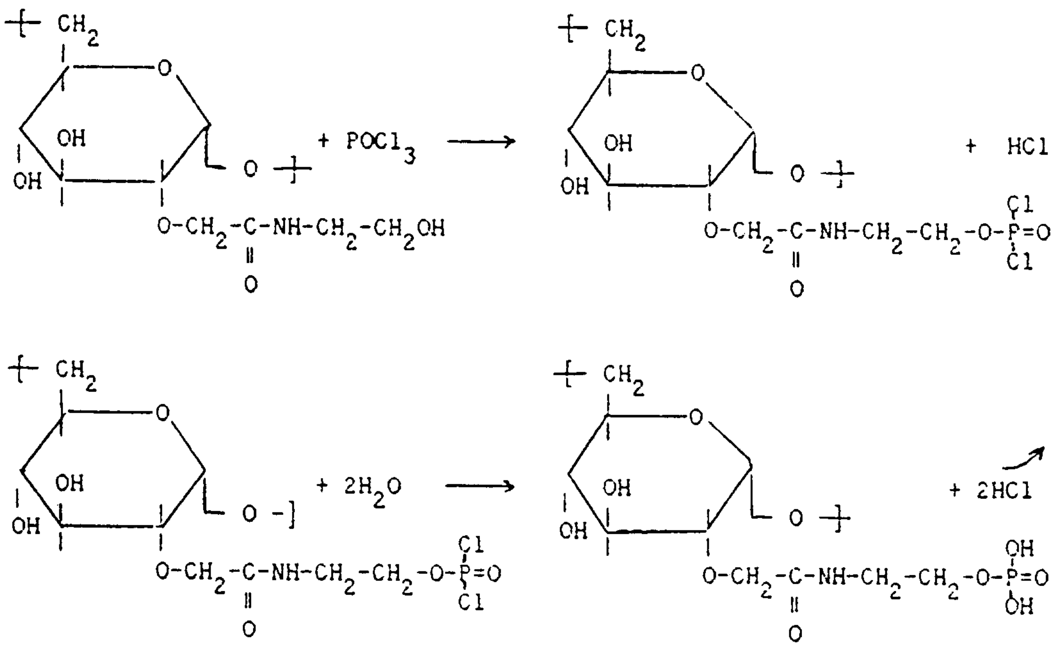


10 g de CM Sephadex® aminohydroxylé sec (à 4 méq/g) sont placés en suspension de triméthylphosphate $PO(OCH_3)_3$. On laisse gonfler la résine dans le solvant pendant 1 à 2 heures à la température ambiante. Puis de l'oxychlorure de phosphore $POCl_3$ (45 méq) ajouté au milieu résine-solvant. L'ensemble est porté à 60 ° C, et la durée de la réaction est de 18 heures. Le produit phosphorylé est alors hydrolysé par addition de quelques millilitres d'eau ; il est ensuite filtré et lavé avec des mélanges $H_2O$/dioxanne de concentrations (1)/(2), puis (2)/(1), puis avec du dioxanne pur et du méthanol. Le produit obtenu est alors essoré puis séché sous vide à 50 ° C.

**Exemple 8 :**

Dans cet Exemple, on décrira la courbe de titration qui est représentée sur la figure 1 et qui est obtenue avec une résine

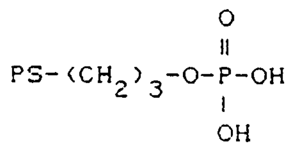

(substitution en groupements phosphate : 60%), obtenue de la même manière qu'aux Exemples $F_2$ (pour la fixation du bras espaceur) et 1 (pour la phosphorylation). Le produit titrant est la soude. On constate que la zone de pH qui définit les conditions optimales d'utilisation de cette résine comme support d'échange d'ions est centrée autour de pH 7. Dans ces conditions, une telle résine favorise la séparation de protéines sans risque de dénaturation de ces dernières.

**Exemple 9 :**

Cet Exemple illustre l'application de la résine de l'Exemple 8 comme support chromatographique.

Les figures 2 à 5 sont des chromatogrammes obtenus en utilisant la résine de l'Exemple 8 dans une colonne en acier inoxydable de 250 x 4 mm, avec un système de chromatographie liquide haute performance. L'élution des protéines se fait soit en tampon citrate 0,16 M pH = 5, soit en tampon Tris 0,05 M pH = 7,6 et cela à un débit de 0,1 ou 0,2 ml/mn.

Cette résine a permis, en particulier, la séparation par chromatographie liquide haute performance d'un mélange constitué de Myoglobine et de Cytochrome C, comme le montre la figure 6.

*Légendes des figures* :

2 : Cytochrome C en tampon citrate 0,16 M, pH = 5, débit : 0,2 ml/mn, temps d'élution : 7,96 mn.

3 : Cytochrome C en tampon Tris 0,05 M, NaCl de 0 à 2M, pH = 7,6, débit : 0,1 ml/mn, temps d'élution: 68,38 mn.

4 : Ribonucléase en tampon citrate 0,16 M, pH = 5, débit : 0,2 ml/mn, temps d'élution : 8,53 mn.

5 : Albumine en tampon citrate 0,16 M, NaCl 2 M, pH = 5, débit : 0,1 ml/mn, temps d'élution : 18,5 mn.

6 : Cytochrome C + Myoglobine en tampon Tris 0,05 M, pH = 7,6, débit : 0,1 ml/mn, NaCl de 0 à 2 M temps d'élution : Myoglobine = 14,12 mn ; Cytochrome C = 71,06 mn.

**Exemple 10 :**

Cet Exemple illustre l'utilisation, comme immunoadsorbant spécifique, de la résine :

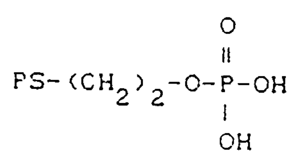

(substitution en groupements phosphate : 70%), obtenue de la même manière qu'aux Exemples $F_2$ (pour la fixation du bras espaceur) et 1 (pour la phosphorylation).

La figure 7 montre l'adsorption d'anticoagulants circulants (A.C.C.) d'un sérum lupique sur une résine porteuse de groupes phosphate. Sur la courbe sont exprimés, an ordonnées, le temps de Céphaline - Kaolin (T.C.K.) en secondes et, en abscisses, la dilution du sérum dans du tampon.

L'allongement du temps de coagulation dû aux A.C.C. n'existe plus si le sérum lupique a été au préalable incubé avec la résine. La courbe est alors identique à celle d'un sérum humain normal.

*Sur la figure 7* :

▲ : Sérum lupique

△ : Sérum lupique incubé avec la résine de l'invention

+ : Sérum humain normal.

De plus, le taux d'anticorps anti-ADN de ce sérum mesuré par le test de FARR, n'a pa varié indiquant que l'adsorption des A.C.C. sur cette résine est hautement spécifique.

**Exemple 11 :**

Cet exemple illustre l'utilisation, comme immunoadsorbant spécifique, de la résine :

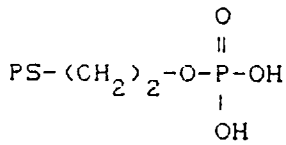

(substitution en groupements phosphate: 30%), obtenue de la même manière qu'à l'Exemple 10.

La figure 8 montre l'isotherme d'adsorption d'IgG anti-ADN d'un sérum lupique sur une résine porteuse de groupements phosphate. Sur les courbes, sont exprimées, en ordonnées, les concentrations molaire; des IgG anti-ADN adsorbées sur la résine à l'équilibre, et, en abscisses, la concentration des mêmes IgG dans le sérum lupique pour différentes quantités de polymère mises en suspension dans le sérum.

*Sur la figure 3* :

● : 322 g de polymère par 1 de sérum
+ : 215 g de polymère par 1 de sérum
♦ : 107 g de polymère par 1 de sérum
▲ : 54 g de polymère par 1 de sérum

**Exemple 12 :**

Cet exemple illustre l'utilisation, comme phase stationnaire en chromatographie d'affinité, de la résine :

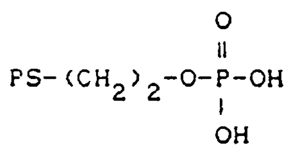

(substitution en groupements phosphate: 64%), obtenue de la même manière qu'à l'Exemple 10, pour la purification d'un facteur de RNA-Polymérase B requis pour la transcription de l'ADN.

Dans cet exemple, un extrait cellulaire contenant le facteur de transcription UEF du promoteur majeur tardif de l'adénovirus-2 et d'autres polymères biologiques en solution dans un tampon Tris-HCl 50 mM à pH 7,9, 50 mM KCl est déposé à 4°C sur une colonne chromatographique constituée de 300 µl de la résine préalablement équilibrée dans le même tampon.

Une élution effectuée avec une solution tampon Tris-HCl 50 mM à pH 7,9, KCl 1 M, permet d'obtenir le facteur UEF à un haut degré de pureté. La caractérisation du facteur est effectuée par deux méthode :

- électrophorèse en gel de polyacrylamide qui révèle la présence du facteur UEF purifié ;
- mise en évidence de l'activité biologique du facteur UEF selon la méthode décrite par V. Moncollin et Coll., Embo Journal, Vol. 5, page 2577-2584 (1986).

Cet exemple démontre que la résine fixe spécifiquement le facteur UEF à l'exclusion des autres constituants du complexe de transcription pourtant présents dans l'extrait cellulaire initial, et illustre les possibilités d'utilisation de la résine en chromatographie d'affinité pour la purification de polymères d'origine biologique.

**Exemple 13 :**

Cet exemple illustre l'utilisation, comme immunoadsorbant spécifique, des polystyrènes phosphorylés obtenus de la même manière qu'à l'Exemple 10.

La figure 9 représente l'adsorption des anticorps anti-ADN d'un sérum lupique à quatre dilutions sur des polystyrènes phosphorylés à différents taux de substitution en groupement phosphate.

*Légende de la figure 9* :

Abscisses : taux de substitution en groupements phosphate (%)
Ordonnées : pourcentage d'anticorps anti-ADN adsorbés
□ : sérum à 100%
♦ : sérum à 80%
■ : sérum à 60%
X : sérum à 40%

Les résultats obtenus indiquent qu'il existe un taux de phosphate optimal (voisin de 30%) pour l'adsorption spécifique des anticorps anti-ADN sur ce type de résine.

**Exemple 14 :**

Cet exemple illustre l'utilisation des polymères synthétisés en tant que supports pour le dosage d'anticorps.

Dans cet exemple, les résines obtenues de la même manière qu'à l'Exemple 10 (substitution à 58 ; 64 et 71%) sont incubées pendant 1/2 heure à 4°C avec trois catégories de plasmas : la première est constituée de plasma humain normal : la seconde, de plasma lupique contenant des anticoagulants circulants (A.C.C.) ; et la dernière, de plasma lupique avec anticorps anti-ADN.

Après centrifugation, élimination du surnageant et lavages des polymères en tampon borate 0,1 M - sérum albumine bovine (BSA) 0,2% (pH = 8,4), on ajoute à la résine une solution d'anticorps anti-Fc humain radiomarqué à l'iode 125. Après 4 heures à 4°C sous agitation, on effectue les mêmes opérations de lavages que précédemment. La résine est alors introduite dans un compteur gamma.

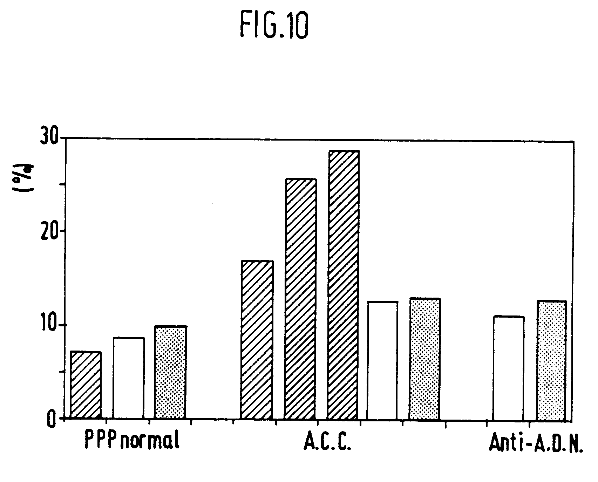

Les quantités d'anticorps anti-Fc humain fixées sur les résines sont alors plus importantes dans le cas des incubations avec les plasmas lupiques (figure 10), notammment avec ceux contenant des A.C.C.. Ceci vérifie bien que les anticorps lupiques sont adsorbés spécifiquement par leur fragment Fab, laissant ainsi leur fragment Fc libre.

La quantification des anticorps fixés spécifiquement est alors directement possible. De plus, le dosage des anticorps présents dans le plasma des malades lupiques est ainsi effectué à l'aide de ces résines.

*Légende de la figure 10* :

Ordonnées : pourcentage d'IgG anti-Fc adsorbées
▨ : résine à 71% de groupes phosphate
□ : résine à 58% de groupes phosphate
▩ : résine à 64% de groupes phosphate

**Revendications**

**Revendications pour les Etats contractants suivants : AT, BE, CH, LI, DE, FR, GB, GR, IT, LU, NL, SE**

**1.** Polymère dérivé d'un polymère ou copolymère réticulé du styrène, ou d'un dextrane réticulé, caractérisé par le fait que la chaîne du polymère ou copolymère de base est substituée par un ou plusieurs groupes, identiques ou différents, appartenant aux catégories suivantes :

$-Z-A_1$ ;
$-Z-A_2$ ;
$-Z-A_1-Z'-A_2$ ;
$-Z-A_1-A_3-A_2$ ;
$-Z-A_1-A_4$

où :
- Z représente une chaîne d'espacement ;
- Z' représente une chaîne de liaison ;
- $A_1$ représente un reste phosphate ;
- $A_2$ représente le reste d'une base purique ou d'une base pyrimidique ;
- $A_3$ représente un reste de sucre ; et
- $A_4$ représente un reste d'une molécule intervenant dans la structure polaire des divers phospholipides, à l'exclusion des polymères uniquement substitués par des groupes $-Z-A_1-A_3-A_2$.

**2.** Polymère selon la revendication 1, caractérisé par le fait que Z est choisi parmi les restes :

- $-(CH_2)_n-$ , n valant de 1 à 6, éventuellement rendu hydrophile par remplacement d'au moins un H par un OH ; ou
- $-SO_2-NH-(CH_2)_m-$, m valant de 1 à 6, le reste $-(CH_2)_m-$ étant éventuellement rendu hydrophile par remplacement d'au moins un H par un OH ; et
- dans le cas de la modification d'un dextrane réticulé, également

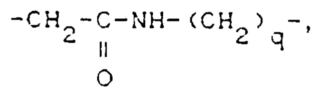

q valant de 1 à 6, le reste $-(CH_2)_q-$ étant éventuellement rendu hydrophile par remplacement d'au moins un H par un OH.

**3.** Polymère selon la revendication 2, caractérisé par le fait que Z est choisi parmi les restes $-CH_2-$ ; $-(CH_2)_2-$ ; $-(CH_2)_3-$ ; $-SO_2-NH-(CH_2)_{2ou3}-$ ; $-SO_2-NH-CHOH-$ ; et $-SO_2-NH-CH_2-CHOH-CHOH-$ ; et, dans le cas de la modification d'un dextrane réticulé, également,

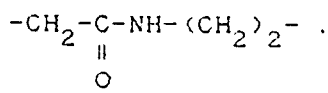

**4.** Polymère selon l'une des revendications 1 à 3, caractérisé par le fait que Z'représente $-(CH_2)_p-$, p valant de 1 à 6, ce reste étant éventuellement rendu hydrophile par remplacement d'au moins un H par un OH.

**5.** Polymère selon l'une revendications 1 à 4, caractérisé par le fait que $A_2$ représente le reste d'une base purique choisie parmi l'adénine et la guanine, ou le reste d'une base pyrimidique choisie parmi la cytosine, la thymine et l'uracile.

**6.** Polymère selon l'une des revendications 1 à 5, caractérisé par le fait que $A_3$ représente le reste d'un pentose, et, notamment, du D-ribose ou du 2-désoxy-D-ribose, relié par son groupement $-CH_2-$ au reste phosphate

**7.** Polymère selon l'une des revendications 1 à 6, caractérisé par le fait que $A_4$ représente une molécule estérifiée de choline, d'éthanolamine, de sérine, de glycérol ou d'inositol.

**8.** Polymère selon l'une des revendications 1 à 7, caractérisé par le fait que le polymère de base est un polymère ou un copolymère réticulé du styrène et que la substitution de la chaîne du polystyrène s'effectue en position para du noyau phényle.

**9.** Polymère selon l'une des revendications 1 à 8, caractérisé par le fait que le polymère de base est un polymère ou un copolymère réticulé du styrène et que chaque groupe phényle non impliqué dans la réticulation peut porter un substituant tel que défini a la revendication 1.

**10.** Polymère selon l'une des revendications 1 à 9, caractérisé par le fait que le polymère de base est un polymère ou un copolymère réticulé du styrène et qu'il a été réticulé avec au plus 5% en poids d'au moins un monomère de réticulation, comme le divinylbenzène.

**11.** Polymère selon l'une des revendications 1 à 10, caractérisé par le fait qu'il résulte de la modification d'un polystyrène de base se présentant sous la forme de billes d'une dimension voisine de 50 $\mu$m.

**12.** Polymère selon l'une des revendication 1 à 7, caractérisé par le fait qu'il résulte de la modification d'un dextrane réticulé par l'épichlorhydrine, ledit dextrane portant éventuellement des substituants carboxyméthyle.

**13.** Procédé de préparation du polymère dérivé du (co)polymère réticulé du styrène, ou du dextrane réticulé, tel que défini à l'une des revendications 1 à 12, caractérisé par le fait qu'il comporte la fixation sur le polymère ou copolymère réticulé de base (polystyrène ou dextrane), en une ou plusieurs étapes, un reste -Z-OH, ou, dans le cas du polymère ou copolymère du styrène, d'un reste -Z-X (où X représente un halogène et Z est tel que défini à la revendication 1), puis

(I) dans le cas où l'on a fixé un reste -Z-X, la réaction du polystyrène ainsi modifié avec une base purique ou pyrimidique, afin de former le substituant $-Z-A_2$;

(II) dans le cas où l'on a fixé un reste -Z-OH,

(IIa) la phosphorylation du polymère ou copolymère ainsi modifié, afin de former le substituant $-Z-A_1$; puis

- lorsque l'on souhaite former le substituant $-Z-A_1-Z'-A_2$, la réaction du reste $-Z-A_1$ avec une base purique modifiée, schématisée par la formule :

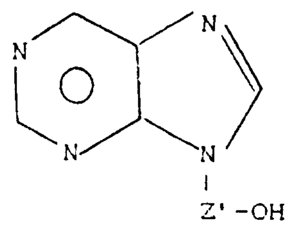

(I)

ou avec une base pyrimidique modifiée schématisée par la formule :

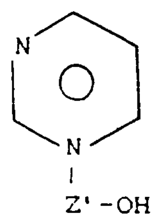

(II)

- lorque l'on souhaite former le substituant $-Z-A_1-A_3-A_2$, la réaction du reste $-Z-A_1$ avec un nucléoside de formule $-A_3-A_2$, résultant de la liaison de l'azote 9 d'une base purique ou de l'azote 1 d'une base pyrimidique avec le carbone 1' d'un sucre ;
- lorsque l'on souhaite former le substituant $-Z-A_1-A_4$, la réaction du groupement $-Z-A_1$ avec une molécule intervenant dans la structure polaire des divers phospholipides ; ou bien

(IIb) lorsque l'on souhaite former directement le substituant $-Z-A_1-A_3-A_2$, la réaction du polymère ou copolymère ainsi modifié avec un nucléoside monophosphate de formule $A_1-A_3-A_2$ ;

(IIc) lorsque l'on souhaite former le substituant $-Z-A_1-Z'-A_2$, la réaction du polymère ou copolymère ainsi modifié avec une base purique transformée, schématisée par la formule :

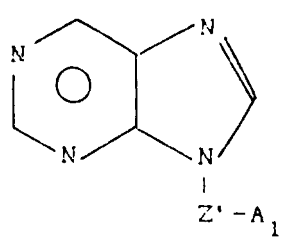

(Ia)

et qui est obtenue par phosphorylation de la base purique modifiée de formule (I), ou avec une base pyrimidique transformée, schématisée par la formule :

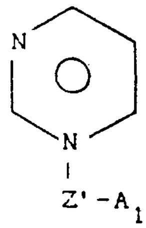 (IIa)

et qui est obtenue par la phosphorylation de la base pyrimidique modifiée de formule (II) ; ou bien
(IId) lorsque l'on souhaite former le substituant $-Z-A_1-A_4$, la réaction du polymère ou copolymère ainsi modifié avec un reste $A_4$ phosphorylé.

**14.** Procédé selon la revendication 13, caractérisé par le fait qu'on utilise, comme polymère de départ, un polystyrène réticulé, et qu'on commence par fixer sur ledit polystyrène le radical $-CH_2-X$ en faisant réagir ledit polystyrène avec un halométhyl méthyl éther en présence de chlorure stannique.

**15.** Procédé selon la revendication 13, caractérisé par le fait qu'on utilise, comme polymère de départ, un polystyrène réticulé, et qu'on commence par fixer sur ledit polystyrène le radical $-CH_2-OH$, en faisant réagir, en présence d'un catalyseur transfert de phase, la résine obtenue par le procédé selon la revendication 13 avec l'acétate de potassium, puis en hydrolysant l'acétate obtenu par la potasse concentrée.

**16.** Procédé selon la revendication 13, caractérisé par le fait qu'on utilise, comme polymère de départ, un polystyrène réticulé, et qu'on commence par fixer sur ledit polystyrène, le radical $-CH_2-CH_2-OH$,
- en faisant réagir la résine obtenue par le procédé selon la revendication 13 avec le cyanure de sodium, en présence d'un catalyseur transfert de phase, pour obtenir le substituant $-CH_2-C{\equiv}N$ ;
- puis en faisant réagir le polystyrène ainsi modifié avec l'acide chlorhydrique en milieu alcoolique, afin d'obtenir le substituant $-CH_2-COOH$ ;
- puis en réduisant par le diborane afin d'obtenir le substituant $-CH_2-CH_2-OH$.

**17.** Procédé selon la revendication 13, caractérisé par le fait qu'on utilise, comme polymère de départ, un polystyrène réticulé, et qu'on commence par fixer sur ledit polystyrène, le radical $-CH_2-CH_2-CH_2-OH$,
- en condensant un malonate d'alkyle sur la résine obtenue par le procédé selon la revendication 13, afin d'obtenir le substituant :

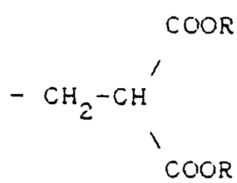

(R étant un reste alkyle) ;
- puis, en hydrolysant la fonction diester ainsi obtenue pour donner une fonction diacide à laquelle on fait subir une décarboxylation en milieu acide, afin d'obtenir le substituant $-CH_2-CH_2-COOH$ ;
- puis en réduisant par le diborane afin d'obtenir le substituant $-CH_2-CH_2-CH_2-OH$.

**18.** Procédé selon 13 revendication 13, caractérisé par le fait qu'on utilise, comme polymère de départ, un polystyrène réticulé, et qu'on commence par fixer sur ledit polystyrène, le radical $-SO_2-NH-(CH_2)_{2\ ou\ 3}-OH$,
- en faisant agir l'acide chlorosulfonique sur le polystyrène de départ, de façon à obtenir le substituant $-SO_2Cl$,
- puis, en faisant agir l'éthanolamine ou la propanolamine sur le substituant précité.

**19.** Procédé selon la revendication 13, caractérisé par le fait qu'on utilise, comme polymère de départ, un polystyrène réticulé, et qu'on commence par fixer sur ledit polystyrène, le radical $-SO_2-NH-CH_2-CHOH-CH_2OH$,

- en faisant réagir l'acide chlorosulfonique sur le polystyrène de départ de façon d'obtenir le substituant $-SO_2Cl$ ;
- puis, en faisant agir l'amino-3 propanediol-1,2 sur le substituant précité.

**20.** Procédé selon la revendication 13, caractérisé par le fait qu'on utilise, comme polymère de départ, un dextrane réticulé éventuellement partiellement substitué par des groupes carboxyméthyle, et qu'on y fixe le radical $-NH-(CH_2)_q-OH$, q allant de 1 à 6, par réaction avec un halogénure de thionyle puis par réaction avec une amine de formule $OH-(CH_2)_q-NH_2$, ou bien à l'aide d'un agent de couplage, par condensation avec ladite amine hydroxylée.

**21.** Procédé selon l'une des revendications 13 à 20, caractérisé par le fait qu'on réalise la phosphorylation à l'aide du dichlorophosphate de méthyle, de l'acide phosphoreux, ou de l'oxychlorure de phosphore, et notamment à l'aide de l'oxychlorure de phosphore.

**22.** Procédé selon l'une des revendications 13 à 21, caractérisé par le fait qu'on prépare les bases modifiées des formules (I) ou (II) dans lesquelles Z' = $-CH_2-CH_2-$ en faisant réagir ladite base avec le carbonate d'éthylène en présence d'un catalyseur transfert de phase.

**23.** Procédé selon l'une des revendications 13 à 22, caractérisé par le fait qu'on réalise la réaction du reste $-Z-A_1$ sur les bases modifiées des formules (I) ou (II) à l'aide d'un agent de couplage, tel que la N,N'-dicyclohexylcarbodiimide.

**24.** Procédé selon l'une des revendications 13 à 22, caractérisé par le fait qu'on prépare une base modifiée de formule (Ia) ou (IIa) en phosphorylant avec $POCl_3$ la base correspondante de formule (I) ou (II), puis on condense ladite base de formule (Ia) ou (IIa) sur le substituant -Z-OH à l'aide d'un agent de couplage, tel que la N,N'-dicyclohexylcarbodiimide.

**25.** Utilisation du polymère dérivé d'un polymère ou copolymère réticulé du styrène, ou d'un dextrane réticulé, tels que définis à l'une des revendications 1 à 12, comme phase stationnaire en chromatographie d'échange d'ions.

**26.** Utilisation du polymère dérivé d'un polymère ou copolymère réticulé du styrène, ou d'un dextrane réticulé, tels que définis à l'une des revendications 1 à 12, comme phase stationnaire en chromatographie d'affinité.

**27.** Utilisation selon la revendication 26, pour réaliser des épurations sélectives des différents types d'anticorps développés par les malades lupiques.

**28.** Utilisation selon l'une des revendications 25 et 26, pour la purification d'enzymes, de co-enzymes ou de complexes enzymatiques, admettant comme substrats, l'ADN, l'ARN et les nucléotides.

**29.** Utilisation selon l'une des revendications 25 et 26 pour la purification à partir de divers extraits plasmatiques, des facteurs de la coagulation sanguine.

**30.** Utilisation du polymère dérivé d'un polymère ou copolymère réticulé du styrène, ou d'un dextrane réticulé, tels que définis à l'une des revendications 1 à 12, pour la purification des molécules d'origine biologique, telles que les protéines intervenant au cours du processus de la coagulation sanguine, et dans des systèmes d'analyse biologique, tels que RIA, ELISA, électrophorèse, immunoélectrophorèse mono- et bi-dimensionnelle, électrofocalisation.

**31.** Utilisation selon la revendication 30, pour réaliser les dosages des différents types d'anticorps lupiques, et, notamment, le dosage des anticoagulants circulants et/ou des anti-phospholipides.

**Revendications pour l'Etat contractant suivant : ES**

**1.** Procédé de préparation du polymère dérivé d'un polymère ou copolymère réticulé du styrène, ou d'un dextrane réticulé, dans lequel la chaîne du polymère ou copolymère de base est substituée par un ou plusieurs groupes, identiques ou différents, appartenant aux catégories suivantes :

$-Z-A_1$ ;
$-Z-A_2$;
$-Z-A_1-Z'-A_2$ ;
$-Z-A_1-A_3-A_2$ ;
$-Z-A_1-A_4$

où:
- Z représente une chaîne d'espacement ,
- Z' représente une chaîne de liaison ;
- $A_1$ représente un reste phosphate ;
- $A_2$ représente le reste d'une base purique ou d'une base pyrimidique ;
- $A_3$ représente un reste de sucre; et
- $A_4$ représente un reste d'une molécule intervenant dans la structure polaire des divers phospholipides, à l'exclusion des polymères uniquement substitués par des groupes $-Z-A_1-A_3-A_2$,

caractérisé par le fait qu'il comporte la fixation sur le polymère ou copolymère réticulé de base (polystyrène ou dextrane), en une ou plusieurs étapes, d'un reste -Z-OH, ou, dans le cas du polymère ou copolymère du styrène, d'un reste -Z-X (où X représente un halogène et Z est tel que défini ci-dessus, puis

(I) dans le cas où l'on a fixé un reste -Z-X, la réaction du polystyrène ainsi modifié avec une base purique ou pyrimidique, afin de former le substituant $-Z-A_2$;

(II) dans le cas où l'on a fixé un reste -Z-OH,

(IIa) la phosphorylation du polymère ou copolymère ainsi modifié, afin de former le substituant $-Z-A_1$; puis

* lorsque l'on souhaite former le substituant $-Z-A_1-Z'-A_2$, la réaction du reste $-Z-A_1$ avec une base purique modifiée, schématisée par la formule :

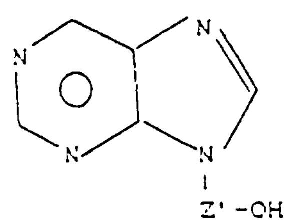

(I)

ou avec une base pyrimidique modifiée schématisée par la formule :

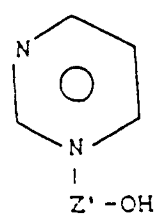

(II)

* lorsque l'on souhaite former le substituant $-Z_1-A_1-A_3-A_2$, la réaction du reste $-Z-A_1$ avec un nucléoside de formule $-A_3-A_2$,résultant de la liaison de l'azote 9 d'une base purique ou de l'azote 1 d'une base pyrimidique avec le carbone 1' d'un sucre;

* lorsque l'on souhaite former le substituant $-Z-A_1-A_4$, la réaction du groupement $-Z-A_1$ avec une molécule intervenant dans la structure polaire des divers phospholipides; ou bien

(IIb) lorsque l'on souhaite former directement le substituant $-Z-A_1-A_3-A_2$, la réaction du polymère ou copolymère ainsi modifié avec un nucléoside monophosphate de formule $A_1-A_3-A_2$;
(IIc) lorsque l'on souhaite former le substituant $-Z-A_1-Z'-A_2$, la réaction du polymère ou copolymère ainsi modifié avec une base purique transformée, schématisée par la formule :

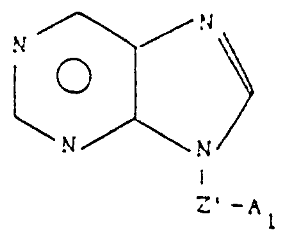

(Ia)

et qui est obtenue par phosphorylation de la base purique modifiée de formule (I), ou avec une base pyrimidique transformée, schématisée par la formule:

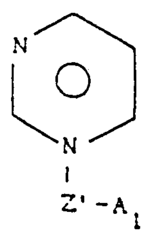

(IIa)

et qui est obtenue par la phosphorylation de la base pyrimidique modifiée de formule (II); ou bien
(IId) lorsque l'on souhaite former le substituant $-Z-A_1-A_4$, la réaction du polymère ou copolymère ainsi modifié avec un reste $A_4$ phosphorylé.

**2.** Procédé selon la revendication 1, caractérisé par le fait que Z est choisi parmi les restes :
- * $-(CH_2)_n-$, n valant de 1 à 6, éventuellement rendu hydrophile par remplacement d'au moins un H par un OH; ou
- * $-SO_2-NH-(CH_2)_m-$, m valant de 1 à 6, le reste -(CH2)m- étant éventuellement rendu hydrophile par remplacement d'au moins un H par un OH; et
- * dans le cas de la modification d'un dextrane réticulé, également

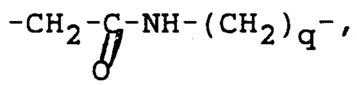

q valant de 1 à 6, le reste $-(CH_2)_q-$ étant éventuellement rendu hydrophile par remplacement d'au moins un H par un OH.

**3.** Procédé selon la revendication 2, caractérisé par le fait que Z est choisi parmi les restes $-CH_2-$; $-(CH_2)_2-$; $-(CH_2)_3-$; $-SO_2-NH-(CH_2)_{2 \text{ ou } 3}-$: $-SO_2-NH-CHOH-$; et $-SO_2-NH-CH_2-CHOH-CHOH-$; et, dans le cas de la modification d'un dextrane réticulé, également,

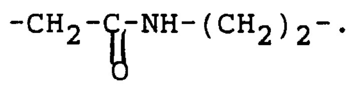

**4.** Procédé selon l'une des revendications 1 à 3, caractérisé par le fait que Z' représente $-(CH_2)_p-$, p valant de 1 à 6, ce reste étant éventuellement rendu hydrophile par remplacement d'au moins un H par un OH.

5. Procédé selon l'une des revendications 1 à 4, caractérisé par le fait que $A_2$ représente le reste d'une base purique choisie parmi l'adénine et la guanine, ou le reste d'une base pyrimidique choisie parmi la cytosine, la thymine et l'uracile.

6. Procédé selon l'une des revendications 1 à 5, caractérisé par le fait que $A_3$ représente le reste d'un pentose, et, notamment, du D-ribose ou du 2-désoxy-D-ribose, relié par son groupement $-CH_2-$ au reste phosphate.

7. Procédé selon l'une des revendications 1 à 6, caractérisé par le fait que $A_4$ représente une molécule estérifiée de choline, d'éthanolamine, de sérine, de glycérol ou d'inositol.

8. Procédé selon l'une des revendications 1 à 7, caractérisé par le fait que le polymère de base est un polymère ou un copolymère réticulé du styrène et que la substitution de la chaîne du polystyrène s'effectue en position para du noyau phényle.

9. Procédé selon l'une des revendications 1 à 8, caractérisé par le fait que le polymère de base est un polymère ou un copolymère réticulé du styrène et que chaque groupe phényle non impliqué dans la réticulation peut porter un substituant tel que défini à la revendication 1.

10. Procédé selon l'une des revendications 1 à 9, caractérisé par le fait que le polymère de base est un polymère ou un copolymère réticulé du styrène et qu'il a été réticulé avec au plus 5% en poids d'au moins un monomère de réticulation, comme le divinylbenzène.

11. Procédé selon l'une des revendications 1 à 10, caractérisé par le fait qu'il résulte de la modification d'un polystyrène de base se présentant sous la forme de billes d'une dimension voisine de 50 $\mu m$.

12. Procédé selon l'une des revendications 1 à 7, caractérisé par le fait qu'il résulte de la modification d'un dextrane réticulé par l'épichlorhydrine, ledit dextrane portant éventuellement des substituants carboxyméthyle.

13. Procédé selon l'une des revendications 1 à 12, caractérisé par le fait qu'on utilise, comme polymère de départ, un polystyrène réticulé, et qu'on commence par fixer sur ledit polystyrène le radical $-CH_2-X$ en faisant réagir ledit polystyrène avec un halométhyl méthyl éther en présence de chlorure stannique.

14. Procédé selon l'une des revendications 1 à 12, caractérisé par le fait qu'on utilise, comme polystyrène de départ, un polymère réticulé, et qu'on commence par fixer sur ledit polystyrène le radical $-CH_2-OH$, en faisant réagir, en présence d'un catalyseur transfert de phase, la résine obtenue par le procédé selon l'une des revendications 1 à 12 avec l'acétate de potassium, puis en hydrolysant l'acétate obtenu par la potasse concentrée.

15. Procédé selon l'une des revendications 1 à 12 caractérisé par le fait qu'on utilise, comme polymère de départ, un polystyrène réticulé, et qu'on commence par fixer sur ledit polystyrène, le radical $-CH_2-CH_2-OH$,
   * en faisant réagir la résine obtenue par le procédé selon l'une des revendications 1 à 12 avec le cyanure de sodium, en présence d'un catalyseur transfert de phase, pour obtenir le substituant $-CH_2-C{\equiv}N$;
   * puis en faisant réagir le polystyrène ainsi modifié avec l'acide chlorhydrique en milieu alcoolique, afin d'obtenir le substituant $-CH_2-COOH$;
   * puis en réduisant par le diborane afin d'obtenir le substituant $-CH_2-CH_2-OH$.

16. Procédé selon l'une des revendications 1 à 12 caractérisé par le fait qu'on utilise, comme polymère de départ, un polystyrène réticulé, et qu'on commence par fixer sur ledit polystyrène, le radical $-CH_2-CH_2-CH_2-OH$,
   * en condensant un malonate d'alkyle sur la résine obtenue par le procédé selon l'une des revendications 1 à 12 afin d'obtenir le substituant:

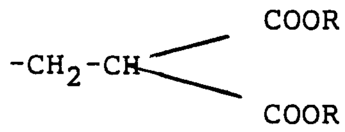

(R étant un reste alkyle);

* puis, en hydrolysant la fonction diester ainsi obtenue pour donner une fonction diacide à laquelle on fait subir une décarboxylation en milieu acide, afin d'obtenir le substituant $-CH_2-CH_2-COOH$;
* puis en réduisant par le diborane afin d'obtenir le substituant $-CH_2-CH_2-CH_2-OH$.

**17.** Procédé selon l'une des revendications 1 à 12 caractérisé par le fait qu'on utilise, comme polymère de départ, un polystyrène réticulé, et qu'on commence par fixer sur ledit polystyrène, le radical $-SO_2-NH-(CH_2)_{2\ ou\ 3}-OH$,

* en faisant agir l'acide chlorosulfonique sur le polystyrène de départ, de façon à obtenir le substituant $-SO_2Cl$,
* puis, en faisant agir l'éthanolamine ou la propanolamine sur le substituant précité.

**18.** Procédé selon l'une des revendications 1 à 12 caractérisé par le fait qu'on utilise, comme polymère de départ, un polystyrène réticulé, et qu'on commence par fixer sur ledit polystyrène, le radical $-SO_2-NH-CH_2-CHOH-CH_2OH$,

* en faisant réagir l'acide chlorosulfonique sur le polystyrène de départ, de façon à obtenir le substituant $-SO_2Cl$;
* puis, en faisant agir l'amino-3 propanediol-1,2 sur le substituant précité.

**19.** Procédé selon l'une des revendications 1 à 12 caractérisé par le fait qu'on utilise, comme polymère de départ, un dextrane réticulé éventuellement partiellement substitué par des groupes carboxyméthyle, et qu'on y fixe le radical $-NH-(CH_2)_q-OH$, q allant de 1 à 6, par réaction avec un halogénure de thionyle puis par réaction avec une amine de formule $OH-(CH_2)_q-NH_2$, ou bien à l'aide d'un agent de couplage, par condensation avec ladite amine hydroxylée.

**20.** Procédé selon l'une des revendications 1 à 19, caractérisé par le fait qu'on réalise la phosphorylation à l'aide du dichlorophosphate de méthyle, de l'acide phosphoreux, ou de l'oxychlorure de phosphore, et notamment à l'aide de l'oxychlorure de phosphore.

**21.** Procédé selon l'une des revendications 1 à 20, caractérisé par le fait qu'on prépare les bases modifiées des formules (I) ou (II) dans lesquelles Z' = $-CH_2-CH_2-$ en faisant réagir ladite base avec le carbonate d'éthylène en présence d'un catalyseur transfert de phase.

**22.** Procédé selon l'une des revendications 1 à 21, caractérisé par le fait qu'on réalise la réaction du reste $-Z-A_1$ sur les bases modifiées des formules (I) ou (II) à l'aide d'un agent de couplage, tel que la N,N'-dicyclohexylcarbodiimide.

**23.** Procédé selon l'une des revendications 1 à 21, caractérisé par le fait qu'on prépare une base modifiée de formule (Ia) ou (IIa) en phosphorylant avec $POCl_3$ la base correspondante de formule (I) ou (II), puis on condense ladite base de formule (Ia) ou (IIa) sur le substituant -Z-OH à l'aide d'un agent de couplage, tel que la N,N'-dicyclohexylcarbodiimide.

**24.** Utilisation du polymère dérivé d'un polymère ou copolymère réticulé du styrène, ou d'un dextrane réticulé, tels que définis à l'une des revendications 1 à 12, comme phase stationnaire en chromatographie d'échange d'ions.

**25.** Utilisation du polymère dérivé d'un polymère ou copolymère réticulé du styrène, ou d'un dextrane réticulé, tels que définis à l'une des revendications 1 à 12, comme phase stationnaire en chromatographie d'affinité.

**26.** Utilisation selon la revendication 25, pour réaliser des épurations sélectives des différents types d'anticorps développés par les malades lupiques.

**27.** Utilisation selon l'une des revendications 24 et 25 , pour la purification d'enzymes, de coenzymes ou de complexes enzymatiques , admettant comme substrats, l'ADN, l'ARN et les nucléotides.

**28.** Utilisation selon l'une des revendications 24 et 25 pour la purification à partir de divers extraits plasmatiques, des facteurs de la coagulation sanguine.

**29.** Utilisation du polymère dérivé d'un polymère ou copolymère réticulé du styrène, ou d'un dextrane réticulé, tels que définis à l'une des revendications 1 à 12, pour la purification des molécules d'origine biologique, telles que les protéines intervenant au cours du processus de la coagulation sanguine, et dans des systèmes d'analyse biologique, tels que RIA, ELISA, électrophorèse, immunoélectrophorèse mono- et bidimensionnelle, électrofocalisation.

**30.** Utilisation selon la revendication 29, pour réaliser les dosages des différents types d'anticorps lupiques, et, notamment, le dosage des anticoagulants circulants et/ou des anti-phospholipides.

**Claims**
**Claims for the following Contracting States : AT, BE, CH, LI, DE, FR, GB, GR, IT, LU, NL, SE**

**1.** Polymer derived from a crosslinked styrene polymer or copolymer, or from a crosslinked dextran, in which the chain of the base polymer or copolymer is substituted with one or more groups, which may be identical or different, belonging to the following categories:

$-Z-A_1$;
$-Z-A_2$;
$-Z-A_1-Z'-A_2$;
$-Z-A_1-A_3-A_2$;
$-Z-A_1-A_4$

where:
- Z denotes a spacer chain;
- Z' denotes a linking chain;
- $A_1$ denotes a phosphate residue;
- $A_2$ denotes the residue of a purine base or of a pyrimidine base;
- $A_3$ denotes a sugar residue; and
- $A_4$ denotes a residue of a molecule participating in the polar structure of the various phospholipids, excluding polymers only substituted with $-Z-A_1-A_3-A_2$ groups.

**2.** Polymer as claimed in claim 1, characterized in that Z is chosen from the residues:
$-(CH_2)_n-$, n equalling from 1 to 6, optionally made hydrophilic by the replacement of at least one H by an OH; or
- $SO_2NH-(CH_2)_m$, m equalling from 1 to 6, the residue $-(CH_2)_m$-optionally being made hydrophilic by the replacement of at least one H by an OH; and
- in the case of the modification of a crosslinked dextran, also

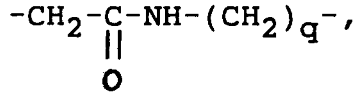

q equalling from 1 to 6, the residue $-(CH_2)_q$-optionally being made hydrophilic by the replacement of at least one H by an OH.

**3.** Polymer as claimed in claim 2, characterized in that Z is chosen from the residues: $-CH_2-$; $-(CH_2)_2$; $-(CH_2)_3-$;$-SO_2-NH-(CH_2)_{2\ \text{or}\ 3}$; $-SO_2-NH-CHOH-$; and$-SO_2-NH-CH_2-CHOH-CHOH-$; and, in the case of the modification of a crosslinked dextran, also

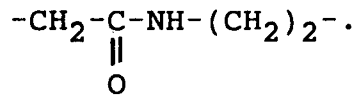

**4.** Polymer as claimed in one of claims 1 to 3, characterized in that Z' denotes $-(CH_2)_p-$, p equalling from 1 to 6, this residue optionally being made hydrophilic by the replacement of at least one H by an OH.

**5.** Polymer as claimed in one of claims 1 to 4 characterized in that $A_2$ denotes the residue of a purine base chosen from adenine and guanine, or the residue of a pyrimidine base chosen from cytosine, thymine and uracil.

**6.** Polymer as claimed in one of claims 1 to 5, characterized in that $A_3$ denotes the residue of a pentose and, in particular, of D-ribose or of 2-deoxy-D-ribose, linked via its $-CH_2-$ group to the phosphate residue.

**7.** Polymer as claimed in one of claims 1 to 6 characterized in that $A_4$ denotes an esterified molecule of choline, ethanolamine, serine, glycerol or of inositol.

**8.** Polymer as claimed in one of claims 1 to 7 characterized in that the base polymer is a crosslinked styrene polymer or copolymer and the substitution of the polystyrene chain is performed at the para position of the phenyl ring.

**9.** Polymer as claimed in one of claims 1 to 8 characterized in that the base polymer is a crosslinked styrene polymer or copolymer and each phenyl group not involved in the cross-linking can bear a substituent as defined in claim 1.

**10.** Polymer as claimed in one of claims 1 to 9 characterized in that the base polymer is a crosslinked styrene polymer or copolymer and has been crosslinked with at most 5% by weight of at least one crosslinking monomer, such as divinylbenzene.

**11.** Polymer as claimed in one of claims 1 to 10 characterized in that it results from the modification of a base polystyrene which takes the form of beads having a size in the region of 50 $\mu$m.

**12.** Polymer as claimed in one of claims 1 to 7 characterized in that it results from the modification of a crosslinked dextran with epichlorohydrin, the said dextran optionally bearing carboxymethyl substituents.

**13.** Method for preparing the polymer derived from the crosslinked styrene (co)polymer or from the crosslinked dextran, as defined in one of claims 1 to 12 characterized in that it comprises the binding to the base crosslinked polymer or copolymer (polystyrene or dextran), in one or more stages, of a residue -Z-OH, or, in the case of the styrene polymer or copolymer, of a residue -Z-X (where X denotes halogen and Z is as defined in claim 1) and then

(I) in the case where a residue -Z-X has been bound, the reaction of the polystyrene thus modified with a purine or pyrimidine base, in order to form the substituent $-Z-A_2$;

(II) in the case where a residue -Z-OH has been bound,

(IIa) the phosphorylation of the polymer or copolymer thus modified, in order to form the substituent $-Z-A_1$; and then

. when it is desired to form the substituent $-Z-A_1-Z'-A_2$, the reaction of the residue $-Z-A_1$ with a modified purine base represented diagrammatically by the formula ;

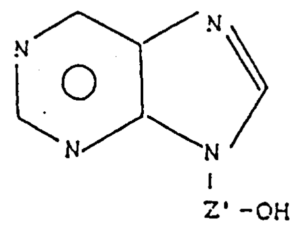

(I)

or with a modified pyrimidine base represented diagrammatically by the formula:

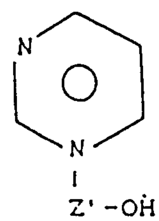

(II)

. when it is desired to form the substituent $-Z-A_1-A_3-A_2$, the reaction of the residue $-Z-A_1$ with a nucleoside of the formula $-A_3-A_2$, resulting from the linking of the nitrogen[9] of a purine base or the nitrogen[1] of a pyrimidine base with a carbon [1'] of a sugar;

. when it is desired to form the substituent $-Z-A_1-A_4$, the reaction of the group $-Z-A_1$ with a molecule participating in the polar structure of various phospholipids; or alternatively

(IIb) when it is desired to form the substituent $-Z-A_1-A_3-A_2$ directly, the reaction of the polymer or copolymer thus modified with a nucleoside monophosphate of the formula $A_1-A_3-A_2$;

(IIc) when it is desired to form the substituent $-Z-A_1-Z'-A_2$, the reaction of the polymer or copolymer thus modified with a converted purine base represented diagrammatically by the formula:

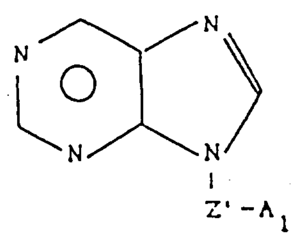

(Ia)

and which is obtained by phosphorylation of the modified purine base of the formula (I), or with a converted pyrimidine base represented diagrammatically by the formula:

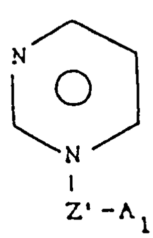

(IIa)

and which is obtained by the phosphorylation of the modified pyrimidine base of formula (II); or alternatively

(IId) when it is desired to form the substituent $-Z-A_1-A_4$, the reaction of the polymer or copolymer thus modified with a phosphorylated residue $A_4$.

**14.** Method as claimed in claim 13 characterized in that a crosslinked polystyrene is used as a starting polymer and the first step is to bind a radical $-CH_2-X$ to the said polystyrene, by reacting the said polystyrene with a halomethyl methyl ether in the presence of stannic chloride.

**15.** Method as claimed in claim 13 characterized in that a crosslinked polystyrene is used as a starting polymer and the first step is to bind a $-CH_2-OH$ radical to the said polystyrene, by reacting, in the presence of a phase transfer catalyst, the resin obtained by the method as claimed in claim 13 with potassium acetate and then by hydrolyzing the acetate obtained with concentrated potassium hydroxide.

**16.** Method as claimed in claim 13 characterized in that a crosslinked polystyrene is used as a starting polymer and the first step is to bind a $-CH_2-CH_2-OH$ radical to the said polystyrene,

. by reacting the resin obtained by the method as claimed in claim 13 with sodium cyanide, in the presence of a phase transfer catalyst, to obtain a substituent $-CH_2-C{\equiv}N$;
. then by reacting the polystyrene thus modified with hydrochloric acid in an alcoholic medium, in order to obtain a substituent $-CH_2-COOH$;
. and then by reducing with diborane, in order to obtain a substituent $-CH_2-CH_2-OH$.

**17.** Method as claimed in claim 13 characterized in that a crosslinked polystyrene is used as a starting polymer and the first step is to bind a $-CH_2-CH_2-CH_2-OH$ radical to the said polystyrene;

. by condensing an alkyl malonate with the resin obtained by the method as claimed in claim 13, in order to obtain a substituent:

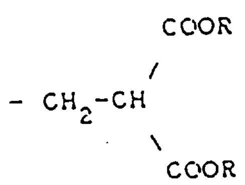

(R being an alkyl residue);
. then by hydrolyzing the diester group thereby obtained to give a diacid group, which is subjected to a decarboxylation in acid medium, in order to obtain a substituent $-CH_2-CH_2-COOH$;
. and then by reducing with diborane in order to obtain a substituent $-CH_2-CH_2-CH_2-OH$.

**18.** Method as claimed in claim 13, characterized in that a crosslinked polystyrene is used as a starting polymer and the first step is to bind an $-SO_2-NH-(CH_2)_{2\ \mathrm{or}\ 3}-OH$ radical to the said polystyrene,

. by reacting chlorosulfonic acid with the starting polystyrene, so as to obtain a substituent $-SO_2Cl$,
. and then by reacting ethanolamine or propanolamine with the abovementioned substituent.

**19.** Method as claimed in claim 13 characterized in that a crosslinked polystyrene is used as a starting polymer and the first step is to bind a $-SO_2-NH-CH_2-CHOH-CH_2OH$ radical to the said polystyrene,

. by reacting chlorosulfonic acid with the starting polystyrene, so as to obtain a substituent $-SO_2Cl$;
. and then by reacting 3-amino-1,2-propanediol with the above mentioned substituent.

**20.** Method as claimed in claim 13 characterized in that a crosslinked dextran optionally partially substituted by carboxymethyl groups is used as a starting polymer and the radical $-NH-(CH_2)_q-OH$, q ranging from 1 to 6, is bound thereto by reaction with a thionyl halide followed by reaction with an amine of formula $OH-(CH_2)_q-NH_2$, or with the aid of a coupling agent, by condensation with the said hydroxylated amine.

**21.** Method as claimed in one of claims 13 to 20 characterized in that the phosphorylation is carried out using methyl dichlorophosphate, phosphorous acid or phosphorus oxychloride, and in particular using phosphorus oxychloride.

**22.** Method as claimed in one of claims 13 to 21, characterized in that the modified bases of formulae (I) or (II) in which Z' = $-CH_2-CH_2-$ are prepared by reacting the said base with ethylene carbonate in the presence of a phase transfer catalyst.

**23.** Method as claimed in one of claims 13 to 22, characterized in that the reaction of the residue $-Z-A_1$ with the modified bases of formula (I) or (II) is carried out using a coupling agent, such as N,N'-dicyclohexylcarbodiimide.

**24.** Method as claimed in one of claims 13 to 22, characterized in that a modified base of formula (Ia) or (IIa) is prepared by phosphorylating the corresponding base of formula (I) or (II) with $POCL_3$, and the said base of formula (Ia) or (IIa) is then condensed with the substituent -Z-OH using a coupling agent, such as N,N'-dicyclohexylcarbodiimide.

**25.** Use of the polymer derived from a crosslinked styrene polymer or copolymer, or from a crosslinked dextran, as defined in one of claims 1 to 12, as a stationary phase in ion-exchange chromatography.

**26.** Use of the polymer derived from a crosslinked styrene polymer or copolymer, or from a crosslinked dextran, as defined in one of claims 1 to 12, as a stationary phase in affinity chromatography.

**27.** Use as claimed in claim 26, for carrying out selective purifications of the different types of antibodies developed by lupus patients.

**28.** Use as claimed in one of claims 25 and 26, for the purification of enzymes, coenzymes or enzyme complexes accepting DNA, RNA and nucleotides as substrates.

**29.** Use as claimed in one of claims 25 and 26 for the purification, starting from various plasma extracts, of blood coagulation factors.

**30.** Use of the polymer derived from a crosslinked styrene polymer or copolymer, or from a crosslinked dextran, as defined in one of claims 1 to 12, for the purification of molecules of biological origin such as the proteins participating in the process of blood coagulation, and in biological analytical systems such as RIA, ELISA, electrophoresis, one- and two-dimensional immunoelectrophoresis and electrofocussing.

**31.** Use, as claimed in claim 30, for carrying out determinations of various types of lupous antibodies and especially the determination of circulating anticoagulants and/or of anti-phospholipids.

**Claims for the following Contracting State : ES**

**1.** Method for preparing the polymer derived from a crosslinked styrene polymer or copolymer, or from a crosslinked dextran,in which the chain of the base polymer or copolymer is substituted with one or more groups, which may be identical or different, belonging to the following categories:

$-Z-A_1$;
$-Z-A_2$;
$-Z-A_1-Z'-A_2$;
$-Z-A_1-A_3-A_2$;
$-Z-A_1-A_4$

where:

- Z denotes a spacer chain;
- Z' denotes a linking chain;
- $A_1$ denotes a phosphate residue;
- $A_2$ denotes the residue of a purine base or of a pyrimidine base;
- $A_3$ denotes a sugar residue, and
- $A_4$ denotes a residue of a molecule participating in the polar structure of the various phospholipids, excluding polymers only substituted with $-Z-A_1-A_2-A_3$ groups, characterized in that it comprises the binding to the base crosslinked polymer or copolymer (polystyrene or dextran), in one or more stages, of a residue -Z-OH, or, in the case of the styrene polymer or copolymer,

of a residue -Z-X (where X denotes halogen and Z is as hereabove defined) and then

(I) in the case where a residue -Z-X has been bound, the reaction of the polystyrene thus modified with a purine or pyrimidine base, in order to form the substituent $-Z-A_2$;

(II) in the case where a residue -Z-OH has been bound,

(IIa) the phosphorylation of the polymer or copolymer thus modified, in order to form the substituent $-Z-A_1$; and then

. when it is desired to form the substituent $-Z-A_1-Z'-A_2$, the reaction of the residue $-Z-A_1$ with a modified purine base represented diagrammatically by the formula :

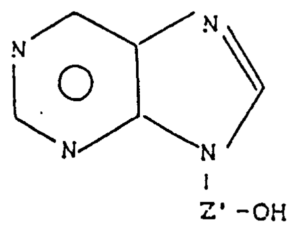

·(I)

or with a modified pyrimidine base represented diagrammatically by the formula:

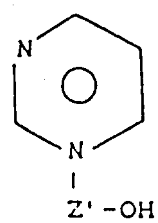

(II)

. when it is desired to form the substituent $-Z-A_1-A_3-A_2$, the reaction of the residue $-Z-A_1$ with a nucleoside of the formula $-A_3-A_2$, resulting from the linking of the nitrogen$^{9}$ of a purine base or the nitrogen$^{1}$ of a pyrimidine base with a carbon $^{1'}$ of a sugar;

. when it is desired to form the substituent $-Z-A_1-A_4$, the reaction of the group $-Z-A_1$ with a molecule participating in the polar structure of various phospholipids; or alternatively

(IIb) when it is desired to form the substituent $-Z-A_1-A_3-A_2$ directly, the reaction of the polymer or copolymer thus modified with a nucleoside monophosphate of the formula $A_1-A_3-A_2$;

(IIc) when it is desired to form the substituent $-Z-A_1-Z'-A_2$, the reaction of the polymer or copolymer thus modified with a converted purine base represented diagrammatically by the formula:

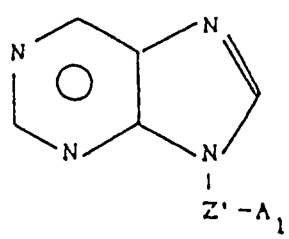

·(Ia)

and which is obtained by phosphorylation of the modified purine base of the formula (I), or with a converted pyrimidine base represented diagrammatically by the formula:

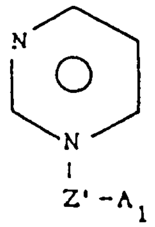 (IIa) :

and which is obtained by the phosphorylation of the modified pyrimidine base of formula (II); or alternatively
(IId) when it is desired to form the substituent $-Z-A_1-A_4$, the reaction of the polymer or copolymer thus modified with a phosphorylated residue $A_4$.

**2.** Method as claimed in claim 1 characterized in that Z is chosen from the residues:
- . $-(CH_2)_n-$, n equalling from 1 to 6, optionally made hydrophilic by the replacement of at least one H by an OH; or
- . $SO_2NH-(CH_2)_m$, m equalling from 1 to 6, the residue - $(CH_2)_m$-optionally being made hydrophilic by the replacement of at least one H by an OH; and
- . in the case of the modification of a crosslinked dextran, also

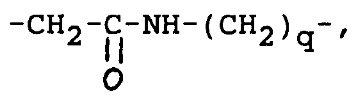

q equalling from 1 to 6, the residue
$-(CH2)_q-$ optionally being made hydrophilic by the replacement of at least one H by an OH.

**3.** Method as claimed in claim 2 characterized in that Z is chosen from the residues : $-CH_2-$; $-(CH_2)_2-$; $-(CH_2)_3-$; $-SO_2-NH-(CH_2)_{2\ or\ 3}-$; $-SO_2-NH-CHOH-$; and $SO_2-NH-CH_2-CHOH-CHOH-$ and, in the case of the modification of a crosslinked dextran, also

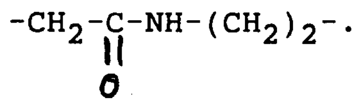

**4.** Method as claimed in one of claims 1 to 3, characterized in that Z' denotes $-(CH_2)_p-$, p equalling from 1 to 6, this residue optionally being made hydrophilic by the replacement of at least one H by an OH.

**5.** Method as claimed in one of claims 1 to 4, characterized in that $A_2$ denotes the residue of a purine base chosen from adenine and guanine, or the residue of a pyrimidine base chosen from cytosine, thymine and uracil.

**6.** Method as claimed in one of claims 1 to 5, characterized in that $A_3$ denotes the residue of a pentose and, in particular, of D-ribose or of 2-deoxy-D-ribose, linked via its $-CH_2-$ group to the phosphate residue.

**7.** Method as claimed in one of claims 1 to 6, characterized in that $A_4$ denotes an esterified molecule of choline, ethanolamine, serine, glycerol or of inositol.

**8.** Method as claimed in one of claims 1 to 7, characterized in that the base polymer is a crosslinked styrene polymer or copolymer and the substitution of the polystyrene chain is performed at the para position of the phenyl ring.

**9.** Method as claimed in one of claims 1 to 8, characterized in that the base polymer is a crosslinked styrene polymer or copolymer and each phenyl group not involved in the crosslinking can bear a substituent as defined in claim 1.

**10.** Method as claimed in one of claims 1 to 9, characterized in that the base polymer is a crosslinked styrene polymer or copolymer and has been crosslinked with at most 5% by weight of at least one crosslinking monomer, such as divinylbenzene.

**11.** Method as claimed in one of claims 1 to 10, characterized in that the polymer results from the modification of a base polystyrene which takes the form of beads having a size in the region of 50 $\mu$m.

**12.** Method as claimed in one of claims 1 to 7 characterized in that the polymer results from the modification of a crosslinked dextran with epichlorohydrin, the said dextran optionally bearing carboxymethyl substituents.

**13.** Method as claimed in one of claims 1 to 12 characterized in that a crosslinked polystyrene is used as a starting polymer and the first step is to bind a radical $-CH_2-X$ to the said polystyrene, by reacting the said polystyrene with a halomethyl methyl ether in the presence of stannic chloride.

**14.** Method as claimed in one of claims 1 to 12 characterized in that a crosslinked polystyrene is used as a starting polymer and the first step is to bind a $-CH_2-OH$ radical to the said polystyrene, by reacting, in the presence of a phase transfer catalyst, the resin obtained by the method as claimed in one of claims 1 to 12 with potassium acetate and then by hydrolyzing the acetate obtained with concentrated potassium hydroxide.

**15.** Method as claimed in one of claims 1 to 12 characterized in that a crosslinked polystyrene is used as a starting polymer and the first step is to bind a $-CH_2-CH_2-OH$ radical to the said polystyrene,

. by reacting the resin obtained by the method as claimed in one of claims 1 to 12 with sodium cyanide, in the presence of a phase transfer catalyst, to obtain a substituent $-CH_2-C\equiv N$;

. then by reacting the polystyrene thus modified with hydrochloric acid in an alcoholic medium, in order to otbain a substituent $-CH_2-COOH$;

. and then by reducing with diborane, in order to obtain a substituent $-CH_2-CH_2-OH$.

**16.** Method as claimed in one of claims 1 to 12 characterized in that a crosslinked polystyrene is used as a starting polymer and the first is to bind a $-CH_2-CH_2-CH_2-OH$ radical to the said polystyrene,

. by condensing an alkyl malonate with the resin obtained by the method as claimed in one of claims 1 to 12, in order to obtain a substituent:

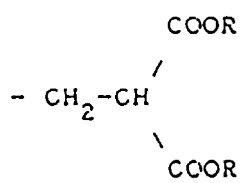

(R being an alkyl residue);

. then by hydrolyzing the diester group thereby obtained to give a diacid group, which is subjected to a decarboxylation in acid medium, in order to obtain a substituent $-CH_2-CH_2-COOH$;

. and then by reducing with diborane in order to obtain a substituent $-CH_2-CH_2-CH_2-OH$.

**17.** Method as claimed in one of claims 1 to 12 characterized in that a crosslinked polystyrene is used as a starting polymer and the first step is to bind an $-SO_2-NH(CH_2)_{2\ or\ 3}-OH$ radical to the said polystyrene,

. by reacting chlorosulfonic acid with the starting polystyrene, so as to obtain a substituent $-SO_2Cl$,

. and then by reacting ethanolamine or propanolamine with the abovementioned substituent.

**18.** Method as claimed in one of claims 1 to 12 characterized in that a crosslinked polystyrene is used as a starting polymer and the first step is to bind a $-SO_2-NH-CH_2-CHOH-CH_2OH$ radical to the said

polystyrene,

. by reacting chlorosulfonic acid with the starting polystyrene, so as to obtain a substituent $-SO_2Cl$;
. and then by reacting 3-amino-1,2-propanediol with the abovementioned substituent.

**19.** Method as claimed in one of claims 1 to 12 characterized in that a crosslinked dextran optionally partially substituted by carboxymethyl groups is used as a starting polymer and the radical $-NH-(CH_2)_q-OH$, q ranging from 1 to 6, is bound thereto by reaction with a thionyl halide followed by reaction with an amine of formula $OH-(CH_2)_q-NH_2$, or with the aid of a coupling agent, by condensation with the said hydroxylated amine.

**20.** Method as claimed in one of claims 1 to 19, characterized in that the phosphorylation is carried out using methyl dichlorophosphate, phosphorous acid or phosphorus oxychloride, and in particular using phosphorus oxychloride.

**21.** Method as claimed in one of claims 1 to 20 characterized in that the modified bases of formulae (I) or (II) in which $Z' = -CH_2-CH_2-$ are prepared by reacting the said base with ethylene carbonate in the presence of a phase transfer catalyst.

**22.** Method as claimed in one of claims 1 to 21, characterized in that the reaction of the residue $-Z-A_1$ with the modified bases of formula (I) or (II) is carried out using a coupling agent, such as N,N'-dicyclohexylcarbodiimide.

**23.** Method as claimed in one of claims 1 to 21, characterized in that a modified base of formula (Ia) or (IIa) is prepared by phosphorylating the corresponding base of formula (I) or (II) with $POCl_3$, and the said base of formula (Ia) or (IIa) is then condensed with the substituent -Z-OH using a coupling agent, such as N,N'-dicyclohexylcarbodiimide.

**24.** Use of the polymer derived from a crosslinked styrene polymer or copolymer, or from a crosslinked dextran, as defined in one of claims 1 to 12, as a stationary phase in ion-exchange chromatography.

**25.** Use of the polymer derived from a crosslinked styrene polymer or copolymer, or from a crosskinked dextran, as defined in one of claims 1 to 12 as a stationary phase in affinity chromatography.

**26.** Use as claimed in claim 25, for carrying out selective purifications of the different types of antibodies developped by lupous patients.

**27.** Use as claimed in one of claims 24 and 25, for the purification of enzymes, coenzymes or enzyme complexes accepting DNA, RNA and nucleotides as substrates.

**28.** Use as claimed in one of claims 24 and 25 for the purification, starting from various plasma extracts, of blood coagulation factors.

**29.** Use of the polymer derived from a crosslinked styrene polymer or copolymer, or from a crosslinked dextran, as defined in one of claims 1 to 12, for the purification of molecules of biological origin such as the proteins participating in the process of blood coagulation, and in biological analytical systems such as RIA, ELISA, electrophoresis, one- and two-dimensional immunoelectrophoresis and electrofocussing.

**30.** Use, as claimed in claim 29, for carrying out determinations of various types of lupous antibodies and especially the determination of circulating anticoagulants and/or anti-phospholipids.

**Patentansprüche**
**Patentansprüche für folgende Vertragsstaaten : AT, BE, CH, LI, DE, FR, GB, GR, IT, LU, NL, SE**

**1.** Polymer, das von einem vernetzten Styrol-Polymer oder -Copolymer oder von einem vernetzten Dextran abgeleitet ist, dadurch gekennzeichnet, daß die Hauptkette des Polymers oder Copolymers durch eine oder mehrere gleichartige oder verschiedene Gruppen substituiert ist, die zu folgenden Kategorien gehören:

-Z-$A_1$;
-Z-$A_2$;
-Z-$A_1$-Z'-$A_2$;
-Z-$A_1$-$A_3$-$A_2$;
-Z-$A_1$-$A_4$;

wobei:

- Z eine abstandhaltende Kette darstellt;
- Z' eine bindende Kette darstellt;
- $A_1$ einen Phosphatrest darstellt;
- $A_2$ den Rest einer Purinbase oder einer Pyrimidinbase darstellt;
- $A_3$ einen Zuckerrest darstellt; und
- $A_4$ einen Rest eines in der polaren Struktur verschiedener Phospholipide vorkommenden Moleküls darstellt, mit Ausnahme von ausschließlich durch die Gruppen -Z-$A_1$-$A_3$-$A_2$ substituierten Polymeren.

**2.** Polymer nach Anspruch 1, dadurch gekennzeichnet, daß Z aus den Resten

- $-(CH_2)_n-$, wobei n einen Wert von 1 bis 6 hat und gegebenenfalls durch den Ersatz wenigstens eines H durch ein OH hydrophil gemacht wird; oder
- $-SO_2-NH-(CH_2)_m-$, wobei m einen Wert von 1 bis 6 hat und der Rest $-(CH_2)_m-$ gegebenenfalls durch den Ersatz wenigstens eines H durch ein OH hydrophil gemacht ist; und
- im Fall der Modifikation eines vernetzten Dextrans

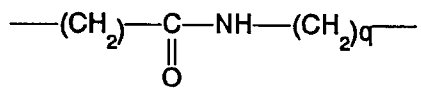 ,

auch wobei q einen Wert von 1 bis 6 hat und der Rest $-(CH_2)_q-$ gegebenenfalls durch den Ersatz wenigstens eines H durch ein OH hydrophil gemacht ist,

ausgewählt ist.

**3.** Polymer nach Anspruch 2, dadurch gekennzeichnet, daß Z aus den Resten $-CH_2-$; $-(CH_2)_2-$; $-(CH_2)_3-$; $-SO_2-NH-(CH_2)_{2\ oder\ 3}-$; $-SO_2-NH-CHOH-$ und $-SO_2-NH-CH_2-CHOH-CHOH-$ und, im Fall einer vernetzten Dextranmodifikation auch

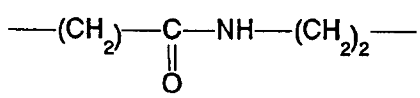 ,

ausgewählt ist.

**4.** Polymer nach einem der Ansprüche 1 bis 3, dadurch gekennzeichnet, daß Z' $-(CH_2)_p-$ darstellt, wobei p einen Wert von 1 bis 6 hat und der Rest gegebenenfalls durch den Ersatz wenigstens eines H durch ein OH hydrophil gemacht ist.

**5.** Polymer nach einem der Ansprüche 1 bis 4, dadurch gekennzeichnet, daß $A_2$ den Rest einer aus Adenin oder Guanin ausgewählten Purinbase oder den Rest einer aus Cytosin, Thymin und Uracil ausgewählten Pyrimidinbase darstellt.

**6.** Polymer nach einem der Ansprüche 1 bis 5, dadurch gekennzeichnet, daß $A_3$ den Rest einer Pentose und besonders einer D-Ribose oder einer 2-Desoxy-D-ribose, die durch ihre $-CH_2-$ -Gruppe an den Phosphatrest gebunden ist, darstellt.

**7.** Polymer nach einem der Ansprüche 1 bis 6, dadurch gekennzeichnet, daß $A_4$ ein verestertes Cholin-, Ethanolamin-, Serin-, Glycerin- oder Inosit-Molekül darstellt.

**8.** Polymer nach einem der Ansprüche 1 bis 7, dadurch gekennzeichnet, daß das Grundpolymer ein vernetztes Styrol-Polymer oder -Copolymer ist und daß die Substitution der Polystyrolkette an der Parastellung des Phenylkerns erfolgt.

**9.** Polymer nach einem der Ansprüche 1 bis 8, dadurch gekennzeichnet, daß das Grundpolymer ein vernetztes Styrol-Polymer oder -Copolymer ist und daß jede nicht in die Vernetzung einbezogene Phenylgruppe einen nach Anspruch 1 definierten Substituenten tragen kann.

**10.** Polymer nach einem der Ansprüche 1 bis 9, dadurch gekennzeichnet, daß das Grundpolymer ein vernetztes Styrol-Polymer oder -Copolymer ist und daß dieses mit höchstens 5 Gew.-% mindestens eines Vernetzungs-Monomers wie Divinylbenzol vernetzt worden ist.

**11.** Polymer nach einem der Ansprüche 1 bis 10, dadurch gekennzeichnet, daß es aus der Modifikation eines in Form von Kugeln mit einer Grösse von ungefähr 50 $\mu$m vorliegenden Grund-Polystyrols hervorgeht.

**12.** Polymer nach einem der Ansprüche 1 bis 7, dadurch gekennzeichnet, daß es aus der Modifikation eines durch Epichlorhydrin vernetzten Dextrans hervorgeht, wobei das Dextran gegebenenfalls Carboxymethyl-Substituenten trägt.

**13.** Verfahren zur Herstellung eines Polymers, das von einem vernetzten, in einem der Ansprüche 1 bis 12 definierten Styrol-(Co)Polymer oder Dextran abstammt, dadurch gekennzeichnet, daß es die Bindung eines Restes -Z-OH, oder im Fall des Styrol-Polymers oder -Copolymers eines Restes -Z-X (wobei X ein Halogen darstellt und Z wie in Anspruch 1 definiert ist) an das vernetzte Grund-Polymer oder -Copolymer (Polystyrol oder Dextran) in einem oder mehreren Schritten umfaßt, und anschließend

(I) in dem Fall, in dem ein Rest -Z-X gebunden wurde, die Reaktion des so modifizierten Polystyrols mit einer Purin- oder Pyrimidinbase, um den Substituenten $-Z-A_2$ zu bilden;

(II) in dem Fall, in dem ein Rest Z-OH gebunden wurde,

(IIa) die Phosphorylierung des so modifizierten Polymers oder Copolymers, um den Substituenten $-Z-A_1$ zu bilden; und anschließend,

- wenn die Bildung des Substituenten $-Z-A_1-Z'-A_2$ gewünscht wird, die Reaktion des Restes $-Z-A_1$ mit einer durch die Formel

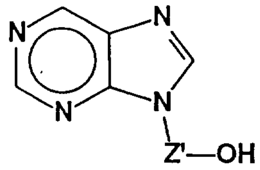

(I)

dargestellten modifizierten Purinbase
oder mit einer modifizierten, durch die Formel

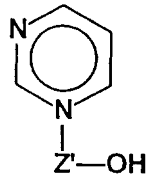

(II)

dargestellten Pyrimidinbase;

- wenn die Bildung des Substituenten $-Z-A_1-A_3-A_2$ gewünscht wird, die Reaktion des Restes $-Z-A_1$ mit einem Nucleosid der Formel $-A_3-A_2$, die aus der Bindung von Stickstoff 9 einer Purinbase oder von Stickstoff 1 einer Pyrimidinbase mit dem Kohlenstoff 1' eines Zuckers abstammt;

- wenn die Bildung des Substituenten -Z-$A_1$-$A_4$ gewünscht wird, die Reaktion der Gruppe -Z-$A_1$ mit einem in der polaren Struktur verschiedener Phospholipide vorkommenden Molekül; oder

(IIb) wenn die direkte Bildung des Substituenten -Z-$A_1$-$A_3$-$A_2$ gewünscht wird, die Reaktion des so modifizierten Polymers oder Copolymers mit einem Nucleosidmonophosphat der Formel $A_1$-$A_3$-$A_2$;

(IIc) wenn die Bildung des Substituenten -Z-$A_1$-Z'-$A_2$ gewünscht wird, die Reaktion des so modifizierten Polymers oder Copolymers mit einer umgewandelten Purinbase, dargestellt durch die Formel

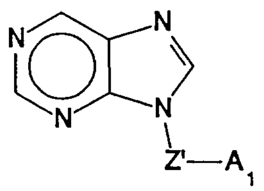

(Ia),

und die durch Phosphorylierung der modifizierten Purinbase der Formel (I) erhalten wird, oder mit einer umgewandelten Pyrimidinbase, dargestellt durch die Formel

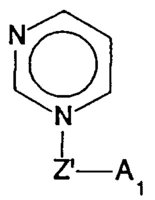

(IIa),

und die durch Phosphorylierung der modifizierten Pyrimidinbase der Formel (II) erhalten wird, oder

(IId) wenn die Bildung des Substituenten -Z-$A_1$-$A_4$ gewünscht wird, die Reaktion des so modifizierten Polymers oder Copolymers mit einem phosphorylierten Rest $A_4$.

**14.** Verfahren nach Anspruch 13, dadurch gekennzeichnet, daß als Ausgangspolymer ein vernetztes Polystyrol verwendet wird und daß man mit der Bindung des Restes $-CH_2-X$ an dieses Polystyrol beginnt, indem das Polystyrol in Gegenwart von Zinnchlorid mit einem Halogenmethylmethylether umgesetzt wird.

**15.** Verfahren nach Anspruch 13, dadurch gekennzeichnet, daß als Ausgangspolymer ein vernetztes Polystyrol verwendet wird und daß man mit der Bindung des Restes $-CH_2-OH$ an dieses Polystyrol beginnt, indem das nach dem Verfahren gemäß Anspruch 13 erhaltene Harz in Gegenwart eines Phasentransfer-Katalysators mit Kaliumacetat umgesetzt und anschließend das erhaltene Acetat mit konzentrierter Kalilauge hydrolisiert wird.

**16.** Verfahren nach Anspruch 13, dadurch gekennzeichnet, daß als Ausgangspolymer ein vernetztes Polystyrol verwendet wird und daß man mit der Bindung des Restes $-CH_2-CH_2-OH$ an dieses Polystyrol beginnt , indem

- das nach dem Verfahren gemäß Anspruch 13 erhaltene Harz in Gegenwart eines Phasentransfer-Katalysators mit Natriumcyanid umgesetzt wird, um den Substituenten $-CH_2-C\equiv N$ zu erhalten;
- anschließend das so modifizierte Polystyrol mit alkoholischer Salzsäure umgesetzt wird, um den Substituenten $-CH_2-COOH$ zu erhalten;
- anschließend mit Diboran reduziert wird, um den Substituenten $-CH_2-CH_2-OH$ zu erhalten.

**17.** Verfahren nach Anspruch 13, dadurch gekennzeichnet, daß als Ausgangspolymer ein vernetztes Polymer verwendet wird und daß man mit der Bindung des Restes $-CH_2-CH_2-CH_2-OH$ an dieses Polystyrol beginnt , indem

- auf dem nach dem Verfahren gemäß Anspruch 13 erhaltenen Harz ein Alkylmalonat kondensiert wird, um den Substituenten

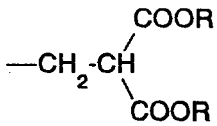

  zu erhalten (R ist ein Alkylrest);
- anschließend durch das Hydrolisieren der so erhaltenen Diester-Funktion eine Disäure-Funktion erhalten wird, die im sauren Milieu decarboxyliert wird, um den Substituenten $-CH_2-CH_2-COOH$ zu erhalten;
- anschließend mit Diboran reduziert wird, um den Substituenten $-CH_2-CH_2-CH_2-OH$ zu erhalten.

**18.** Verfahren nach Anspruch 13, dadurch gekennzeichnet, daß als Ausgangspolymer ein vernetztes Polystyrol verwendet wird und daß man mit der Bindung des Restes $-SO_2-NH-(CH_2)_{2\ \mathrm{oder}\ 3}-OH$ an dieses Polystyrol beginnt, indem
- das Ausgangsstyrol mit Chlorsulfonsäure umgesetzt wird, um den Substituenten $-SO_2Cl$ zu erhalten,
- anschließend Ethanolamin oder Propanolamin auf den zuvor erwähnten Substituenten einwirken gelassen wird.

**19.** Verfahren nach Anspruch 13, dadurch gekennzeichnet, daß als Ausgangspolymer ein vernetztes Polystyrol verwendet wird und daß man mit der Bindung des Restes $-SO_2-NH-CH_2-CHOH-CH_2OH$ an dieses Polystyrol beginnt, indem
- das Ausgangspolymer mit Chlorsulfonsäure umgesetzt wird, um den Substituenten $-SO_2Cl$ zu erhalten,
- anschließend Amino-3-propandiol-1,2 auf den zuvor erwähnten Substituenten einwirken gelassen wird.

**20.** Verfahren nach Anspruch 13, dadurch gekennzeichnet, daß als Ausgangspolymer ein vernetztes, gegebenenfalls teilweise durch Carboxymethyl-Gruppen substituiertes Dextran verwendet wird, worauf durch Reaktion mit einem Thionylhalogenid und anschließend durch Reaktion mit einem Amin der Formel $OH-(CH_2)_q-NH_2$ oder mit Hilfe eines Kupplungs-Reagenzes durch Kondensation mit dem hydroxyliertem Amin der Rest $-NH-(CH_2)_q-OH$, wobei q einen Wert von 1 bis 6 hat, gebunden wird.

**21.** Verfahren nach einem der Ansprüche 13 bis 20, dadurch gekennzeichnet, daß die Phosphorylierung mit Hilfe von Methyldichlorphosphat, phosphoriger Säure oder Phosphoroxychlorid und insbesondere mit Hilfe von Phosphoroxychlorid durchgeführt wird.

**22.** Verfahren nach einem der Ansprüche 13 bis 21, dadurch gekennzeichnet, daß die modifizierten Basen mit den Formeln (I) oder (II), in denen Z' = $-CH_2-CH_2-$ ist, durch Umsetzung der Base mit Ethylencarbonat in Gegenwart eines Phasentransfer-Katalysators herstellt wird.

**23.** Verfahren nach einem der Ansprüche 13 bis 22, dadurch gekennzeichnet, daß die Reaktion des Restes $-Z-A_1$ mit den modifizierten Basen mit den Formeln (I) oder (II) mit Hilfe eines Kupplungs-Reagenzes wie N,N'-Dicyclohexylcarbodiimid durchgeführt wird.

**24.** Verfahren nach einem der Ansprüche 13 bis 22, dadurch gekennzeichnet, daß eine modifizierte Base mit der Formel (Ia) oder (IIa) durch das Phosphorylieren der entsprechenden Base mit der Formel (I) oder (II) mit $POCl_3$ hergestellt wird und anschließend die Base mit der Formel (Ia) oder (IIa) mit Hilfe eines Kupplungs-Reagenzes wie N,N'-Dicyclohexylcarbodiimid mit dem Substituenten -Z-OH kondensiert wird.

**25.** Verwendung eines in den Ansprüchen 1 bis 12 definierten, von einem vernetzten Styrol-Polymer oder -Copolymer oder von einem vernetzten Dextran abgeleiteten Polymers als stationäre Phase bei der Ionenaustausch-Chromatographie.

**26.** Verwendung eines in den Ansprüchen 1 bis 12 definierten, von einem vernetzten Styrol-Polymer oder -Copolymer oder von einem vernetzten Dextran abgeleiteten Polymers als stationäre Phase bei der Affinitäts-Chromatographie.

**27.** Verwendung nach Anspruch 26 zur Erzielung selektiver Reinigungen verschiedener, durch lupusartige Krankheiten entwickelter Antikörper-Typen.

**28.** Verwendung nach einem der Ansprüche 25 und 26 zur Reinigung von Enzymen, Coenzymen oder enzymatischen Komplexen, die als Substrate die DNS, die RNS und die Nucleotide zuläßt.

**29.** Verwendung nach einem der Ansprüche 25 und 26 zur Reinigung von Blutgerinnungs-Faktoren, ausgehend von verschiedenen plasmatischen Extrakten.

**30.** Verwendung eines in den Ansprüchen 1 bis 12 definierten, von einem vernetzten Styrol-Polymer oder -Copolymer oder von einem vernetzten Dextran abgeleiteten Polymers zur Reinigung von Molekülen biologischen Ursprungs wie der im Verlauf der Blutgerinnung und in biologischen Analytiksystemen wie RIA, ELISA, Elektrophorese, ein- und zweidimensionaler, Immunoelektrophorese, Elektrofokussierung auftretenden Proteine.

**31.** Verwendung nach Anspruch 30, um Dosierungen der verschiedenen lupusartigen Antikörper-Typen und insbesondere die Dosierung von gerinnungshemmenden Kreislaufmitteln und/oder von Anti-Phospholipiden herzustellen.

**Patentansprüche für folgenden Vertragsstaat : ES**

**1.** Verfahren zur Herstellung eines Polymers, das von einem vernetzten Styrol-Polymer oder -Copolymer oder von einem vernetzten Dextran abgeleitet ist, wobei die Hauptkette des Polymers oder Copolymers durch eine oder mehrere gleichartige oder verschiedene Gruppen substituiert ist, die zu folgenden Kategorien gehören:

$-Z-A_1$;
$-Z-A_2$;
$-Z-A_1-Z'-A_2$;
$-Z-A_1-A_3-A_2$;
$-Z-A_1-A_4$;

wobei:
- Z eine abstandhaltende Kette darstellt;
- Z' eine bindende Kette darstellt;
- $A_1$ einen Phosphatrest darstellt;
- $A_2$ den Rest einer Purinbase oder einer Pyrimidinbase darstellt;
- $A_3$ einen Zuckerrest darstellt; und
- $A_4$ einen Rest eines in der polaren Struktur verschiedener Phospholipide vorkommenden Moleküls darstellt, mit Ausnahme von ausschließlich durch die Gruppen $-Z-A_1-A_3-A_2$ substituierten Polymeren,

dadurch gekennzeichnet, daß es die Bindung eines Restes -Z-OH, oder im Fall des Styrol-Polymers oder -Copolymers eines Restes -Z-X (wobei X ein Halogen darstellt und Z wie oben definiert ist) an das vernetzte Grund-Polymer oder -Copolymer (Polystyrol oder Dextran) in einem oder mehreren Schritten umfaßt, und anschließend

(I) in dem Fall, in dem ein Rest -Z-X gebunden wurde, die Reaktion des so modifizierten Polystyrols mit einer Purin- oder Pyrimidinbase, um den Substituenten $-Z-A_2$ zu bilden;

(II) in dem Fall, in dem ein Rest Z-OH gebunden wurde,

(IIa) die Phosphorylierung des so modifizierten Polymers oder Copolymers, um den Substituenten $-Z-A_1$ zu bilden; und anschließend,

- wenn die Bildung des Substituenten $-Z-A_1-Z'-A_2$ gewünscht wird, die Reaktion des Restes $-Z-A_1$ mit einer durch die Formel

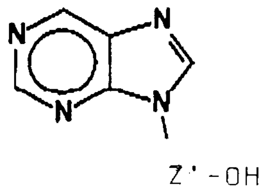

(I)

dargestellten modifizierten Purinbase
oder mit einer modifizierten, durch die Formel

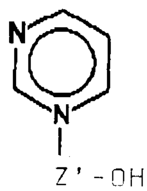

(II)

dargestellten Pyrimidinbase;

- wenn die Bildung des Substituenten $-Z-A_1-A_3-A_2$ gewünscht wird, die Reaktion des Resten $-Z-A_1$ mit einem Nucleosid der Formel $-A_3-A_2$, die aus der Bindung von Stickstoff 9 einer Purinbase oder von Stickstoff 1 einer Pyrimidinbase mit dem Kohlenstoff 1' eines zuckers abstammt;
- wenn die Bildung des Substituenten $-Z-A_1-A_4$ gewünscht wird, die Reaktion der Gruppe $-Z-A_1$ mit einem in der polaren Struktur verschiedener Phospholipide vorkommenden Molekül; oder

(IIb) wann die direkte Bildung des Substituenten $-Z-A_1-A_3-A_2$ gewünscht wird, die Reaktion des so modifizierten Polymers oder Copolymers mit einem Nucleosidmonophosphat der Formel $A_1-A_3-A_2$;

(IIc) wenn die Bildung des Substituenten $-Z-A_1-Z'-A_2$ gewünscht wird, die Reaktion des so modifizierten Polymers oder Copolymers mit einer umgewandelten Purinbase, dargestellt durch die Formel

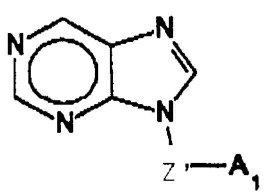

(Ia),

und die durch Phosphorylierung der modifizierten Purinbase der Formel (I) erhalten wird, oder mit einer umgewandelten Pyrimidinbase, dargestellt durch die Formel

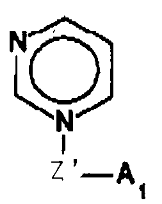

(IIa),

und die durch Phosphorylierung der modifizierten Pyrimidinbase der Formel (II) erhalten wird, oder

(IId) wenn die Bildung des Substituenten $-Z-A_1-A_4$ gewünscht wird, die Reaktion des so modifizierten Polymers oder Copolymers mit einem phosphorylierten Rest $A_4$.

**2.** Verfahren nach Anspruch 1, dadurch gekennzeichnet, daß Z aus den Resten

- $-(CH_2)_n-$, wobei n einen Wert von 1 bis 6 hat und gegebenenfalls durch den Ersatz wenigstens, eines H durch ein OH hydrophil gemacht wird; oder
- $-SO_2-NH-(CH_2)_m-$, wobei m einen Wert von 1 bis 6 hat und der Rest $-(CH_2)_m-$ gegebenenfalls durch den Ersatz wenigstens eines H durch ein OH hydrophil gemacht ist; und
- im Fall der Modifikation eines vernetzten Dextrans auch

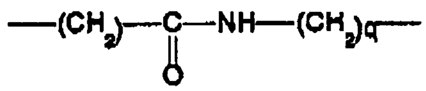 ,

wobei q einen Wert von 1 bis 6 hat und der Rest $-(CH_2)_q-$ gegebenenfalls durch den Ersatz wenigstens eines H durch ein OH hydrophil gemacht ist,

ausgewählt ist.

**3.** Verfahren nach Anspruch 2, dadurch gekennzeichnet, daß Z aus den Resten $-CH_2-$; $-(CH_2)_2-$; $-(CH_2)_3-$; $-SO_2-NH-(CH_2)_{2\ \text{oder}\ 3}-$; $-SO_2-NH-CHOH-$ und $-SO_2-NH-CH_2-CHOH-CHOH-$ und, im Fall einer vernetzten Dextranmodifikation auch

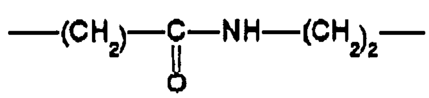 ,

ausgewählt ist.

**4.** Verfahren nach einem der Ansprüche 1 bis 3, dadurch gekennzeichnet, daß Z' $-(CH_2)_p-$ darstellt, wobei p einen Wert von 1 bis 6 hat und der Rest gegebenenfalls durch den Ersatz wenigstens eines H durch ein OH hydrophil gemacht ist.

**5.** Verfahren nach einem der Ansprüche 1 bis 4, dadurch gekennzeichnet, daß $A_2$ den Rest einer aus Adenin oder Guanin ausgewählten Purinbase oder den Rest einer aus Cytosin, Thymin und Uracil ausgewählten Pyrimidinbase darstellt.

**6.** Verfahren nach einem der Ansprüche 1 bis 5, dadurch gekennzeichnet, daß $A_3$ den Rest einer Pentose und besondere einer D-Ribose oder einer 2-Desoxy-D-ribose, die durch ihre $-CH_2-$ -Gruppe an den Phosphatrest gebunden ist, darstellt.

**7.** Verfahren nach einem der Ansprüche 1 bis 6, dadurch gekennzeichnet, daß $A_4$ ein verestertes Cholin-, Ethanolamin-, Serin-, Glycerin- oder Inosit-Molekül darstellt.

**8.** Verfahren nach einem der Ansprüche 1 bis 7, dadurch gekennzeichnet, daß das Grundpolymer ein vernetztes Styrol-Polymer oder -Copolymer ist und daß die Substitution der Polystyrolkette an der Parastellung des Phenylkerns erfolgt.

**9.** Verfahren nach einem der Ansprüche 1 bis 8, dadurch gekennzeichnet, daß das Grundpolymer ein vernetztes Styrol-Polymer oder -Copolymer ist und daß jede nicht in die Vernetzung einbezogene Phenylgruppe einen nach Anspruch 1 definierten Substituenten tragen kann.

**10.** Vorfahren nach einem der Ansprüche 1 bis 9, dadurch gekennzeichnet, daß das Grundpolymer ein vernetztes Styrol-Polymer oder -Copolymer ist und daß dieses mit höchstens 5 Gew.-% mindestens eines Vernetzungs-Monomers wie Divinylbenzol vernetzt worden ist.

**11.** Verfahren nach einem der Ansprüche 1 bis 10, dadurch gekennzeichnet, daß es aus der in Form von Kugeln mit einer Größe von ungefähr 50 µm vorliegenden Modifikation des Grund-Polystyrols hervorgeht.

**12.** Verfahren nach einem der Ansprüche 1 bis 7, dadurch gekennzeichnet, daß es aus der Modifikation eines in Form von Kugeln mit einer Grösse von ungefähr 50 µm vorliegenden Grund-Polystyrols hervorgeht.

**13.** Verfahren nach einem der Ansprüche 1 bis 12, dadurch gekennzeichnet, daß als Ausgangspolymer ein vernetztes Polystyrol verwendet wird und daß man mit der Bindung des Restes $-CH_2-X$ an dieses Polystyrol beginnt, indem das Polystyrol in Gegenwart von Zinnchlorid mit einem Halogenmethylmethylether umgesetzt wird.

**14.** Verfahren nach einem der Ansprüche 1 bis 12, dadurch gekennzeichnet, daß als Ausgangspolymer ein vernetztes Polystyrol verwendet wird und daß man mit der Bindung des Restes $-CH_2-OH$ an dieses Polystyrol beginnt, indem das nach einem der Verfahren gemäß der Ansprüche 1 bis 12 erhaltene Harz in Gegenwart eines Phasentransfer-Katalysators mit Kaliumacetat umgesetzt und anschließend das erhaltene Acetat mit konzentrierter Kalilauge hydrolisiert wird.

**15.** Verfahren nach einem der Ansprüche 1 bis 12, dadurch gekennzeichnet, daß als Ausgangspolymer ein vernetztes Polystyrol verwendet wird und daß man mit der Bindung des Restes $-CH_2-CH_2-OH$ an dieses Polystyrol beginnt, indem

- das nach einem der Verfahren gemäß der Ansprüche 1 bis 12 erhaltene Harz in Gegenwart eines Phasentransfer-Katalysators mit Natriumcyanid umgesetzt wird, um den Substituenten $-CH_2-C\equiv N$ zu erhalten;
- anschließend das so modifizierte Polystyrol mit alkoholischer Salzsäure umgesetzt wird, um den Substituenten $-CH_2-COOH$ zu erhalten;
- anschließend mit Diboran reduziert wird, um den Substituenten $-CH_2-CH_2-OH$ zu erhalten.

**16.** Verfahren nach einem der Ansprüche 1 bis 12, dadurch gekennzeichnet, daß als Ausgangspolymer ein vernetztes Polymer verwendet wird und daß man mit der Bindung des Restes $-CH_2-CH_2-CH_2-OH$ an dieses Polystyrol beginnt, indem

- auf dem nach dem Verfahren gemäß der Ansprüche 1 bis 12 erhaltenen Harz ein Alkylmalonat kondensiert wird, um den Substituenten

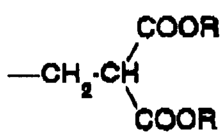

  zu erhalten (R ist ein Alkylrest);
- anschließend durch das Hydrolisieren der so erhaltenen Diester-Funktion eine Disäure-Funktion erhalten wird, die im sauren Milieu decarboxyliert wird, um den Substituenten $-CH_2-CH_2-COOH$ zu erhalten;
- anschließend mit Diboran reduziert wird, um den Substituenten $-CH_2-CH_2-CH_2-OH$ zu erhalten.

**17.** Verfahren nach einem der Ansprüche 1 bis 12, dadurch gekennzeichnet, daß als Ausgangspolymer ein vernetztes Polystyrol verwendet wird und daß man mit der Bindung des Restes $-SO_2-NH-(CH_2)_{2\ oder\ 3}-OH$ an dieses Polystyrol beginnt, indem

- das Ausgangsstyrol mit Chlorsulfonsäure umgesetzt wird, um den Substituenten $-SO_2Cl$ zu erhalten,
- anschließend Ethanolamin oder Propanolamin auf den zuvor erwähnten Substituenten einwirken gelassen wird.

**18.** Verfahren nach einem der Ansprüche 1 bis 12, dadurch gekennzeichnet, daß als Ausgangspolymer ein vernetztes Polystyrol verwendet wird und daß man mit der Bindung des Restes $-SO_2-NH-CH_2-CHOH-CH_2OH$ an dieses Polystyrol beginnt, indem

- das Ausgangspolymer mit Chlorsulfonsäure umgesetzt wird, um den Substituenten $-SO_2Cl$ zu erhalten,
- anschließend Amino-3-propandiol-1,2 auf den zuvor erwähnten Substituenten einwirken gelassen wird.

**19.** Verfahren nach einem der Ansprüche 1 bis 12, dadurch gekennzeichnet, daß als Ausgangspolymer ein vernetztes, gegebenenfalls teilweise durch Carboxymethyl-Gruppen substituiertes Dextran verwendet wird, worauf durch Reaktion mit einem Thionylhalogenid und anschließend durch Reaktion mit einem Amin der Formel $OH-(CH_2)_q-NH_2$ oder mit Hilfe eines Kupplungs-Reagenzes durch Kondensation mit dem hydroxyliertem Amin der Rest $-NH-(CH_2)_q-OH$, wobei q einen Wert von 1 bis 6 hat, gebunden wird.

**20.** Verfahren nach einem der Ansprüche 1 bin 19, dadurch gekennzeichnet, daß die Phosphorylierung mit Hilfe von Methyldichlorphosphat, phosphoriger Säure oder Phosphoroxychlorid und insbesondere mit Hilfe von Phosphoroxychlorid durchgeführt wird.

**21.** Verfahren nach einem der Ansprüche 1 bis 20, dadurch gekennzeichnet, daß die modifizierten Basen mit den Formeln (I) oder (II), in denen Z' = $-CH_2-CH_2-$ ist, durch Umsetzung der Base mit Ethylencarbonat in Gegenwert eines Phasentransfer-Katalysators herstellt wird.

**22.** Verfahren nach einem der Ansprüche 1 bis 21, dadurch gekennzeichnet, daß die Reaktion des Restes $-Z-A_1$ mit den modifizierten Basen mit den Formeln (I) oder (II) mit Hilfe eines Kupplungs-Reagenzes wie N,N'-Dicyclohexylcarbodiimid durchgeführt wird.

**23.** Verfahren nach einem der Ansprüche 1 bis 21, dadurch gekennzeichnet, daß eine modifizierte Base mit der Formel (Ia) oder (IIa) durch das Phosphorylieren der entsprechenden Base mit der Formel (I) oder (II) mit $POCl_3$ hergestellt wird und anschließend die Base mit der Formel (Ia) oder (IIa) mit Hilfe eines Kupplungs-Reagenzes wie N,N'-Dicyclohexylcarbodiimid mit dem Substituenten -Z-OH kondensiert wird.

**24.** Verwendung eines in den Ansprüchen 1 bis 12 definierten, von einem vernetzten Styrol-Polymer oder -Copolymer oder von einem vernetzten Dextran abgeleiteten Polymers als stationäre Phase bei der Ionenaustausch-Chromatographie.

**25.** Verwendung eines in den Ansprüchen 1 bis 12 definierten, von einem vernetzten Styrol-Polymer oder -Copolymer oder von einem vernetzten Dextran abgeleiteten Polymers als stationäre Phase bei der Affinitäts-Chromatographie.

**26.** Verwendung nach Anspruch 25 zur Erzielung selektiver Reinigungen verschiedener, durch lupusartige Krankheiten entwickelter Antikörper-Typen.

**27.** Verwendung nach einem der Ansprüche 24 und 25 zur Reinigung von Enzymen, Coenzymen oder enzymatischen Komplexen, die als Substrate die DNS, die RNS und die Nucleotide zuläßt.

**28.** Verwendung nach einem der Ansprüche 24 und 25 zur Reinigung von Blutgerinnungs-Faktoren, ausgehend von verschiedenen plasmatischen Extrakten.

**29.** Verwendung eines in den Ansprüchen 1 bis 12 definierten, von einem vernetzten Styrol-Polymer oder -Copolymer oder von einem vernetzten Dextran abgeleiteten Polymers zur Reinigung von Molekülen biologischen Ursprungs wie der im Verlauf der Blutgerinnung und in biologischen Analytiksystemen wie RIA, ELISA, Elektrophorese, ein-und Zweidimensionaler Immunoelektrophorese, Elektrofokussierung auftretenden Proteine.

**30.** Verwendung nach Anspruch 29, um Dosierungen der verschiedenen lupusartigen Antikörper-Typen und insbesondere die Dosierung von gerinnungshemmenden Kreislaufmitteln und/oder von Anti-Phospholipiden herzustellen.

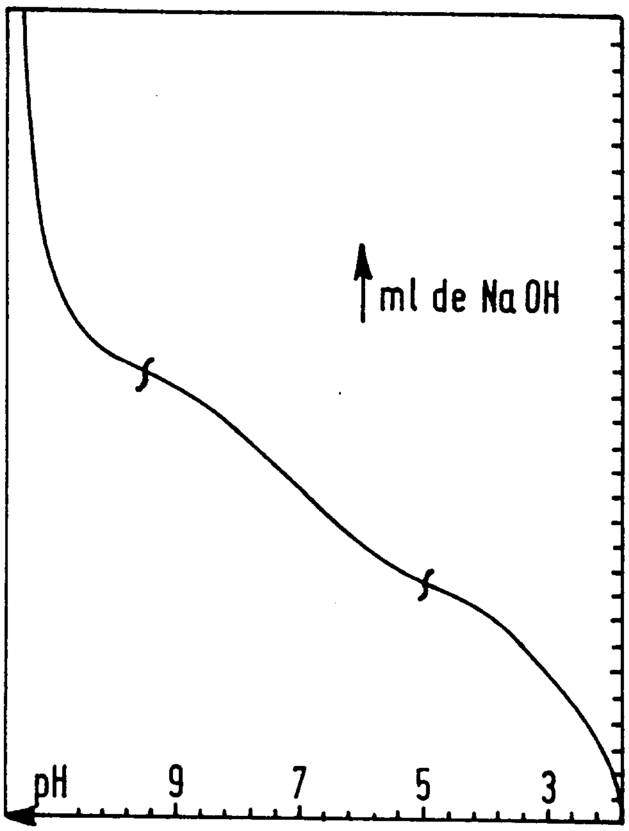


FIG.1

FIG.2

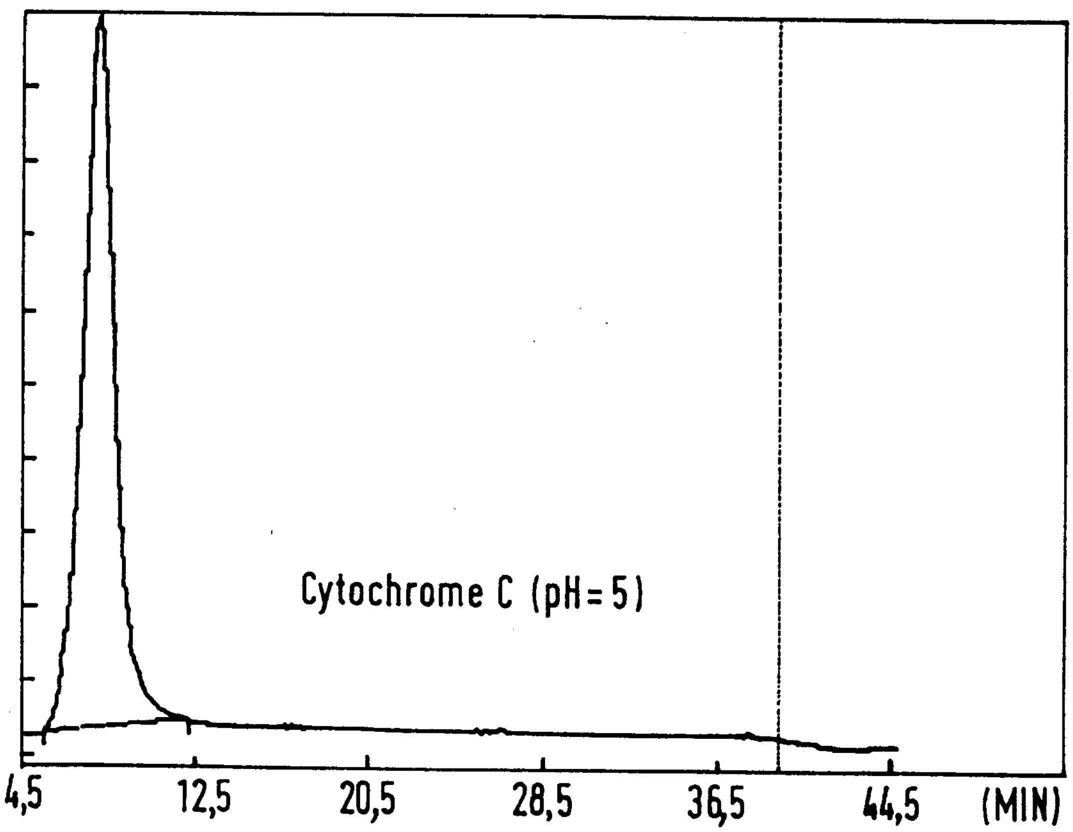

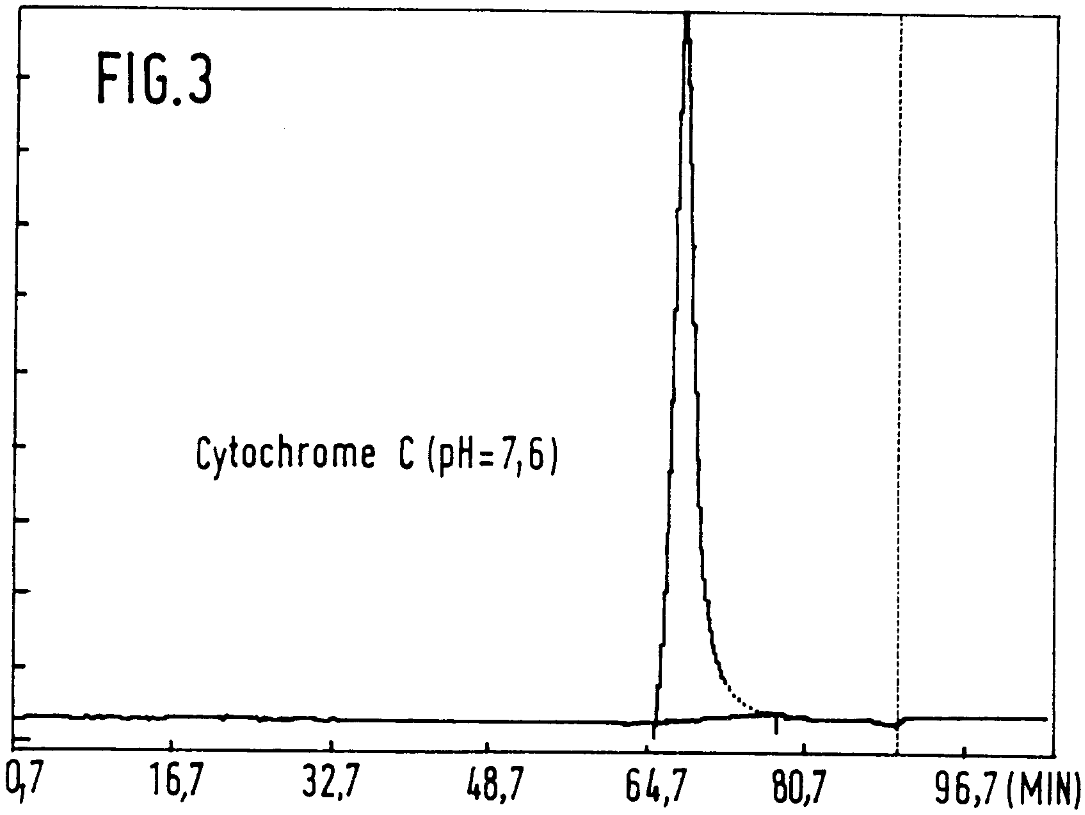
FIG.3
Cytochrome C (pH=7,6)
0,7
16,7
32,7
48,7
64,7
80,7
96,7 (MIN)

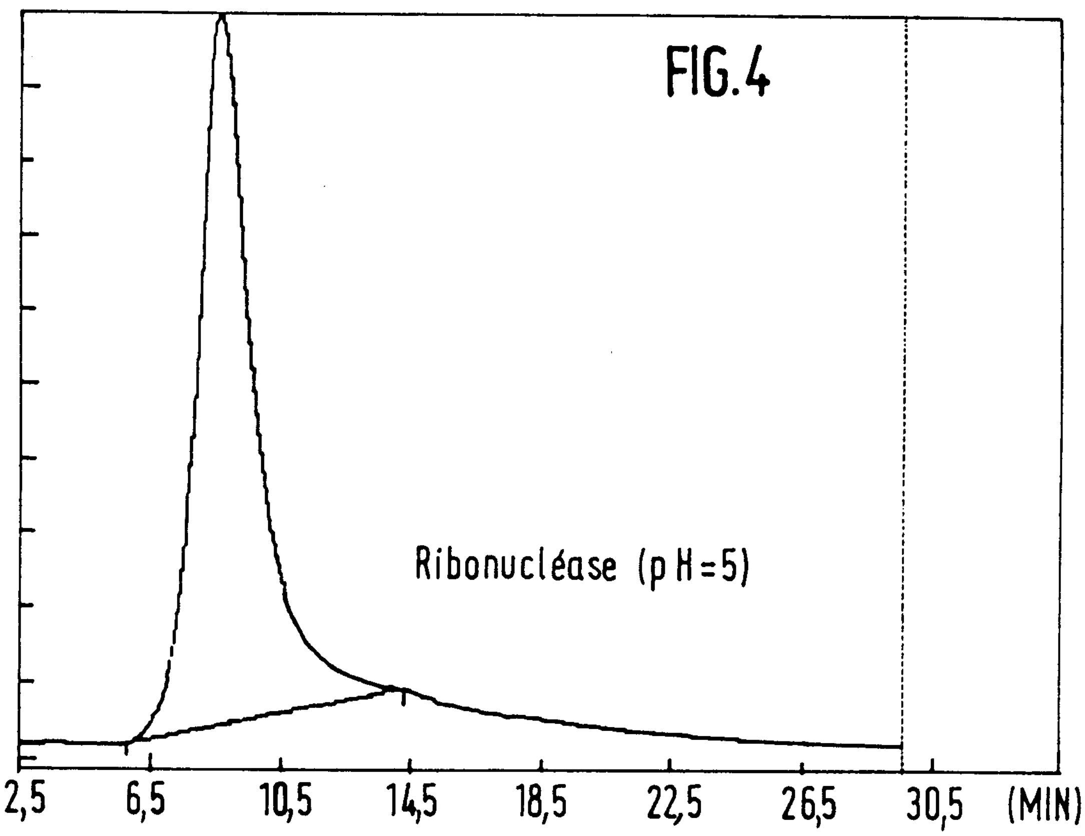
FIG.4
Ribonucléase (pH=5)
2,5
6,5
10,5
14,5
18,5
22,5
26,5
30,5
(MIN)

FIG.5

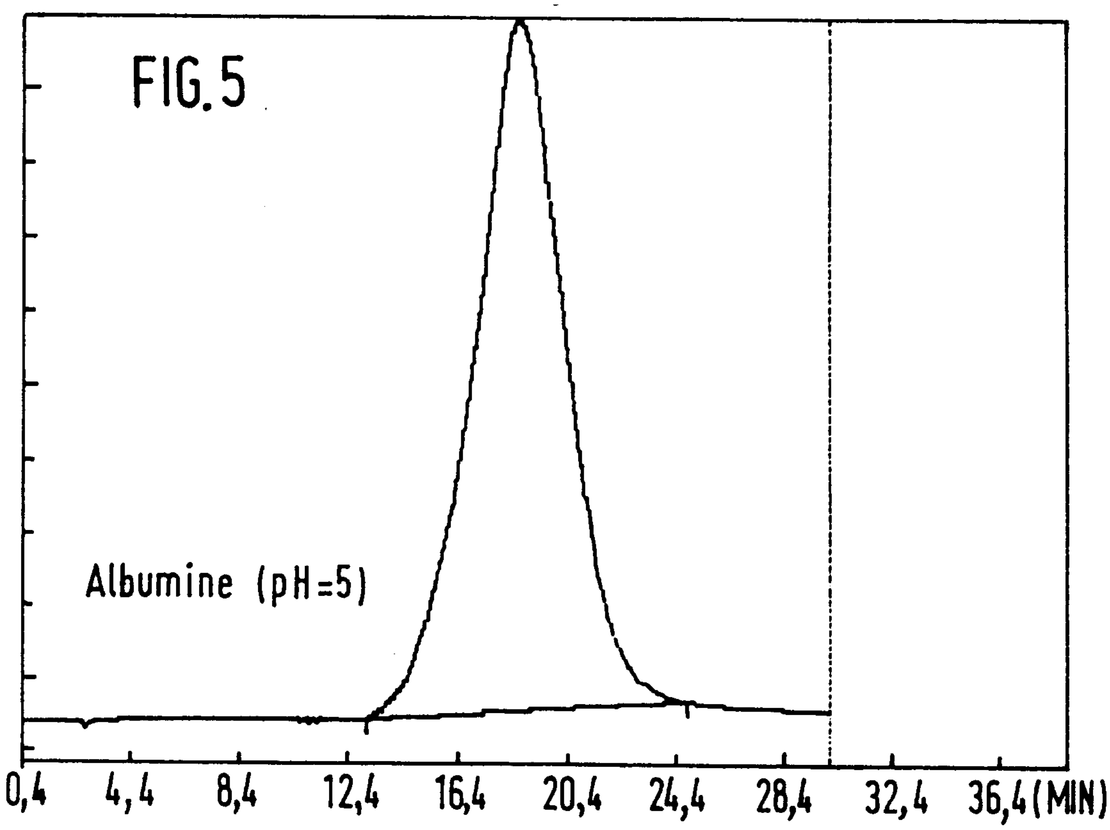
Albumine (pH=5)
0,4
4,4
8,4
12,4
16,4
20,4
24,4
28,4
32,4
36,4(MIN)

FIG.6

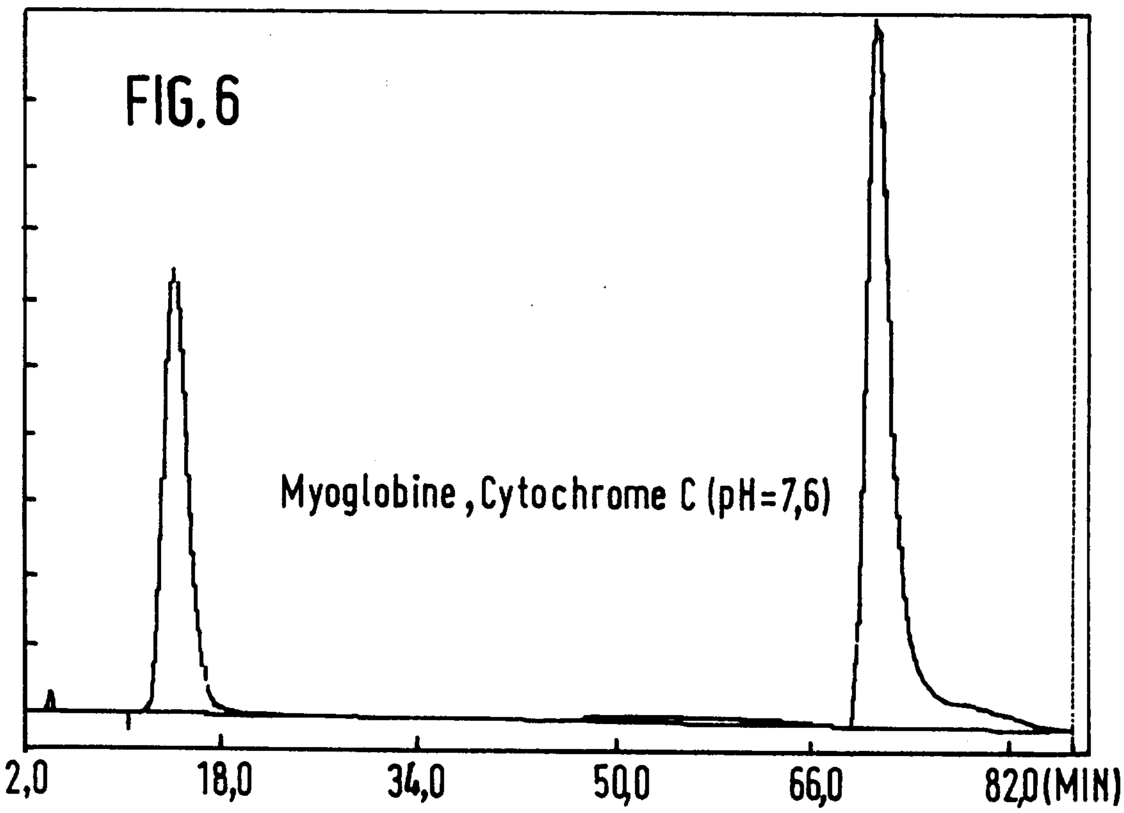
Myoglobine, Cytochrome C (pH=7,6)
2,0
18,0
34,0
50,0
66,0
82,0(MIN)

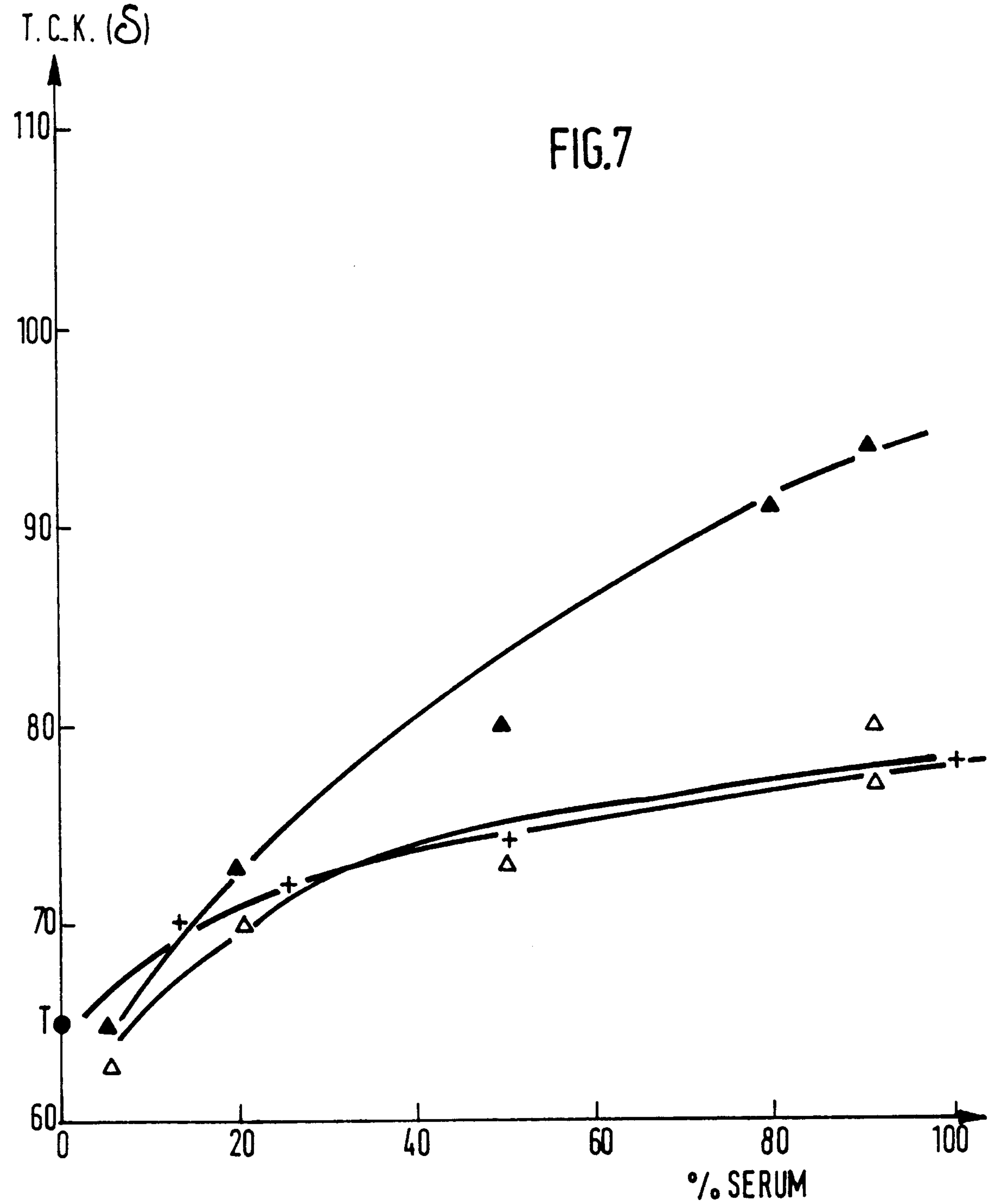


FIG.7

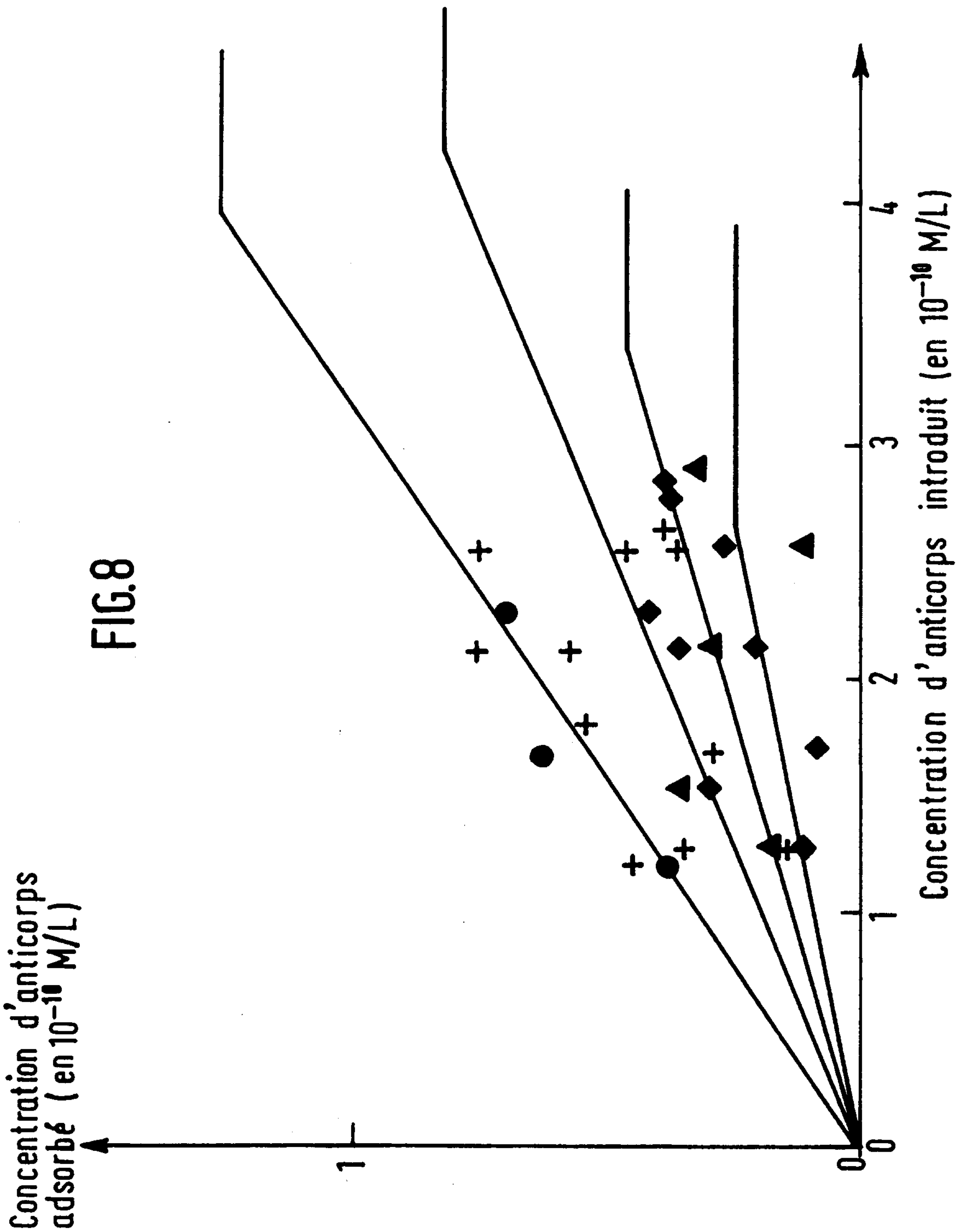
Concentration d'anticorps adsorbé (en 10⁻¹⁰ M/L)
1
0
0
1
2
3
4
Concentration d'anticorps introduit (en 10⁻¹⁰ M/L)

FIG.8

FIG.9

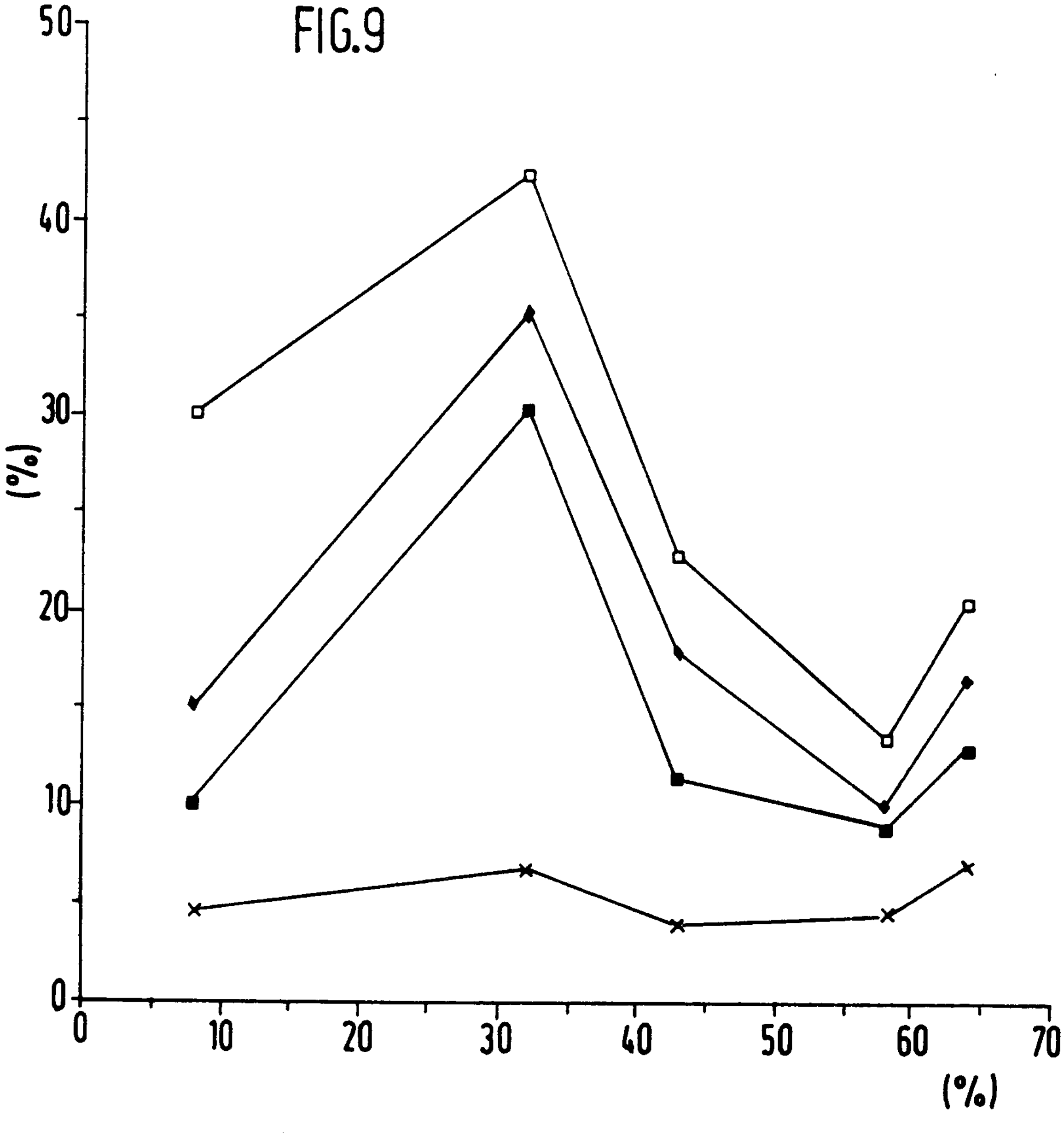

FIG.10